# EUROPEAN PATENT APPLICATION

(11) **EP 4 449 972 A1**
(43) Date of publication of application: **23.10.2024**
(21) Application number: 22893941.9
(22) Date of filing: 07.12.2022
(51) Int. Cl.: A47L 23/20, A47B 61/04, F26B 21/00, F26B 9/00, F26B 7/00, F26B 3/02, A61L 2/07

(54) **SHOE CARE APPARATUS**

(30) Priority: 17.12.2021 KR 20210182006
(71) Applicant: LG Electronics Inc., Seoul 07336 (KR)
(72) Inventor: KIM, Jae Young, Seoul 08592 (KR); NAM, Bo Hyun, Seoul 08592 (KR); SONG, Seung Hyun, Seoul 08592 (KR)
(74) Representative: Schornack, Oliver
(86) International application number: PCT/KR2022/019850
(87) International publication number: WO 2023/113368

(57) **Abstract**

Provided is a shoes care device configured to treat shoes for air circulation. The shoes care device comprise: an inner cabinet having an accommodation space configured to accommodate shoes; a outlet formed on a bottom surface of the inner cabinet so as to suck air inside the inner cabinet; a connection path forming a flow path through which air in the accommodation space is discharged from the inner cabinet through the outlet and then introduced back into the accommodation space; a blowing part disposed in the connection path and configured to ventilate air; a dehumidifying part disposed in the connection path and configured to dehumidify air; and a main shelf installed to cover the bottom surface of the inner cabinet and having an upper surface on which the shoes can be settled.

## Description

### Technical Field

The present invention relates to a shoes care device, and more particular, to a shoes care device which treats shoes by air circulation.

### Background Art

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

According to Prior Document 1 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 1 above, the air from which moisture has been removed by a deodorization module including zeolite and activated carbon can be discharged to the outside of the apparatus for sterilization disposal of shoes.

However, in Prior Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

According to Prior Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 2 above, the air is circulated by a circulating fan in a shoe closet, and the sanitary filter portion is disposed on a circulation path of the air to remove germs and odors in the air.

However, since Prior Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience. There is a need to develop a shoes care device that takes these matters into consideration.

In addition, the reduction of manufacturing costs, the convenience of manufacturing and assembling each component, and the convenience of maintenance needs to be considered in the development of the shoes care device.

### Disclosure

### Technical Problem

An object to be achieved by the present invention is to solve the aforementioned problems caused by a shoes care device which treats shoes by air circulation.

Specifically, an object to be achieved by the present invention is to provide a shoes care device which can ensure proper performance for shoes treatment at all times by performing dehumidification and deodorization on the shoes using a dehumidifying material to refresh the shoes and regenerating the used dehumidifying material.

An object to be achieved by the present invention is to provide a shoes care device which prevents air used for dehumidification and deodorization of shoes from being exposed to a user by an air circulation structure capable of dehumidifying the air inside an inner cabinet where the shoes are placed by using a dehumidifying material and supplying the dehumidified air again to the inner cabinet.

An object to be achieved by the present invention is to provide a shoes care device which can easily manage the inner cabinet by preventing bottom surfaces of the shoes accommodated in the shoes care device from being in direct contact with a bottom surface of the inner cabinet.

The technical objects of the present disclosure are not restricted to those set forth Here, and other unmentioned technical objects will be clearly understood by one of ordinary skill in the art to which the present disclosure pertains by referencing the detailed description of the present disclosure given below.

### Technical Solution

In order to achieve the above and other objects, a shoes care device according to one aspect of the present invention is configured not only to perform dehumidification and deodorization of shoes using a dehumidifying part but also to regenerate the used dehumidifying part. Specifically, the shoes care device is configured not only to collect moisture and bacteria in air ventilated by the dehumidifying part in a module chamber but also to heat and regenerate the dehumidifying part in the module chamber.

In addition, according to one aspect of the present invention, the shoes care device is configured such that the air used for dehumidifying and deodorizing the shoes has an air circulation structure inside the shoes care device. Specifically, a connection path through which air is circulated is formed between a outlet and a nozzle disposed inside an inner cabinet, respectively.

In addition, according to one aspect of the present invention, the shoes care device is configured such that bottom surfaces of the shoes accommodated in the inner cabinet is not in direct contact with a bottom surface of the inner cabinet. Specifically, the shoes care device is configured such that the shoes are settled on an upper surface of a main shelf covering the bottom surface of the inner cabinet.

In addition, according to one aspect of the present invention, the shoes care device is configured such that the main shelf may be detached from the inner cabinet.

In addition, according to one aspect of the present invention, the shoes care device is configured such that a main isolation rib may be formed on an upper surface of the main shelf.

In addition, according to one aspect of the present invention, the shoes care device is configured such that the main isolation rib may be formed in an oblique direction.

In addition, according to one aspect of the present invention, the shoes care device is configured such that the main isolation rib may be formed in a symmetrical shape.

In addition, according to one aspect of the present invention, the shoes care device is configured such that the main isolation rib may be formed to move water on the main shelf toward a front side of the inner cabinet.

In addition, according to one aspect of the present invention, the shoes care device is configured such that a part corresponding to an upper part of the outlet of the main shelf may be formed to penetrate.

In addition, according to one aspect of the present invention, the shoes care device is configured such that the main shelf may be inclined downwardly toward the front side of the inner cabinet having the outlet formed therein.

In addition, according to one aspect of the present invention, the shoes care device is configured such that the position of the shoes may be guided by forming a guide rib on the main shelf.

In addition, according to one aspect of the present invention, the shoes care device is configured such that a pair of guide ribs may be formed symmetrical with each other.

In addition, according to one aspect of the present invention, the shoes care device is configured such that the guide rib may be formed in a curved shape.

In addition, according to one aspect of the present invention, the shoes care device is configured such that the guide rib may be formed on the front side of the inner cabinet.

In addition, according to one aspect of the present invention, the shoes care device is configured such that a main water stop rib may be formed along an inner circumferential surface of the main shelf.

In addition, according to one aspect of the present invention, the shoes care device is configured such that the main water stop rib may be in close contact with the inner surface of the inner cabinet.

In addition, according to one aspect of the present invention, the shoes care device is configured such that a central part of the main shelf may be convexly formed.

In addition, according to one aspect of the present invention, the shoes care device is configured such that a space may be formed between the main shelf and the bottom surface of the inner cabinet.

In addition, according to one aspect of the present invention, the shoes care device is configured such that a door drain surface may protrude to induce condensed water flowing down along the inner surface to the inside of the inner cabinet.

In addition, according to one aspect of the present invention, the shoes care device is configured such that the door drain surface may protrude to pass through an upper part of the main water stop rib.

In addition, according to one aspect of the present invention, the shoes care device is configured such that a depression may be formed on the door drain surface to correspond to the shape of the guide rib.

In addition, according to one aspect of the present invention, the shoes care device is configured such that a drain rib may be formed on an upper part of the depression of the door drain surface.

The technical objects of the present disclosure are not restricted to those set forth Here, and other unmentioned technical objects will be clearly understood by one of ordinary skill in the art to which the present disclosure pertains by referencing the detailed description of the present disclosure given below.

Further scope of applicability of the present disclosure will become apparent from the detailed description given hereinafter. It should be understood, however, that the detailed description and specific examples, such as the preferred implementation of the invention, are given by way of illustration only, since various changes and modifications within the spirit and scope of the invention will be apparent to those skilled in the art.

### Description of Drawings

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together.
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12 is a perspective view illustrating a modified example of a state in which the door, the outer cabinet, and the inner cabinet are removed from the shoes care device according to one embodiment of the present invention.
FIG. 13 is a perspective view illustrating a part of the machine room of the shoes care device illustrated in FIG. 12.
FIG. 14 is a cross-sectional view illustrating a sump and a drain tank in more detail in a part of the machine room of the shoes care device illustrated in FIG. 12.
FIG. 15 is a view illustrating a front surface of a machine room in the shoes care device according to one embodiment of the present invention.
FIG. 16 is a perspective view illustrating the drain tank in more detail in the shoes care device according to one embodiment of the present invention.
FIG. 17 is a view exemplarily illustrating a detachable state of the drain tank in the shoes care device according to one embodiment of the present invention.
FIG. 18 is a cross-sectional view illustrating a module chamber and a main shelf in more detail in the shoes care device according to one embodiment of the present invention.
FIG. 19 is a perspective view illustrating the main shelf in the shoes care device according to one embodiment of the present invention.
FIG. 20 is a cross-sectional view taken along Ac-A'c of the main shelf illustrated in FIG. 19.
FIG. 21 is a cross-sectional view taken along Bc-B'c of the main shelf illustrated in FIG. 19.
FIG. 22 is a view exemplarily illustrating a state in which the shoes are settled on the main shelf in the shoes care device according to one embodiment of the present invention.
FIG. 23 is a view illustrating the inside of the shoes care device according to one embodiment of the present invention in a state in which the door is closed.
FIG. 24 is a view illustrating a state in which an auxiliary shelf is installed in the shoes care device according to one embodiment of the present invention.
FIG. 25 is a cross-sectional view illustrating an inner surface of the door illustrated in FIG. 23.
FIG. 26 is a perspective view illustrating the auxiliary shelf in the inner surface of the door according to one embodiment of the present invention.
FIG. 27 is a view illustrating the auxiliary shelf and a nozzle duct in the shoes care device according to one embodiment of the present invention.
FIG. 28 is a view illustrating a state in which the nozzle duct is coupled to a cross section taken along line Cc-C'c of the auxiliary shelf illustrated in FIG. 26.
FIG. 29 is a view exemplarily illustrating a state in which the shoes are settled on the auxiliary shelf in the shoes care device according to one embodiment of the present invention.
FIG. 30 is a view illustrating a control panel in more detail in the shoes care device according to one embodiment of the present invention.
FIG. 31 is a view exemplarily illustrating a state in which a user uses the control panel in the shoes care device according to one embodiment of the present invention.
FIG. 32 is a view exemplarily illustrating a fixed UI section and a variable UI section of the control panel illustrated in FIG. 30.
FIGS. 33a and 33b are views exemplarily illustrating a modified example of the control panel illustrated in FIG. 32.
FIG. 34a is an exploded perspective view illustrating a condenser according to one embodiment of the present invention.
FIG. 34b is a view illustrating an internal state of the condenser of FIG. 34a.
FIG. 34c is a front view illustrating the condenser of FIG. 34b.
FIG. 35 is a cross-sectional view illustrating a first module chamber of a module housing in the shoes care device according to the present invention.
FIG. 36 is a perspective view illustrating a dehumidifying part and a dehumidifying material housing according to one embodiment of the present invention.
FIG. 37 is a bottom perspective view illustrating a module cover according to one embodiment of the present invention.
FIG. 38 is a cross-sectional view illustrating a third module chamber of the module housing in the shoes care device according to the present invention.
FIG. 39 is a perspective view illustrating a steam separator according to one embodiment of the present invention.
FIG. 40a is a cross-sectional view illustrating a part of a separating inlet of the steam separator in the shoes care device according to one embodiment of the present invention.
FIG. 40b is a cross-sectional view illustrating a separating connector of the steam separator in the shoes care device according to one embodiment of the present invention.
FIGS. 41a and 41b are perspective views illustrating the main shelf according to one embodiment of the present invention.
FIG. 42 is a cross-sectional view illustrating the shoes care device according to one embodiment of the present invention, and schematically illustrating a state in which the shoe is settled on an upper side of the main shelf.
FIG. 43 is a cross-sectional view illustrating the shores care device according to one embodiment of the present invention and is a view schematically illustrating a state in which the shoes having a larger size than that of FIG. 42 is settled on the upper side of the main shelf.
FIG. 44 is a view illustrating the inside of the module housing according to one embodiment of the present invention. FIG. 44 illustrates that a schematic flow direction of air inside the module housing is indicated by an arrow.
FIG. 45 is a view illustrating the inside of the module housing according to one embodiment of the present invention. FIG. 45 illustrates a dehumidifying material cover together.
FIG. 46 is a perspective view illustrating a drying module according to one embodiment of the present invention.
FIG. 47 is a bottom perspective view illustrating the dehumidifying material cover illustrated in FIG. 46.
FIG. 48 is a cross-sectional view illustrating the drying module of FIG. 46.
FIGS. 49a and 49b are views illustrating simulation results of air speed distribution when an inner rib is not formed in the module housing according to one embodiment of the present invention, and FIGS. 50a and 50b are diagrams illustrating simulation results of the air speed distribution when the inner rib is formed in the module housing according to another embodiment of the present invention.
FIG. 51a is a view illustrating the entire area of the dehumidifying part divided into six areas in a plan view in the shoes care device according to one embodiment of the present invention, and FIG. 51b is a view illustrating a mass flow rate in each area of FIG. 51a.
FIG. 52 is a perspective view illustrating a state in which the inner cabinet and the module housing are coupled according to one embodiment of the present invention.
FIG. 53 is a perspective view illustrating the inner cabinet illustrated in FIG. 52.
FIG. 54 is a perspective view of the inner cabinet illustrated in FIG. 53 when viewed from the back.
FIG. 55a is a front view illustrating the inner cabinet illustrated in FIG. 53, and FIG. 55a is a rear view illustrating the inner cabinet illustrated in FIG. 53.
FIGS. 56a and 56b are cross-sectional perspective views illustrating the inner cabinet illustrated in FIG. 53.
FIGS. 57a and 57b are side cross-sectional views illustrating the inner cabinet illustrated in FIG. 53.
FIG. 58a is a cross-sectional view schematically illustrating a mold used in manufacturing the inner cabinet according to one embodiment of the present invention, and FIG. 58b is a schematic view illustrating a state in which the mold of FIG. 58a is closed.
FIG. 59 is a side view illustrating a state in which the inner cabinet, the module housing, and a dry air duct are separated from each other in the shoes care device according to one embodiment of the present invention.
FIG. 60a is a side view illustrating the module cover according to one embodiment of the present invention.
FIG. 60b is a cross-sectional view illustrating the inner cabinet according to one embodiment of the present invention.
FIG. 61a is a cross-sectional perspective view of a part of the shores care device before the coupling of the module housing and the inner cabinet is completed, and
FIG. 61b is a cross-sectional perspective view of a part of the shores care device where the coupling of the module housing and the inner cabinet is completed.
FIG. 62 is a cross-sectional view illustrating a state in which the inner cabinet and the module housing are coupled to each other in the shores care device according to one embodiment of the present invention.
FIG. 63a is a perspective view illustrating a lower side of the inner cabinet and a part of a first wall, and FIG. 63b is a rear perspective view illustrating the lower side of the inner cabinet and a part of the first wall.
FIG. 64 is a front view illustrating a state in which the door is removed from the shores care device illustrated in FIG. 1b. FIG. 64 illustrates the shoes accommodated in the inner cabinet together.
FIG. 65 is a cross-sectional view illustrating a state in which the door is removed from the shores care device illustrated in FIG. 4c. FIG. 65 illustrates the shoes accommodated in the inner cabinet together.
FIG. 66a is a cross-sectional view illustrating the inner cabinet according to one embodiment of the present invention and is a cross-sectional view illustrating an inner upper plate of the inner cabinet of a first management device.
FIG. 66b is a cross-sectional view illustrating the inner cabinet according to one embodiment of the present invention and is a cross-sectional view illustrating the inner upper plate of the inner cabinet of a second management device.
FIG. 67a is a view illustrating a state in which an angle of the nozzle duct illustrated in FIG. 65 is adjusted and is a view illustrating a state in which the length direction of the nozzle duct is parallel to the horizontal direction thereof.
FIG. 67b is a view illustrating a state in which the angle of the nozzle duct illustrated in FIG. 65 is adjusted and is a view illustrating a state in which the angle of the nozzle duct is adjusted to descend the nozzle.
FIG. 68 is a cross-sectional view illustrating a state in which the door is removed from the shoes care device illustrated in FIG. 4c. FIG. 68 illustrates the inside of the inner cabinet of the second management device.
FIG. 69 is a perspective view illustrating the shoes care device and a visible shoes care device of a shoes management set according to one embodiment of the present invention.
FIG. 70 is a perspective view illustrating a state in which the visible shoes care device of the shoes management set of FIG. 69 is stacked on an upper surface of the shoes care device.
FIG. 71 is a perspective view of the shoes care device of FIG. 69 when viewed from another direction, illustrating a state in which the door is opened.
FIG. 72a is a perspective view illustrating the visible shoes care device of FIG. 69.
FIG. 72b is a view illustrating a state in which a display space of the visible shoes care device of FIG. 72 is opened
FIG. 73 is a perspective view illustrating a bottom surface of the visible shoes care device and the upper surface of the shoes care device, which constitutes the shoes management set of FIG. 69.
FIG. 74 is a partial side view of the shoes management set of FIG. 70.
FIG. 75a is a partial cross-sectional view of the shoes management set of FIG. 74 cut in an XZ plane and is a view illustrating Part A of FIG. 74.
FIG. 75b is a partial cross-sectional view of the shoes management set of FIG. 74 cut into the XZ plane and is a view illustrating Part B of FIG. 74.
FIG. 76 is a top plan view illustrating the upper surface of the shoes care device of FIG. 69.
FIG. 77 is a bottom view illustrating a bottom surfaces of the visible shoes care device of FIG. 69.
FIG. 78 is a partial front view of the shoes management set of FIG. 70.
FIG. 79a is a partial cross-sectional view of the shoes management set of FIG. 78 cut into an YZ plane and is a view illustrating Part C of FIG. 78
FIG. 79b is a partial cross-sectional view of the shoes management set of FIG. 78 cut into the YZ plane and is a view illustrating Part D of FIG. 78.

### [Modes for the Invention]

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

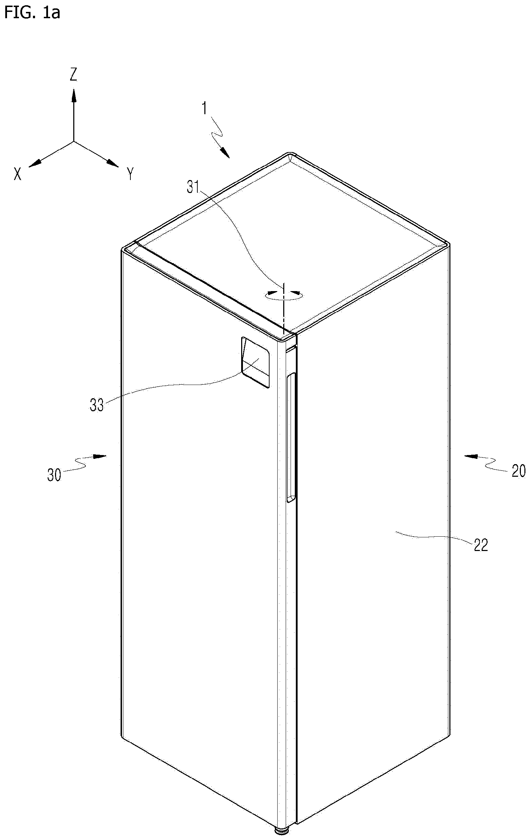

FIG. 1a is a perspective view illustrating a shoes care device according to one embodiment of the present invention.

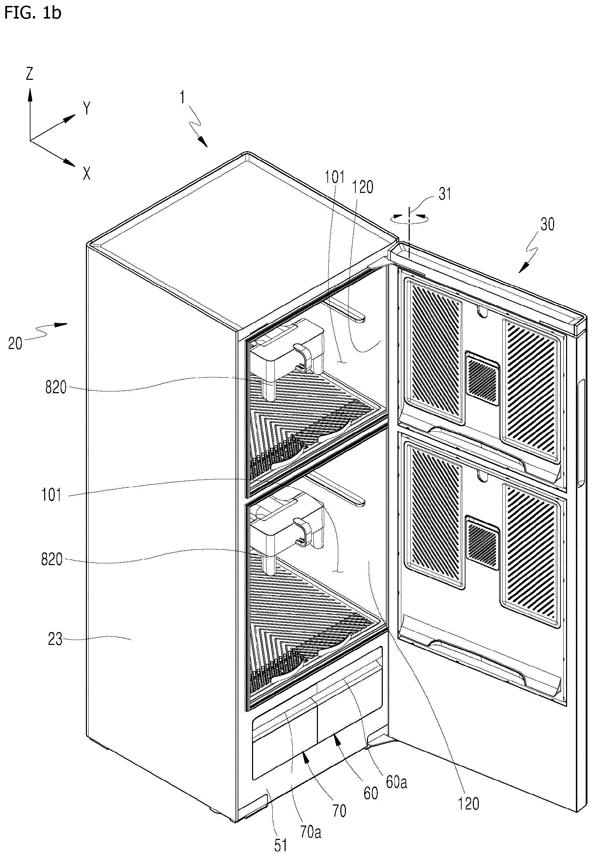

FIG. 1b is a perspective view of the shoes care device of FIG. 1a, when viewed from another direction, illustrating a state in which a door is opened.

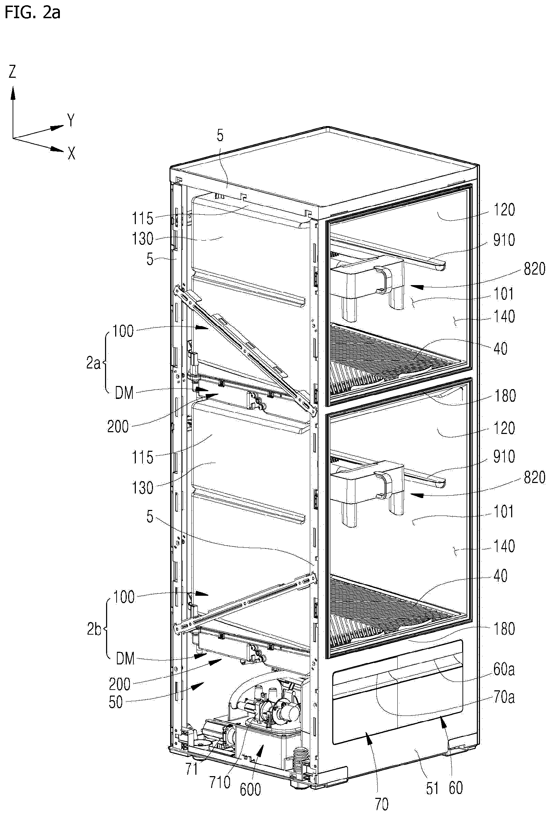

FIG. 2a is a perspective view illustrating a state in which a part of the door and an outer cabinet are removed from the shoes care device of FIG. 1b.

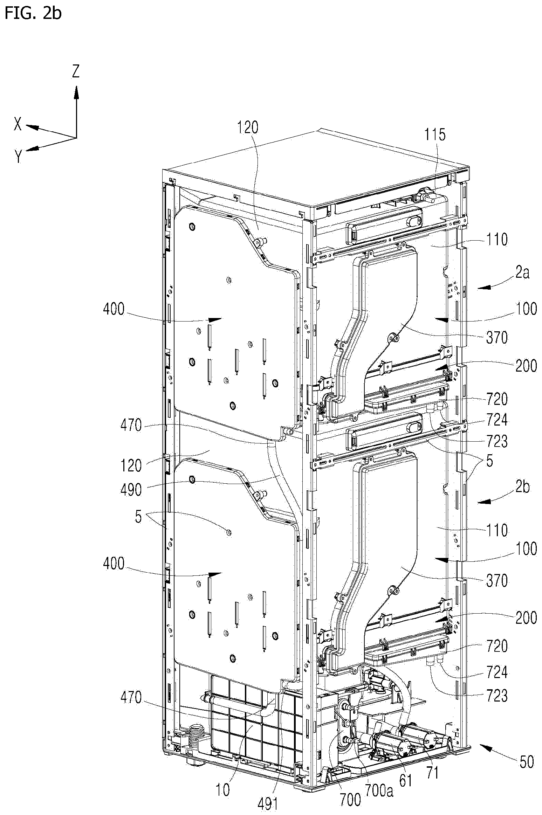

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

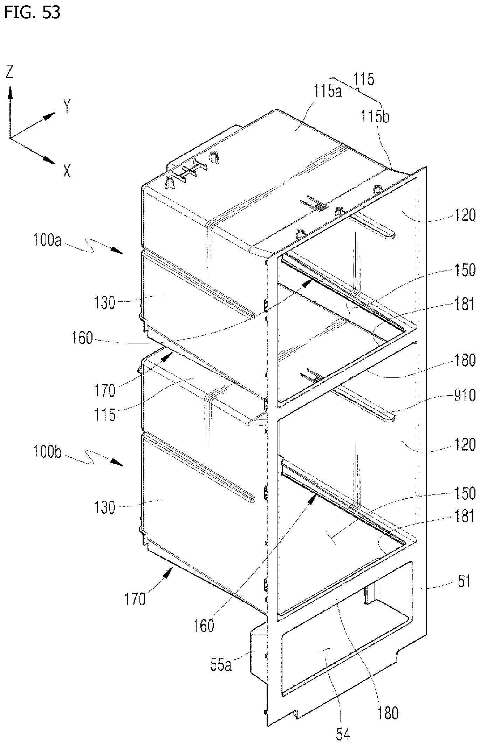

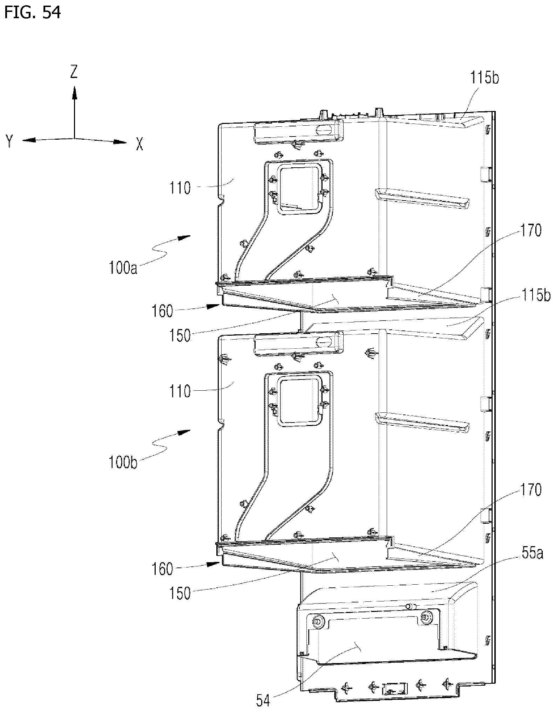

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIGS. 53 and 54).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

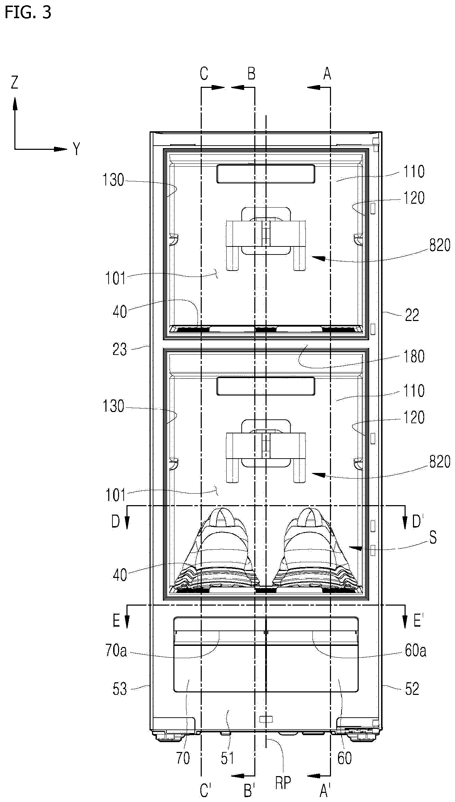

FIG. 3 is a front view illustrating a state in which a door is removed from the shoes care device illustrated in FIG. 1b. FIG. 3 illustrates shoes accommodated in an inner cabinet together.

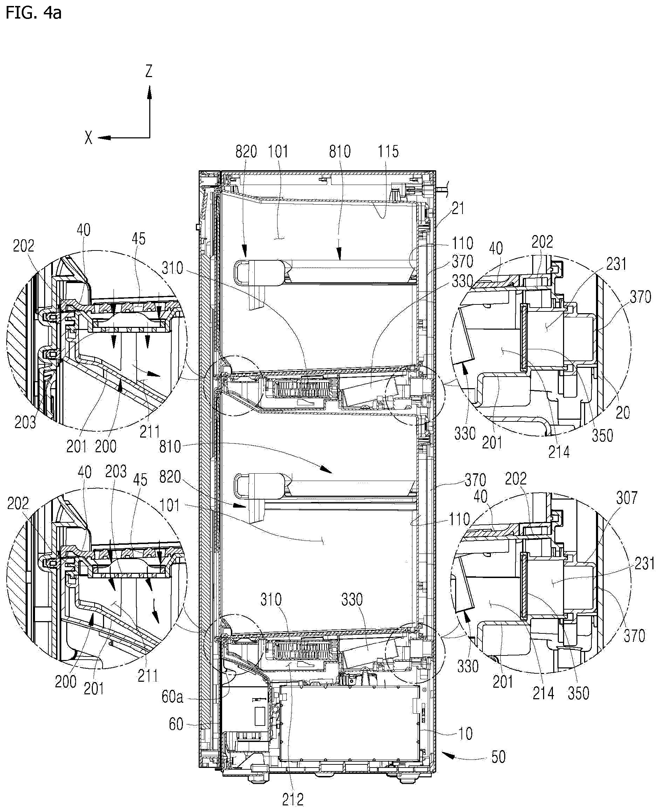

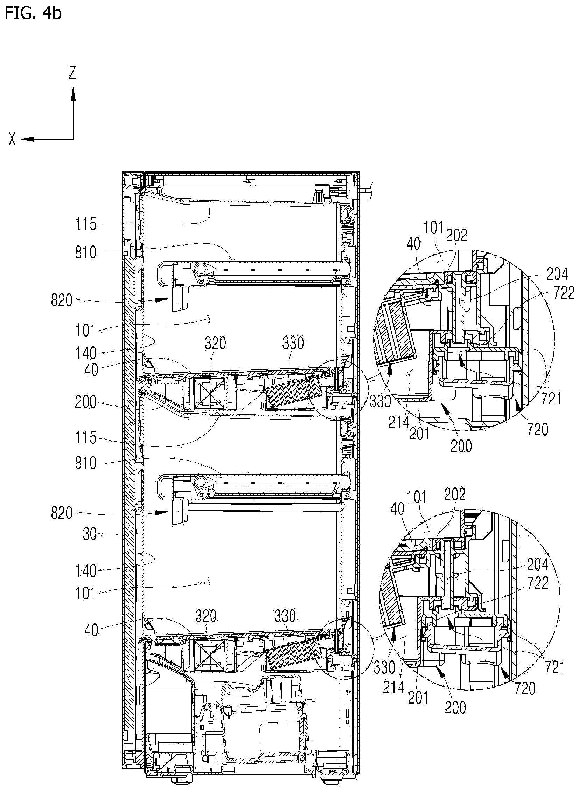

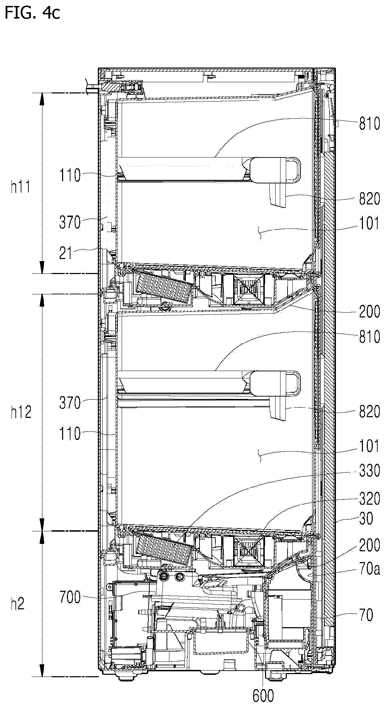

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include a outlet 203 and a nozzle 820.

The management devices 2a and 2b may include the module housing 200 forming a connection path F10.

The management devices 2a and 2b may include a regeneration path F20. The management devices 2a and 2b may include a heating part 320.

The management devices 2a and 2b may include a conversion flow path F10a.

The management devices 2a and 2b may include a damper 350.

The shoes care device 1 may include a steam generator 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoes care device 1 moves.

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam generator 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

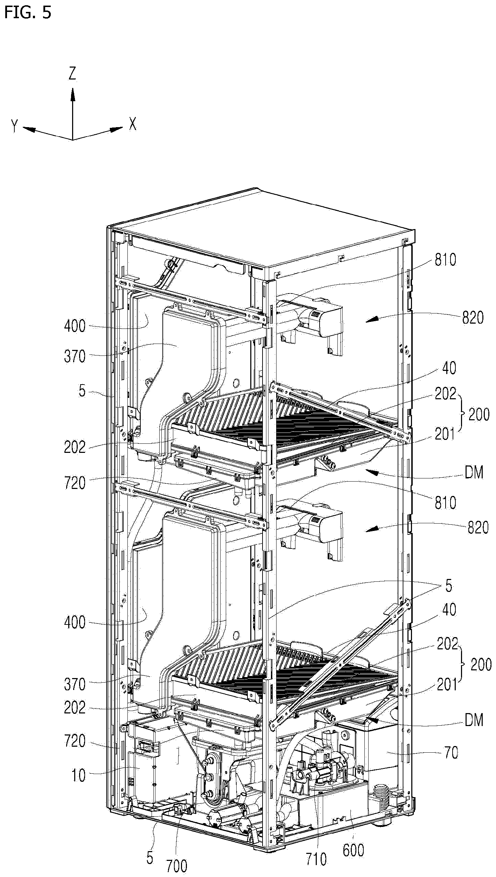

FIG. 5 is a perspective view illustrating a state in which the door 30, the outer cabinet 20, and the inner cabinet 100 are removed from the shoes care device 1 according to one embodiment of the present invention.

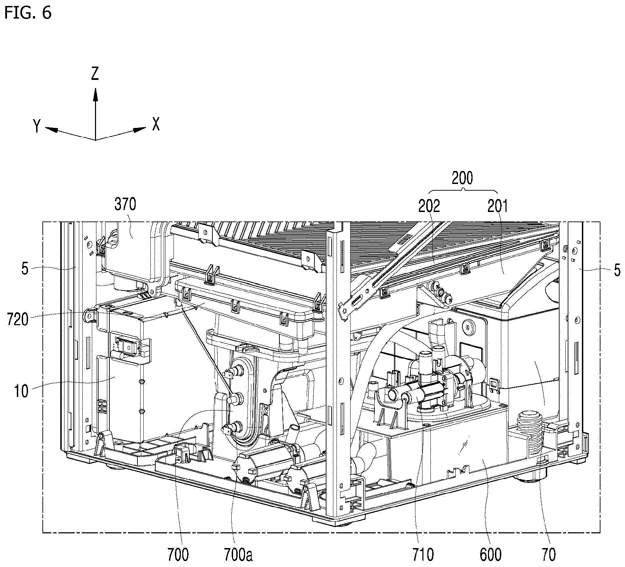

FIG. 6 is a perspective view illustrating a machine room part of the shoes care device 1 illustrated in FIG. 5.

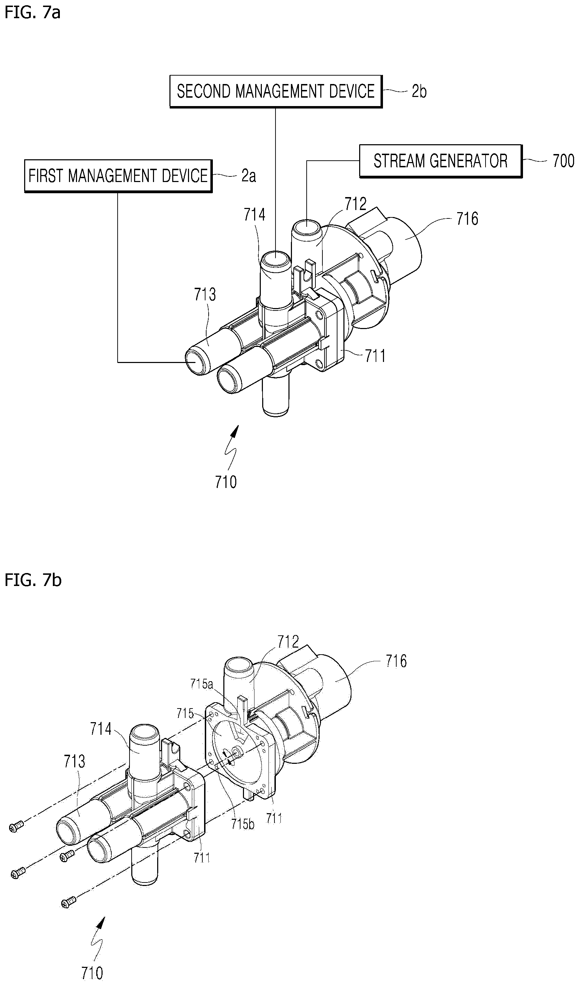

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoes care device 1 according to one embodiment may be provided with one steam generator 700, and the shoes care device 1 according to another embodiment may be provided with two or more steam generators 700.

When the shoes care device 1 is provided with one steam generator 700, the steam generator 700 may be configured to supply steam into the inner cabinet 100 of the first management device 2a and/or into the inner cabinet 100 of the second management device 2b.

When the shoes care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

An external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoes care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoes care device 1.

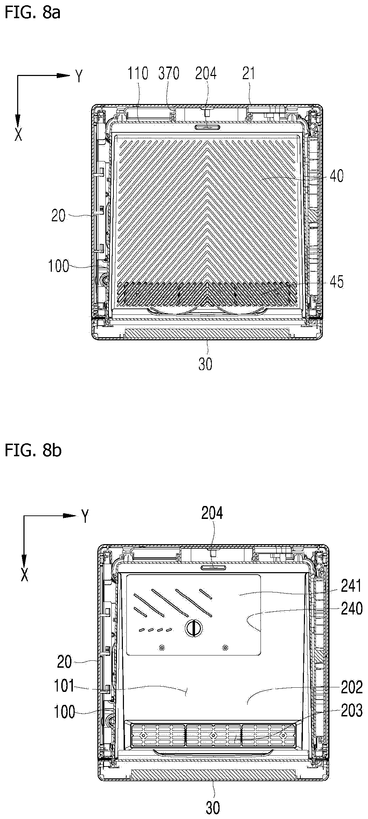

FIG. 8a is a cross-sectional view taken along line D-D' of the shoes care device 1 illustrated in FIG. 3.

FIG. 8b is a view illustrating a state in which the main shelf 40 is removed from the shoes care device 1 illustrated in FIG. 8a.

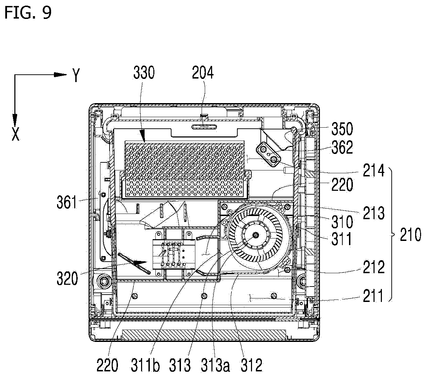

FIG. 9 is a cross-sectional view taken along line E-E' of the shoes care device illustrated in FIG. 3.

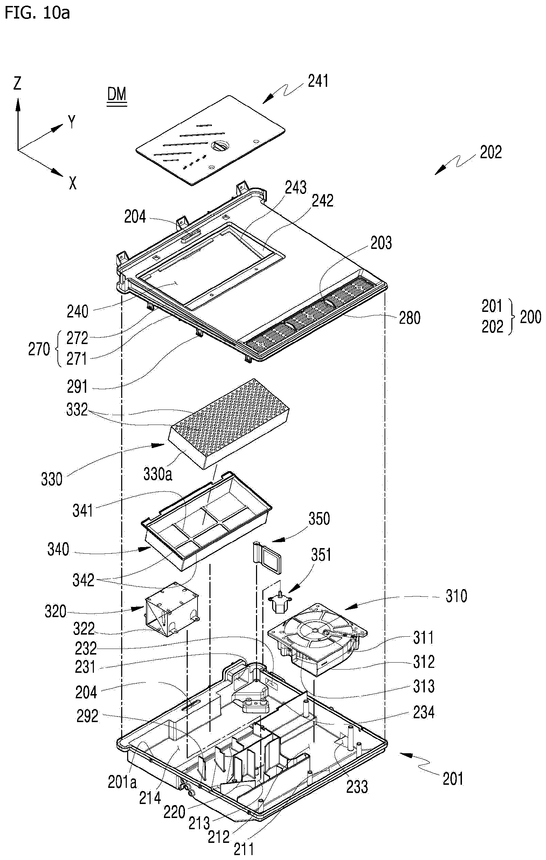

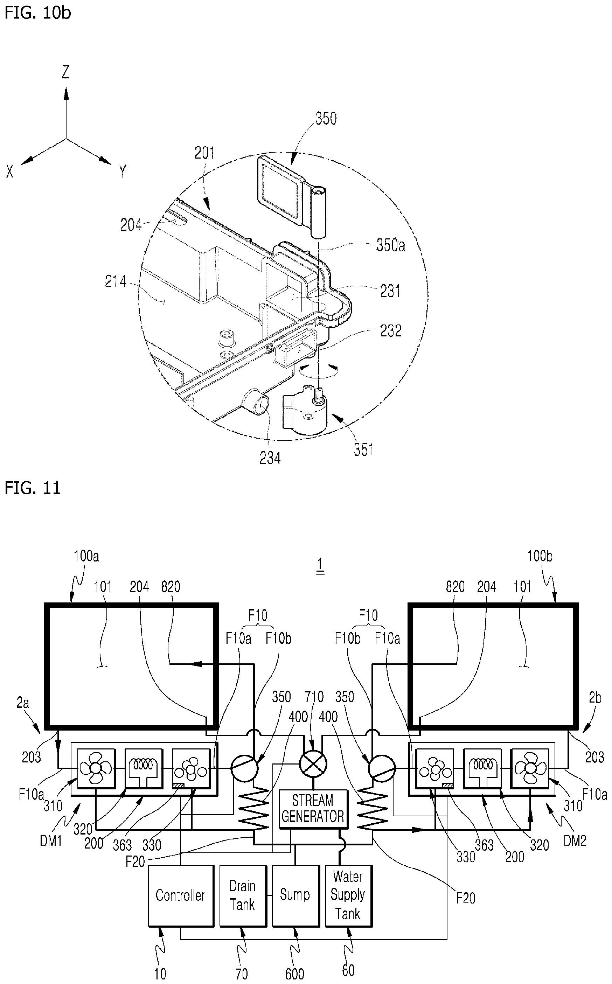

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air.

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (A) are regularly arranged, and may function as dehumidification by adsorbing the moisture in the air.

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of the blowing part 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed.

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user.

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may reproduce bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoes care device 1 according to one embodiment of the present invention, steam generated by the steam generator 700 is supplied to the accommodation space 101 of the inner cabinet 100, and to this end, the shoes care device 1 includes a steam inlet 204.

The steam inlet 204 forms an inlet through which steam is supplied to the accommodation space 101 of the inner cabinet 100.

In the shoes care device according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam generator 700 and the steam valve 710 are disposed in a lower part, the distance from the steam generator 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam generator 700 to the steam inlet 204

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the blowing part 310, the heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 (the dehumidifying material 331) are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration path F20 forms a passage through which air and/or condensed water inside the shoes care device moves.

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoes care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path F10a and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

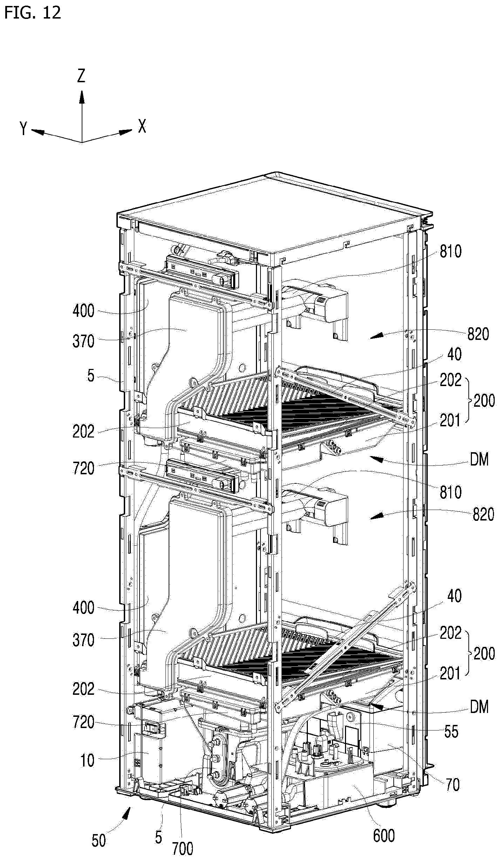

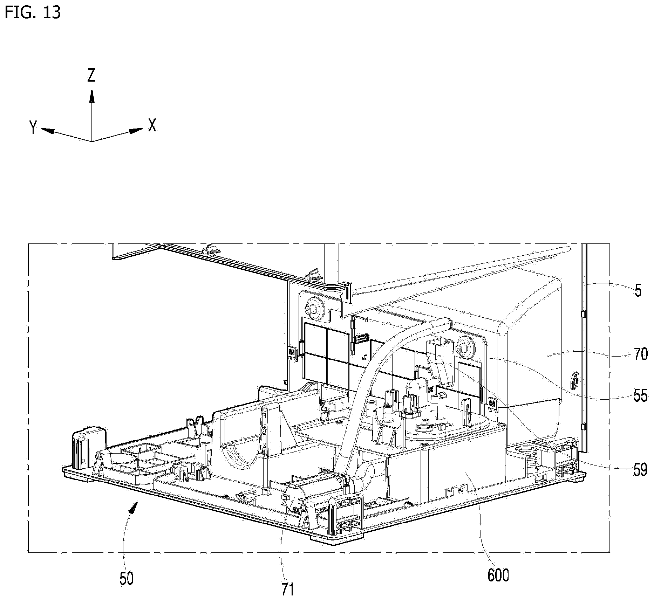

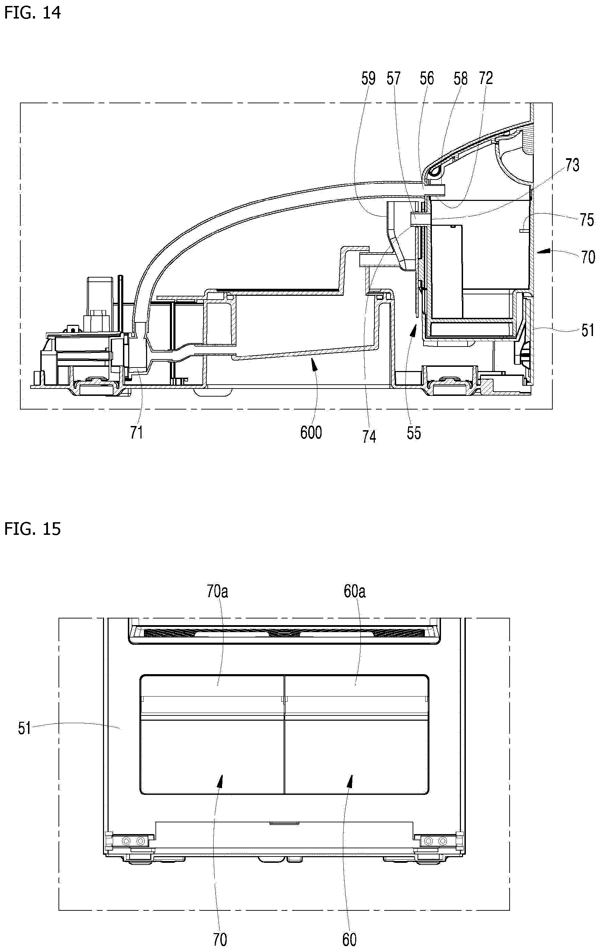

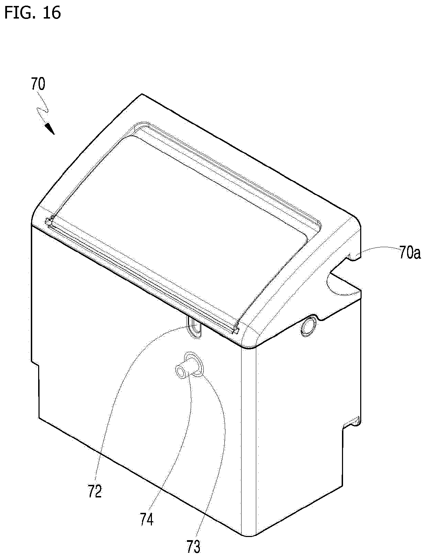

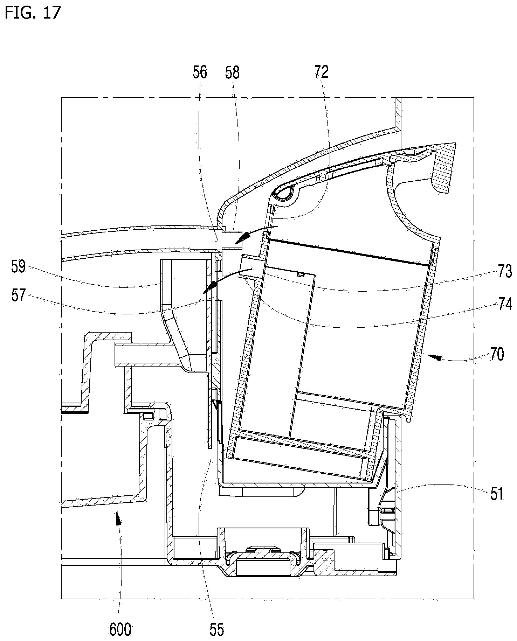

FIG. 12 is a perspective view illustrating a modified example of a state in which the door 30, the outer cabinet 20, and the inner cabinet 100 are removed from the shoes care device 1 according to one embodiment of the present invention. FIG. 13 is a perspective view illustrating a part of the machine room 50 of the shoes care device 1 illustrated in FIG. 12. FIG. 14 is a cross-sectional view illustrating the sump 600 and the drain tank 70 in more detail in the part of the machine room 50 of the shoes care device 1 illustrated in FIG. 12. FIG. 15 is a view illustrating a front surface of the machine room 50 in the shoes care device 1 according to one embodiment of the present invention. FIG. 16 is a perspective view illustrating the drain tank 70 in more detail in the shoes care device 1 according to one embodiment of the present invention. FIG. 17 is a view exemplarily illustrating a detachable state of the drain tank 70 in the shoes care device 1 according to one embodiment of the present invention.

The shoes care device 1 according to one embodiment of the present invention may include the inner cabinet 100, the connection path F10, the blowing part 310, a dehumidifying part 330, the heating part 320, the regeneration path F20, the sump 600, and the drain tank 70. In this case, the drain tank 70 may include a first drain inlet hole 72 and a first water level limit hole 73.

The inner cabinet 100 is a part in which an accommodation space 101 for accommodating the shoes is formed, and the outlet 203 and a nozzle 820 may be disposed inside.

The connection path F10 forms a flow path through which air from the accommodation space 101 is discharged from the inner cabinet 100 and then introduced back into the accommodation space 101, and the air inside the inner cabinet 100 is absorbed to the module chamber 210 and ventilated and is dehumidified in the process of passing through the dehumidifying part 330.

The blowing part 310 is a part disposed in the connection path F10 and configured to ventilate air, and may inhale air from the inner cabinet 100 by the operation of the blowing part 310 and ventilate the inhaled air from the connection path F10.

The dehumidifying part 330 is a part disposed in the connection path F10 and configured to dehumidify air, and with the heating of the dehumidifying material 331, the dehumidifying material 331 may be regenerated in a state where moisture adsorbed on the dehumidifying material 331 is separated to perform a dehumidifying function.

The heating part 320 is a part disposed in the connection path F10 and configured to heat air, and the dehumidifying part 330 may be heated by heating the air supplied to a dehumidifying material portion.

The regeneration path F20 is a flow path formed to allow the air heated by the heating part 320 to move and branched from the connection path F10, and may form a passage through which air and/or condensed water through the dehumidifying part 330 moves.

The module chamber 210 is a part configured to ventilate air from the accommodation space 101, disposed on a path of air through which the dehumidifying part 330 is ventilated and capable of heating the dehumidifying part 330.

In this case, with the heating of the dehumidifying part 330, the dehumidifying part 330 may be generated in a state where the moisture adsorbed to the dehumidifying part 330 is separated to perform the dehumidifying function.

To this end, the module chamber 210 may include the dehumidifying part 330, the blowing part 310, the heating part 320, the damper 350, etc.

Specifically, the module chamber 210 may form a part of the regeneration path F20 branched and ventilated from the connection path F10 in which air circulates between the outlet 203 and the nozzle 820 and the connection path F10 passing through the dehumidifying part 330.

Accordingly, the air in the accommodation space 101 may move to the connection path F10 or the regeneration path F20 while the air is ventilated to the module chamber 210 to pass through the dehumidifying part 330.

The controller 10 is a part that controls the shoes care device 1, and control the module chamber 210 to selectively open and close the connection path F10 and the regeneration path F20 depending on whether the dehumidifying part 330 is heated.

That is, the controller 10 may selectively open and close the connection path F10 and the regeneration path F20 depending on whether the dehumidifying part 330 is regenerated.

The connection path F10 is a part where air is circulated between the outlet 203 and the nozzle 820, and the outlet 203 may form an inlet of the connection path F10, and the nozzle 820 may form an outlet of the connection path F10.

That is, the connection path F10 may be an air flow path of a path through which the air inside the inner cabinet 100 is dehumidified while being sucked and ventilated into the module chamber 210 to pass through the dehumidifying part 330 and is supplied back into the inner cabinet 100.

As described above, the shoes care device 1 according to one embodiment of the present invention can not only collect moisture and bacteria in the air ventilated by placing the dehumidifier 330 in the module chamber 210 but also heat and regenerate the dehumidifying part 330, thus appropriately maintaining the performance for a shoes treatment all the times.

In addition, the shoes care device 1 according to one embodiment of the present invention can form the connection path F10 through which air circulates between the outlet 203 and the nozzle 820 respectively disposed inside the inner cabinet 100, thus preventing the air used for dehumidification and deodorization of shoes from being exposed to the user.

The sump 600 has a structure capable of accommodating water. The sump 600 is configured to accommodate condensed water generated by the shoes care device 1 inside. The sump 600 is configured to receive the condensed water condensed in the condenser 400.

In this case, the condensed water collected in the sump 600 may be moved to the drain tank 70 through a separate pipe, etc., may be immediately discharged to the outside of the shoes care device 1, or may be reused in another component.

The drain tank 70 may be configured to store water discharged from the shoes care device 1 inside. The drain tank 70 may store water condensed inside the shoes care device 1. The drain tank 70 may be configured to store water drained from the sump 600.

The first drain inlet hole 72 is a part formed in an upper end such that the water drained from the sump 600 may be introduced, and may be a passage through which water flows into the drain tank 70. Since the first drain inlet hole 72 is formed in an upper end of the drain tank 70, water may be accommodated inside the drain tank 70 up to the height at which the first drain inlet hole 72 is formed.

However, when water is accommodated in the drain tank 70 above the height at which the first drain inlet hole 72 is formed, the water may flow to the outside of the drain tank 70. Specifically, when water flows into the drain tank 70 to a height close to the height at which the first drain inlet hole 72 is formed, the water may overflow when the drain tank 70 shakes or tilts. Accordingly, a water level needs to be appropriately adjusted at a height lower than the height at which the first drain inlet hole 72 is formed.

Accordingly, it is necessary for the user to confirm that the water is accommodated at a predetermined level or higher inside the drain tank 70 and discharge the water accommodated in the drain tank 70.

However, since the user's carelessness or difficulty in confirming an inner level of the drain tank 70 causes the water not to be appropriately discharged, the water may flow to the outside of the drain tank 70 and then overflow.

In this case, since the water overflowing outside the drain tank 70 may leak into an unintended part, the function of each component of the shoes care device 1 may be degraded, or problems of contamination and bacterial growth may occur.

Accordingly, when the water is accommodated in the drain tank 70 at a predetermined height or more, the water level inside the drain tank 70 needs to be appropriately regulated by introducing the water back into the sump 600.

To this end, the first water level limit hole 73 is a part formed in a lower part of the first drain inlet hole 72 and connected to the sump 600, and when the water level inside the drain tank 70 reaches the height at which the first drain inlet hole 72 is formed, water may be reintroduced into the sump 600.

That is, as illustrated in FIG. 14, the water flowing into the drain tank 70 through the first drain inlet hole 72 may be accommodated in the drain tank 70 up to the height at which the first drain inlet hole 72 is formed. Further, when the water level inside the drain tank 70 reaches the height at which the first drain inlet hole 72 is formed, the water may be discharged through the first drain inlet hole 72 and the water level inside the drain tank 70 may not exceed the height at which the first drain inlet hole 72 is formed.

In this way, in the shoes care device 1 according to one embodiment of the present invention, since the first water level limit hole 73 connected to the sump 600 is formed in a lower part of the first drain inlet hole 72 connected again to the sump 600 and allowing the water to flow into the drain hole 70, water above a water level that can be accommodated in the drain tank 70 may be discharged to the sump 600. This can prevent leakage to an unintended part in the process of discharging water through the drain tank 70.

The shoes care device 1 according to one embodiment of the present invention may further include the machine room 50 provided in the lower side of the inner cabinet 100, and the sump 600 and the drain tank 70 may be disposed in the machine room 50.

That is, as illustrated in FIGS. 12 and 13, the machine room 50 may be provided in the lower side of the inner cabinet 100 to accommodate some components constituting the shoes care device 1. In this case, the components accommodated in the machine room 50 may be fixed to the main frame 5 or may be fixed to the inner cabinet 100 or the inner cabinet 20.

In this case, the inner cabinet 100 and the machine room 50 may form separate spaces inside the shoes care device 1. Further, the controller 10, the sump 600, the steam generator 700, and the steam valve 710 may be accommodated in the machine room 50. Furthermore, the machine room 50 may be configured to accommodate the water supply tank 60 and the drain tank 70.

Accordingly, since the inner cabinet 100 may form a space that mainly accommodates the articles (the shoes S) to be managed, only the minimum components necessary for functionality is placed inside.

In this way, in the shoes care device 1 according to one embodiment of the present disclosure, since the sump 600 and the drain tank 70 are disposed in the machine room 50 below the inner cabinet 100, water can be discharged naturally from the shoes care device 1 in a downward direction, and the components arranged in the inner cabinet 100 can be minimized.

The shoes care device 1 according to one embodiment of the present invention further includes a water pump 71 that presses the water drained from the sump 600 to the first drain inlet hole 72, and the sump 600 may be installed on a bottom surface of the machine room 50.

That is, as illustrated in FIGS. 12 to 14, a water pump (a second water pump 71) may be connected to the drain tank 70 to discharge water from the sump 600 to the drain tank 70. The drain pipe 70 for moving water and the second water pump 71 may be connected by a pipe, a hose, etc.

According to the pressing of water through the water pump 71, there is no need for the drain tank 70 to be disposed lower than the sump 600. That is, even if the sump 600 is disposed lower than the drain tank 70, water may be smoothly discharged from the sump 600 to the drain tank 70.

Meanwhile, the sump 600 for accommodating the condensed water on a water discharge path is preferably disposed at the lowest part in the shoes care device 1. In this case, the drain tank 70 may be preferably placed at the lowest part in the shoes care device 1. However, given that the user has to frequently check the level of the drain tank 70 and discharge the water from the drain tank 70 to the outside in some cases, there may be some limitations in arranging the drain tank 70 in a low position.

Therefore, the sump 600 is disposed in the lowest position in the shoes care device 1, and when the drain tank 70 is disposed in a higher position than the sump 600, water is discharged smoothly through the pressing of the water pump 71.

As described above, since the shoes care device 1 according to one embodiment of the present invention presses water from the sump 600 installed on the bottom surface of the machine room 50 and moves the pressed water to the drain tank 70, the sump 600 that accommodates the condensed water inside the shoes care device 1 can be disposed in the lowest position and drained smoothly.

In the shoes care device 1 according to one embodiment of the present invention, the drain tank 70 may be installed on the first wall 51 formed on the front surface of the machine room 50 and exposed to the outside of the first wall 51.

Specifically, as illustrated in FIG. 15, the machine room 50 may include the first wall 51. In that case, the first wall 51 may form the front wall surface of the machine room 50.

The first wall 51 may be erected in the vertical direction or may be erected in a substantially vertical direction. In one embodiment, the first wall 51 may form a wall surface orthogonal to or inclined with the first direction (X direction).

The drain tank 70 may be coupled to the machine room 50 to get exposed from the outside of one wall surface of the machine room 50. Specifically, the drain tank 70 may be disposed in front of the machine room 50.

The drain tank 70 may form one wall surface of the machine room 50 along with the first wall 51. When the first wall 51 forms the front surface of the machine room 50, the drain tank 70 may be exposed from the front of the machine room 50 and may be coupled to the machine room 50 to get exposed from the outside of the first wall 51.

In this way, in the shoes care device 1 according to one embodiment of the present invention, since the drain tank 70 is exposed outwards from the first wall 51 on the front surface of the machine room 50, the drain tank 70 can be easily checked even if the user does not check the inside of the machine room 50.

In the shoes care device 1 according to one embodiment of the present invention, the drain tank 70 may be detachably installed on the first wall 51.

That is, as illustrated in FIG. 17, the drain tank 70 may be separated from the machine room 50 in an outward direction of the first wall 51. In this case, for the easy attachment and detachment of the drain tank 70, the handle 70a of the drain tank 70 may be formed on the outer surface of the drain tank 70.

Accordingly, when the water stored in the drain tank 70 needs to removed, the user may separate the drain tank 70 from the first wall 51 and then discard the water. In addition, even if the drain tank 70 needs to be cleaned or inspected and replaced, a predetermined operation can be performed after separating the drain tank 70 from the first wall 51.

In this way, in the shoes care device 1 according to one embodiment of the present invention, since the drain tank 70 is detached from the first wall 51 on the front surface of the machine room 50, the water stored in the drain tank 70 can be easily discharged and the drain tank 70 can be easily managed.

In the shoes care device 1 according to one embodiment of the present invention, the drain tank 70 may be accommodated in an accommodation housing 55 formed to be recessed from the first wall 51 to the inside of the machine room 50.

That is, as illustrated in FIGS. 14 and 17, the accommodation housing 55 may be coupled to the rear of the first wall 51 of the machine room 50. The accommodation housing 55 may be formed to surround a lower surface and a rear surface of the drain tank 70 to support the drain tank 70 mounted on the first wall 51.

Specifically, since the accommodation housing 55 covers the rear of the first wall 51 while separating the drain tank 70, the inside of the machine room 50 can be exposed at a minimum in the front of the shores care device 1.

As described above, in the shoes care device 1 according to one embodiment of the present invention, since the accommodation housing 55 for accommodating the drain tank 70 is formed from the first wall 51 to the inside of the machine room 50, the drain tank 70 can be installed more stably, and the inside of the machine room 50 can be exposed at a minimum even when the drain tank 70 is separated.

In the shoes care device 1 according to one embodiment of the present invention, the accommodation housing 55 may include a second drain inlet hole 56 formed in an upper end of the rear surface thereof and connectable to the first drain inlet hole 72, and a second water level limit hole 57 formed under the second drain inlet hole 56 and connectable to the first water level limit hole 73.

That is, as illustrated in FIGS. 14 and 17, the second drain inlet hole 56 formed in the accommodation housing 55 may be connected to the first drain inlet hole 72 formed in the drain tank 70 to form a passage through which water flows from the sump 600 to the drain tank 70.

In addition, the second water level limit hole 57 formed in the accommodation housing 55 may be connected to the first water level limit hole 73 formed in the drain pipe 70 to form a passage through which water flows back into the sump 600.

As described above, in the shoes care device 1 according to one embodiment of the present invention, since the second drain inlet hole 56 and the second water level limit hole 57 are formed in the accommodation housing 55, the drain tank 70 accommodated in the accommodation housing 55 can be smoothly connected to the sump 600.

In the shoes care device 1 according to one embodiment of the present invention, the accommodation housing 55 may further include a drain inlet 58 protruding from the second drain inlet hole 56 and inserted into the first drain inlet hole 72, and the drain tank 70 may further include a water level limit pipe 74 protruding from the first water level limit hole 73 and inserted into the second water level limit hole 57.

That is, a drain inlet pipe 58 may protrude from the second drain inlet hole 56 and be inserted into the first drain inlet hole 72. Furthermore, the water level limit pipe 74 may protrude from the first water level limit hole 73 and be inserted into the second water level limit hole 57.

Accordingly, when the drain tank 70 is mounted in the accommodation housing 55, the drain inlet pipe 58 can be easily inserted into the first drain inlet hole 72, and the water level limit pipe 74 can be easily inserted into the second water level limit hole 57.

That is, as illustrated in FIG. 17, the user mounts the drain tank 70 in the accommodation housing 55 from the front surface of the machine room 50 while the drain tank 70 is partially inclined.

In this case, when both the drain inlet pipe 58 and the water level limit pipe 74 protrude from the accommodation housing 55, in the inclined drain tank 70, it may be difficult for the first drain inlet hole 72 and the first water level limit hole 73 to be arranged in the drain inlet pipe 58 and the water level limit pipe 74.

Conversely, even if both the drain inlet pipe 58 and the water level limit pipe 74 protrude from the drain tank 70, in the inclined drain tank 70, it may be difficult for the drain inlet pipe 58 and the water level limit pipe 74 to be arranged with the second drain inlet hole 56 and the second water level limit hole 57.

Accordingly, as the drain inlet pipe 58 and the water level limit pipe 74 is made to protrude piece by piece in the accommodation housing 55 and the drain tank 70, respectively, the drain tank 70 needs to be easily detached from the accommodation housing 55.

In the shoes care device 1 according to one embodiment of the present invention described above, since the drain inlet pipe 58 protrudes from the second drain inlet hole 56 and the water level limit pipe 74 protrudes from the first water level limit hole 73, the drain tank 70 can be easily detached from the accommodation housing 55.

In the shoes care device 1 according to one embodiment of the present invention, the accommodation housing 55 may cover a rear side of the second water level limit hole 57 to accommodate water discharged from the second water level limit hole 57, and may further include a connection cover 59 connected to the sump 600.

That is, as illustrated in FIG. 14, the water discharged through the second water level limit hole 57 may not directly flow into the sump 600, and a part thereof may be temporarily accommodated in the connection cover 59.

In this case, since the inside of the connection cover 59 has a predetermined space, water may be partially accommodated by the volume of the corresponding space. At the same time, since water may flow to the sump 600 through a lower part of the connection cover 59, the water reintroduced from the drain tank 70 to the sump 600 may not move rapidly, but may be partially buffered and moved from the connection cover 59.

As described above, in the shoes care device 1 according to one embodiment of the present invention, since the connection cover 59 receives the water discharged from the second water level limit hole 57 and transmits the water to the sump 600, a buffer space for water reintroduced from the drain tank 70 to the sump 600 may be partially formed.

In the shoes care device 1 according to one embodiment of the present invention, a part of the upper surface of the sump 600 may protrude upwards, and the connection cover 59 may be connected to a protruding part of the upper surface of the sump 600.

That is, since the lower part of the connection cover 59 is arranged with the protruding part of the upper surface of the sump 600, water may be moved from the connection cover 59 to the sump 600 through the corresponding part.

As described above, the sump 600 and the drain tank 70 may have different arrangement heights, and in particular, when the sump 600 is disposed on the bottom surface of the machine room 50, the drain tank 70 may be disposed higher than the sump 600.

Accordingly, in order to facilitate the movement of water reintroduced from the drain tank 70 to the sump 600, a protruding part may be formed on the upper surface of the sump 600 at a height corresponding to the lower part of the connection cover 59.

Specifically, as illustrated in FIGS. 13 and 14, when the lower part of the connection cover 59 is assembled with the protruding part of the upper surface of the sump 600, a separate pipe is not required to improve convenience and structural simplicity.

As described above, in the shoes care device 1 according to one embodiment of the present invention, since a part of the upper surface of the sump 600 protrudes and is connected to the connection cover 59, the accommodation housing 55 and the sump 600 can be more easily coupled to each other.

The shoes care device 1 according to one embodiment of the present invention may further include a water level sensor 75 installed to sense a level of water stored in the drain tank 70.

That is, the water level sensor 75 is installed inside the drain tank 70 to sense the level of water stored in the drain tank 70. Further, when the water level sensed by the water level sensor 75 reaches a set height, the user can recognize that the drain tank 70 needs to be managed by informing the user of this state.

In this case, the water level sensor 75 may include a magnetic sensor etc., but the present invention is not limited thereto, and the water level sensor 75 may include various components for sensing the water level.

On the other hand, when the water level sensor 75 does not operate normally due to malfunctioning, etc., a certain amount of water may be restricted by allowing water to flow back from the drain tank 70 to the sump 600 as described above.

In addition, when the water flows back from the drain tank 70 to the sump 600 and the water level capacity of the sump 600 reaches the limit, the operation of the shoes care device 1 may be controlled to stop due to a drainage error.

As described above, since the shoes care device 1 according to one embodiment of the present invention is provide with the water level sensor 75 that senses the water level inside the drain tank 70, it can be easily recognized whether the water needs to be discharged from the drain tank 70.

The shoes care device 1 according to one embodiment of the present invention further includes the condenser 400 that forms a part of the regeneration path F20, and allows air to be introduced and condensed along the regeneration path F20, and the condensed water condensed in the condenser 400 may be introduced and accommodated in the sump 600.

Specifically, the moisture separated from the dehumidifying part 330 may be condensed after moving to the condenser 400 with air moving along the regeneration path F20. In addition, the condensed water condensed in the condenser 400 may move to the sump 600 through the regeneration path F20, and may be collected from the lower part of the sump 600 and then discharged.

The condenser 400 may be configured to be entirely metallic. The condenser 400 may be made of a metal having excellent thermal conductivity. The condenser 400 may be made of aluminum.

As described above, since the shoes care device 1 according to one embodiment of the present invention includes the condenser 400 that generates condensed water on the regeneration path F20, moisture generated during the regeneration process of the dehumidifying part 330 may be condensed and accommodated in the sump 600.

In the shoes care device 1 according to one embodiment of the present invention, the condenser 400 may be disposed at a position relatively higher than the sump 600.

Accordingly, since the condensed water condensed inside the condenser 400 may flow into the sump 600 while being naturally lowered by gravity, stream can be effectively condensed and discharged when the dehumidifying material 331 is regenerated.

As described above, since the shoes care device 1 according to one embodiment of the present invention is configured to have a lower height from the condenser 400 to the sump 600, the condensed water can move smoothly between the condenser 400 and the sump 600.

The shoes care device 1 according to one embodiment of the present invention may further include the water supply tank 60 installed on the first wall 51 parallel to the drain tank 70 and exposed to the outside of the first wall 51.

Specifically, the water supply tank 60 may be configured to store water supplied into the shoes care device 1 inside. The water supply tank 60 may be configured to store water supplied to the steam generator 700.

In order to supply water from the water supply tank 60 into the shoes care device 1, the water pump 61 (the first water pump 61) may be connected to the water supply tank 60. The water supply tank 60 for moving water and the first water pump 61 may be connected by a pipe, a hose, etc.

Specifically, as illustrated in FIG. 15, the water supply tank 60 may form one wall surface of the machine room 50 along with the first wall 51. When the first wall 51 forms the front surface of the machine room 50, the water supply tank 60 may be exposed from the front of the machine room 50 and may be coupled to the machine room 50 to get exposed from the outside of the first wall 51.

In this way, in the shoes care device 1 according to one embodiment of the present invention, since the water supply tank 60 is exposed outwards from the first wall 51 on the front surface of the machine room 50, the water supply tank 60 can be easily checked even if the user does not check the inside of the machine room 50.

In the shoes care device 1 according to one embodiment of the present invention, the water supply tank 60 may be detachably installed on the first wall 51.

That is, the water supply tank 60 may be separated from the machine room 50 in the outer direction of the first wall 51. In this case, in order to facilitate the attachment and detachment of the water supply tank 60, the handle 60a of the water supply tank 60 may be formed on the outer surface of the water supply tank 60.

Accordingly, when water needs to be supplied to the water supply tank 60, the user may separate the water supply tank 60 from the first wall 51 and then fill the water supply tank 60 with water. In addition, even if the water supply tank 60 needs to be cleaned or inspected and replaced, the water supply tank 60 can be separated from the first wall 51, and a predetermined operation can be then performed.

In this way, in the shoes care device 1 according to one embodiment of the present invention, since the water supply tank 60 is detached from the first wall 51 on the front surface of the machine room 50, the water can be easily supplied to the water supply tank 60 and the water supply tank 60 can be easily managed.

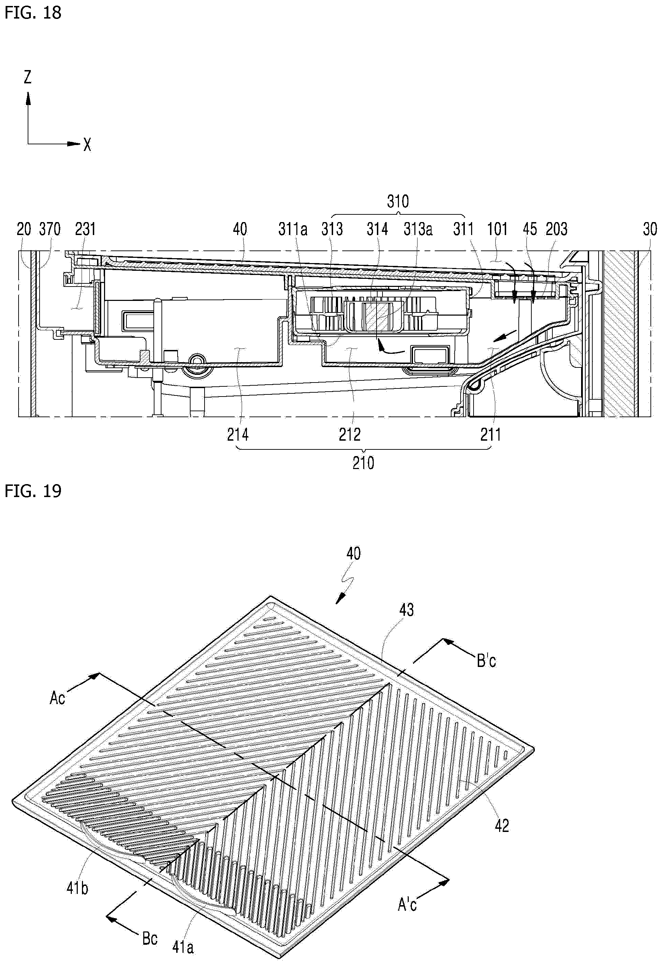

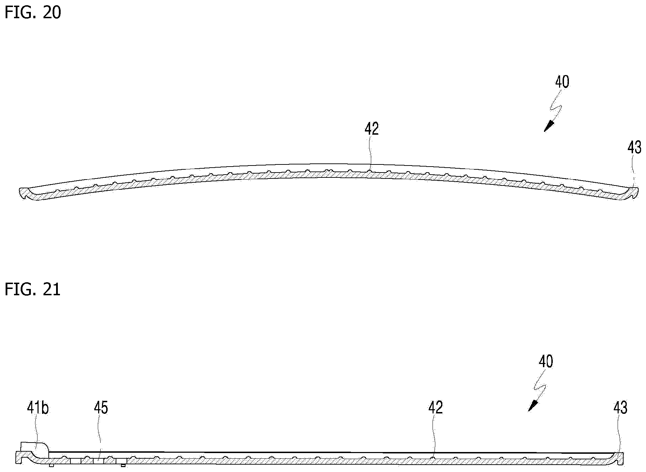

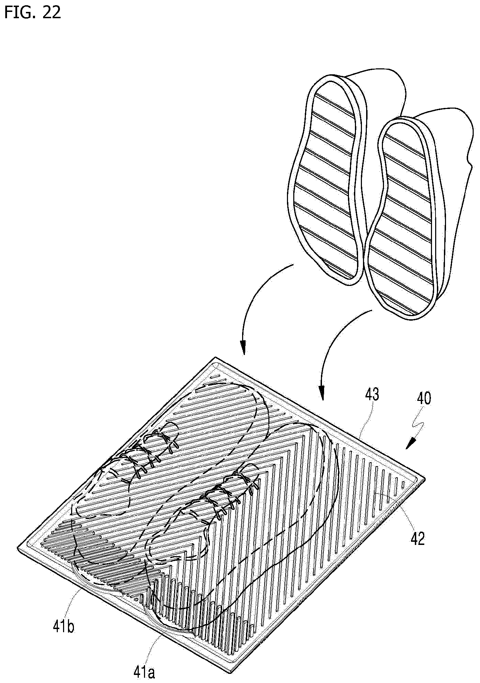

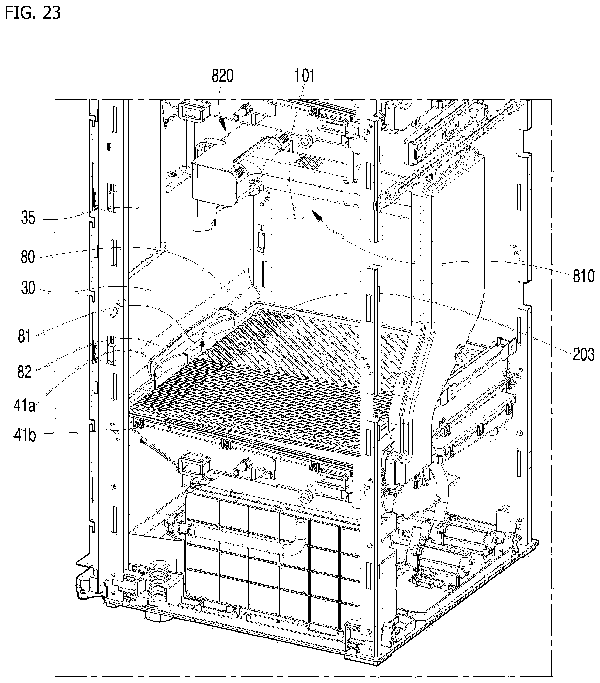

FIG. 18 is a cross-sectional view illustrating the module chamber 210 and the main shelf 40 in more detail in the shoes care device 1 according to one embodiment of the present invention. FIG. 19 is a perspective view illustrating the main shelf 40 in the shoes care device 1 according to one embodiment of the present invention. FIG. 20 is a cross-sectional view taken along Ac-A'c of the main shelf 40 illustrated in FIG. 19. FIG. 21 is a cross-sectional view taken along Bc-B'c of the main shelf 40 illustrated in FIG. 19. FIG. 22 is a view exemplarily illustrating a state in which the shoes are settled on the main shelf 40 in the shoes care device 1 according to one embodiment of the present invention. FIG. 23 is a view illustrating the inside of the shoes care device 1 according to one embodiment of the present invention in a state in which the door 30 is closed.

The shoes care device 1 according to one embodiment of the present invention may include the inner cabinet 100, the outlet 203, the connection path F10, the blowing part 310, the dehumidifying part 330, and the main shelf 40.

The outlet 203 is a part formed on one side of the inner cabinet 100 and capable of sucking the air in the accommodation space 101, and the air inside the inner cabinet 100 may move to the connection path F10 through the outlet 203. In this case, the outlet 203 may be formed on the bottom surface of the inner cabinet 100.

The main shelf 40 is a part installed to cover the bottom surface of the inner cabinet 100 and capable of settling the shoes on the top surface thereof, and in the accommodation space 101 of the inner cabinet 100, the shoes can be treated while being settled on the main shelf 40.

Since the bottom surfaces of the shoes has a relatively high degree of contamination, when the bottom surfaces of the shoes are in direct contact with the inner cabinet 100, the inner cabinet 100 may be contaminated.

In addition, in order to effectively treat the bottom surfaces of the shoes with the relatively high degree of contamination, the bottom surfaces of the shoes are preferably prevented from getting in contact with the bottom surface of the inner cabinet 100 as much as possible.

Accordingly, the shoes need to be treated while preventing the bottom surface of the inner cabinet 100 from getting in direct contact with the bottom surfaces of the shoes.

In this way, in the shoes care device 1 according to one embodiment of the present invention, since the shoe is settled on the upper surface of the main shelf 40 covering the bottom surface of the inner cabinet 100, the inner cabinet 100 can be easily managed by preventing contamination by the bottom surfaces of the shoes.

In the shoes care device 1 according to one embodiment of the present invention, the main shelf 40 may be installed to be detachable from the bottom surface of the inner cabinet 100. That is, the main shelf 40 may be separated in an outer direction of the inner cabinet 100.

Accordingly, when foreign substances are accumulated on the main shelf 40 and need to be removed or when the contaminants on the main shelf 40 need to be removed, the user can separate the main shelf 40 from the inner cabinet 100 and perform cleaning and washing.

In addition, even when the main shelf 40 needs to be inspected and replaced, a predetermined operation may be performed after the main shelf 40 is separated from the inner cabinet 100.

In this way, in the shoes care device 1 according to one embodiment of the present invention, since the main shelf 40 is detached from the inner cabinet 100, the main shelf 40 can be easily managed.

In the shoes care device 1 according to one embodiment of the present invention, the main shelf 40 may have a main isolation rib 42 protruding upwards from an upper surface thereof.

That is, as illustrated in FIG. 22, since the main isolation rib 42 protrudes from the upper surface of the main shelf 40, the bottom surfaces of the shoes may be settled on the main isolation rib 42.

When the shoes are settled on the upper surface of the planar main shelf 40, the upper surface of the main shelf 40 and the bottom surfaces of the shoes are mostly in close contact with each other. In this case, since it is difficult for air to get in contact with the bottom surfaces of the shoes, there may be any difficulty treating the bottom surfaces of the shoes. Further, when water is present between the main shelf 40 and the shoes, it may be difficult to move or evaporate such water.

Accordingly, a separation space through which air or water may move may be preferably formed between the upper surface of the main shelf 40 and the bottom surfaces of the shoes.

In this way, in the shoes care device 1 according to one embodiment of the present invention, since the main isolation rib 42 is formed on the upper surface of the main shelf 40, the separation space for a movement of air or water may be formed between the upper surface of the main shelf 40 and the bottom surfaces of the shoes.

In the shoes care device 1 according to one embodiment of the present invention, the main isolation rib 42 may be formed in an oblique direction between the front surface and the rear surface of the inner cabinet 100.

That is, as illustrated in FIGS. 19 and 22, the main isolation rib 42 may be formed in the oblique direction so as not to get parallel to the horizontal or vertical direction of the planar main shelf 40.

In general, the protrusion structure for preventing slipping may be formed on the bottom surfaces of the shoes, and such a protrusion structure may be formed on the bottom surfaces of the shoes along a transverse direction and/or a vertical direction.

Accordingly, when the main isolation rib 42 is formed horizontally or vertically on the main shelf 40, a relatively large part may be in parallel with a direction of the protrusion structure formed on the bottom surfaces of the shoes.

In this case, when the protrusion structure formed on the bottom surfaces of the shoes engages with the main isolation rib 42, the shoes and the upper surfaces of the main shelf 40 come into relatively more contact, and as described above, the larger contact area may result in some disadvantages in terms of the function and maintenance of the shoes care device 1.

Accordingly, the main isolation rib 42 may be preferably formed in the oblique direction such that the protrusion structure formed on the bottom surfaces of the shoes are not parallel to the main isolation rib 42 as much as possible.

As described above, in the shoes care device 1 according to one embodiment of the present invention, since the main isolation rib 42 is formed in the oblique direction, an area in which the upper surface of the main shelf 40 and the bottom surfaces of the shoes come into contact can be further reduced.

In the shoes care device 1 according to one embodiment of the present invention, the main isolation rib 42 may be formed symmetrically from a central part of the inner cabinet 100 toward opposite sides.

Since a pair of shoes are generally provided, the pair of shoes used are exposed to the relatively same environment as described above. Accordingly, the treatment required for the pair of shoes may also be virtually the same as described above.

Accordingly, preferably, the main isolation rib 42 is also formed symmetrically from the central part toward opposite sides in order to simultaneously settle the pair of shoes on one main shelf 40 and perform a uniform treatment thereof.

In this way, since the main isolation rib 42 is formed in a symmetrical shape, the shoes care device 1 according to one embodiment of the present invention may be most efficient in placing the pair of shoes on opposite sides of the main shelf 40.

In the shoes care device 1 according to one embodiment of the present invention, the bottom surface of the inner cabinet 100 is inclined downwardly toward the front thereof, and the main isolation rib 42 may be formed to face a front surface of the inner cabinet 100 from the central part of the bottom surface of the inner cabinet 100 toward opposites sides.

Since the door 30 is generally installed on the front side of the inner cabinet 100, and the front side of the inner cabinet 100 is relatively less vulnerable to moisture, water generated inside the inner cabinet 100 may be preferably induced.

Accordingly, the bottom surface of the inner cabinet 100 needs to be inclined downwardly toward the front side thereof. In this case, the main shelf 40 installed on the bottom surface of the inner cabinet 100 may also be inclined downwardly toward the front side thereof.

In addition, water staying on the upper surface of the main shelf 40 is preferably moved toward the front side of the inner cabinet 100.

Accordingly, with the formation of the main isolation rib 42 to face the front surface of the inner cabinet 100 from the central part of the bottom surface of the inner cabinet 100 toward opposite sides, water can be induced to the front side of the inner cabinet 100 along a formation direction of the main isolation rib 42.

In this way, in the shoes care device 1 according to one embodiment of the present invention, since the main isolation rib 42 is formed so that the water on the main shelf 40 moves toward the front side of the inner cabinet 100, water can be prevented from accumulating in the certain part on the main shelf 40.

In the shoes care device 1 according to one embodiment of the present invention, the main shelf 40 may be formed to penetrate a part covering the outlet 203. That is, as illustrated in FIGS. 18 and 19, a plurality of holes 45 as vertically penetrated may be formed in the main shelf 40.

The outlet 203 is formed in the module housing 200. The outlet 203 is formed to communicate with the accommodation space 101 of the inner cabinet 100, and the air of the accommodation space 101 of the inner cabinet 100 forms the inlet of the module housing 200 absorbed into the module housing 200. The outlet 203 may be disposed below the main shelf 40.

In this case, the main shelf 40 may be formed so as not to prevent the air in the accommodation space 101 from getting sucked into the outlet 203. To this end, when the main shelf 40 is formed in a plate shape, the plurality of holes 45 may be formed in the main shelf 40 to move air.

In addition, water moved from the upper part of the main shelf 40 may also be introduced into the outlet 203 through the hole 45 and then discharged. Therefore, water can be smoothly discharged without having to form a separate outlet configured so as to discharging water generated inside the inner cabinet 100.

As described above, in the shoes care device 1 according to one embodiment of the present invention, since a part of the main shelf 40 corresponding to the upper part of the outlet 203 is configured to penetrate, the water moved on the main shelf 40 may be discharged through the outlet 203.

In the shoes care device 1 according to one embodiment of the present invention, the outlet 203 may be formed on the front side of the bottom surface of the inner cabinet 100, and the main shelf 40 may be inclined downwardly toward the front side of the inner cabinet 100.

As described above, since water from the inner cabinet 100 is preferably induced ahead, the outlet 203 may be disposed relatively close to the door 30 in the module cover 202. That is, the outlet 203 may be disposed relatively in the front side of the module cover 202.

The upper surface of the module cover 202 may be inclined downwardly toward the outlet 203. That is, a part of the module cover 202 in which the outlet 203 is formed may be formed to be the lowest. Accordingly, when there is water on the module cover 202 or the main shelf 40, such water may flow along the surface of the module cover 202 by gravity and flow into the inlet 203.

Accordingly, since the main shelf 40 is also inclined downwardly toward the front side of the inner cabinet 100, water may flow into the inlet 203 along the upper surface of the main shelf 40 by gravity.

In the shoes care device 1 according to one embodiment of the present invention, since the main shelf 40 is inclined downwardly toward the front side of the inner cabinet 100 in which the outlet 203 is formed, water moved on the main shelf 40 can move smoothly toward the outlet 203.

In the shoes care device 1 according to one embodiment of the present invention, guide ribs 41a and 41b may be formed on the upper surface of the main shelf 40 to guide the position of the shoes.

Specifically, as illustrated in FIGS. 19 and 22, the guide ribs 41a and 41b may protrude from the main shelf 40. The guide ribs 41a and 41b are meant to guide the user to the position of the shoes, and a part of the shoes (particularly, a heel part) is fitted to the guide ribs 41a and 41b and the positions of the shoes for treatment may be arranged.

The guide ribs 41a and 41b are formed to correspond to a position of the nozzle 820 such that the nozzle 820 can be smoothly inserted into the upper surface of the shoes arranged to fit in the guide ribs 41a and 41b.

In this way, in the shoes care device 1 according to one embodiment of the present invention, since the guide ribs 41a and 41b are formed on the main shelf 40 to guide the positions of the shoes, the position in which the shoes are settled for treatment can be properly guided.

In the shoes care device 1 according to one embodiment of the present invention, a pair of guide ribs 41a and 41b may be formed in a symmetrical manner from the central part of the inner cabinet 100) toward opposite sides.

As described above, when the guide ribs 41a and 41b guide the arrangement positions of the shoes, the pair of guide ribs 41a and 41b need to be formed in order to simultaneously treat the pair of shoes.

In this way, in the shoes care device 1 according to one embodiment of the present invention, since the pair of guide ribs 41a and 41b are formed in a symmetrical manner, the position in which the shoes are settled can be appropriately guided even when treating the pair of shoes.

In the shoes care device 1 according to one embodiment of the present invention, the guide ribs 41a and 41b may be formed in a shape including a curved surface.

In order for the guide ribs 41a and 41b to be used for shoes with a larger size, the guide ribs 41a and 41b may be arranged to be biased toward the front side of the bottom surface of the inner cabinet 100 to fit in front or rear heels of the shoes (especially preferable heel parts).

In this case, preferably, since both the front and rear heels of the shoes are formed in a shape including a curved surface, the guide ribs 41a and 41b are also formed in the shape including a curved surface.

Specifically, when the guide ribs 41a and 41b are formed in the shape including a curved surface, not only the positions in front and rear directions of the shoes fitted to the guide ribs 41a and 41b but also the positions of the shoes in the left and right directions may be partially guided.

Accordingly, preferably, the guide ribs 41a and 41b is formed in the shape including a curved surface, and a depression 81 is also formed in the shape including a curved surface.

As described above, in the shoes care device 1 according to one embodiment of the present invention, since the guide ribs 41a and 41b are formed in a curved shape, the settling position of the shoes may be guided in an optimal structure by reflecting the curved shape of the shoes.

In the shoes care device 1 according to one embodiment of the present invention, the guide ribs 41a and 41b may be formed on the front side of the bottom surface of the inner cabinet 100.

Preferably, the guide ribs 41a and 41b are formed on the front side of the inner cabinet 100 close to the door 30 so that the user can use them more easily. Further, preferably, with the diversity of the size of shoes, the guide ribs 41a and 41b is biased maximally to the front side of the inner cabinet 100 such that they are applicable to shoes with a larger size.

As described above, in the shoes care device 1 according to one embodiment of the present invention, since the guide ribs 41a and 41b are formed on the front side of the inner cabinet 100, the user can more easily recognize the settling position of the shoes.

In the shoes care device 1 according to one embodiment of the present invention, the main shelf 40 may be provided with a main water stop rib 43 continuously protruding upward along a circumferential surface.

That is, as illustrated in FIGS. 19 to 21, a circumferential surface of the main shelf 40 may be formed to protrude upward from the remaining part. Accordingly, the water staying on the upper surface of the main shelf 40 may be blocked by the main water stop rib 43 and thus blocked from moving outside the circumferential surface of the main shelf 40.

In this way, in the shoes care device 1 according to one embodiment of the present invention, since the main water stop rib 43 is formed along the circumferential surface of the main shelf 40, water moved on the main shelf 40 may be prevented from flowing to an unintended part.

In the shoes care device 1 according to one embodiment of the present invention, the main water stop rib 43 may be formed to get in close contact with an inner surface of the inner cabinet 100. That is, in a state where the main shelf 40 is installed in the inner cabinet 100, the main water stop rib 43 may be in close contact with the inner surface of the inner cabinet 100.

Accordingly, condensed water flowing down along the inner surface of the inner cabinet 100 may be induced to the inside of the main shelf 40 after reaching the main water stop rib 43.

Accordingly, the condensed water flowing down the inner surface of the inner cabinet 100 may be induced to the outlet 203 through the main water stop rib 43 and may be discharged quickly without leaking to unnecessary parts.

In this way, in the shoes care device 1 according to one embodiment of the present invention, since the main water stop rib 43 is in close contact with the inner surface of the inner cabinet 100, water flowing along the inner surface of the inner cabinet 100 may be guided to the upper surface of the main shelf 40 and then discharged.

In the shoes care device 1 according to one embodiment of the present invention, the main shelf 40 may be formed in a convex cross-sectional shape from the central part of the inner cabinet 100 to opposite sides. That is, as illustrated in FIG. 20, the main shelf 40 may be formed in a convex shape such that the central part thereof is higher than the opposite side.

Accordingly, since water staying in the central part of the main shelf 40 may flow to the opposite sides, the occurrence of residual water can be minimized on the main shelf 40.

In addition, considering the bottom surface of shoes formed mostly flat, the upper surface of the main shelf 40 may have a convex cross-sectional shape, which can minimize a contact area between the upper surface of the main shelf 40 and the bottom surfaces of the shoes.

In this way, since the central part of the main shelf 40 is convexly formed, the shoes care device 1 according to one embodiment of the present invention prevents water from accumulating in the central part of the main shelf 40, and minimizes an area where the upper surface of the main shelf 40 is in contact with the bottom surfaces of the shoes.

In the shoes care device 1 according to one embodiment of the present invention, the main shelf 40 may be installed to be partially spaced apart from the bottom surface of the inner cabinet 100.

As described above, preferably, a certain space between the upper surface of the main shelf 40 and the bottom surfaces of the shoes is formed to prevent contamination and bacterial growth.

In addition, a partial space needs to be also formed between the main shelf 40 and the bottom surface of the inner cabinet 100 to prevent contamination and bacterial growth. If there is no such space, it may be difficult for water staying between the main shelf 40 and the bottom surface of the inner cabinet 100 to be moved or evaporated.

Accordingly, preferably, a space through which air or water can move is formed between the main shelf 40 and the bottom surface of the inner cabinet 100.

In this way, since the shoes care device 1 according to one embodiment of the present invention has a space between the main shelf 40 and the bottom surface of the inner cabinet 100, the contamination and bacterial growth can be prevented from occurring due to residual water.

The shoes care device 1 according to one embodiment of the present invention may further include a door 30 that opens and closes the front surface of the inner cabinet 100, and the door 30 may include a door drain surface 80 that inclinedly protrudes to the inside of the inner cabinet 100 while being closer to the bottom surface of the inner cabinet 100 in a state where the front surface of the inner cabinet 100 is closed.

The door 30 is a part that opens and closes the front surface of the inner cabinet 100, and is configured to open and close an interior of the shoes care device 1. The door 30 may form any one surface of the shoes care device 1. The door 30 may form a left side or a right side of the shoes care device 1, or may form a front side of the shoes care device 1.

Hereinafter, except for a particularly limited case, it will be described that a surface on which the door 30 is formed in the shoes care device 1 is defined as the front surface of the shoes care device 1.

The shoes care device 1 may configured to include one door 30, but the shoes care device 1 may include a plurality of doors 30 as necessary.

On the other hand, when a large amount of moisture is present in the accommodation space 101 of the inner cabinet 100, the moisture may be condensed on an inner surface of the door 30 and flow down along the inner surface of the door 30.

In this case, condensed water flowing down the inner surface of the door 30 may be discharged through the inlet 203 formed on the bottom surface of the inner cabinet 100, but the condensed water not guided to the inlet 203 may be leaked to the outside through a gap between the door 30 and the inner cabinet 100.

Accordingly, the door 30 may include the door drain surface 80 that inclinedly protrudes to the inside of the inner cabinet 100 while being closer to the bottom surface of the inner cabinet 100 in a state where the front surface of the inner cabinet 100 is closed.

That is, as illustrated in FIG. 23, the door 30 may include the door drain surface 80 formed to protrude inside to a part in contact with the bottom surface of the inner cabinet 100, and the door drain surface 80 may be inclinedly formed to allow the condensed water flowing down to the door drain surface 80 to be induced to the inside of the inner cabinet 100.

In this way, in the shoes care device 1 according to one embodiment of the present invention, since the door drain surface 80 protrudes on the inner surface of the door 30 to allow the condensed water flowing down the inner surface to the inside of the inner cabinet 100, the condensed water on the inner surface of the door 30 may be prevented from leaking to the outside of the door 30.

In the shoes care device 1 according to one embodiment of the present invention, the door drain surface 80 may be formed to protrude further from an upper part of the main water stop rib 43 to the inside of the inner cabinet 100. That is, as illustrated in FIG. 23, the door drain surface 80 may protrude to pass through the upper part of the main water stop rib 43 in the front of the main shelf 40.

As described above, when the condensed water flowing down to the door drain surface 80 is induced inside the inner cabinet 100, if the condensed water is induced outside the main water stop rib 43, leakage may occur in a gap between the main shelf 40 and the door 30.

Accordingly, preferably, the condensed water flowing down to the door drain surface 80 may be induced to the upper surface of the main shelf 40 inside the main water stop rib 43 so as to get discharged quickly through the outlet 203.

As described above, in the shoes care device 1 according to one embodiment of the present invention, since the door drain surface 80 protrudes to pass through the upper part of the main water stop rib 43, the condensed water on the inner surface of the door 30 may be induced to the upper surface of the main shelf 40 and then discharged.

In the shoes care device 1 according to one embodiment of the present invention, the main shelf 40 may have the guide ribs 41a and 41b formed on the upper surface thereof to guide the position of the shoes, and the door drain surface 80 may have the depression 81 partially recessed along the shape of the guide ribs 41a and 41b.

As described above, since the door drain surface 80 is formed to protrude from the door 30 to the inside of the inner cabinet 100 in a part in contact with the bottom surface of the inner cabinet 100, it may interfere with the guide ribs 41a and 41b biased to the front side of the bottom surface of the inner cabinet 100.

In addition, when the door drain surface 80 is formed to protrude to the upper part of the guide ribs 41a and 41b, the condensed water flowing down along the door drain surface 80 may drop to an upper part of the shoes fitted to the guide ribs 41a and 41b.

Therefore, preferably, a part where the guide ribs 41a and 41b is formed to recess the door drain surface 80 so as to solve the aforementioned problems.

In this way, in the shoes care device 1 according to one embodiment of the present invention, since the depression 81 is formed in the door drain surface 80 to correspond to the shape of the guide ribs 41a and 41b, the condensed water can be prevented from being induced to the upper part in which the shoes are placed.

In the shoes care device 1 according to one embodiment of the present invention, a drain rib 82 extending to the remaining part of the door drain surface 80 may be formed in the upper part of the depression 81.

That is, as illustrated in FIG. 23, since the drain rib 82 protruding upwards is formed in the upper part of the depression 81, the condensed water induced to the upper part of the depression 81 may be blocked from flowing down by the drain rib 82.

Accordingly, the condensed water induced to the upper part of the depression 81 may be guided along the drain rib 82 in a transverse direction in which the depression 81 is not formed and may flow down the slope from the part in which the depression 81 is not formed and then be induced to the inlet 203.

As described above, in the shoes care device 1 according to one embodiment of the present invention, since the drain rib 82 is formed in the upper part of the depression 81 of the door drain surface 80, the condensed water on the door drain surface 80 may be induced to a part where the depression 81 is not formed and then discharged.

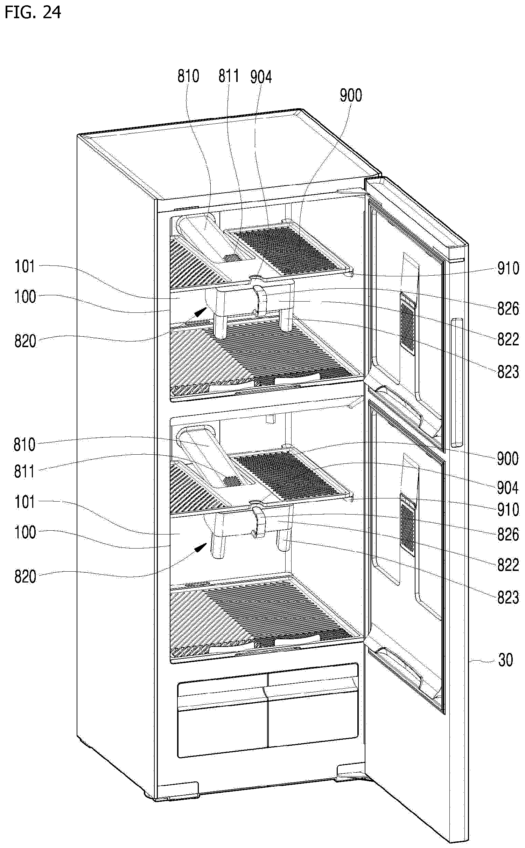

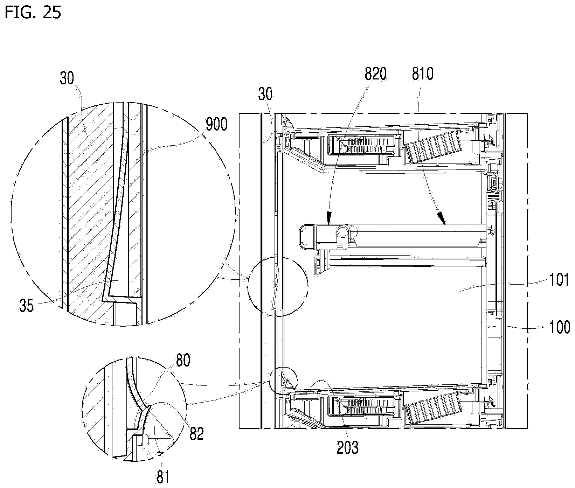

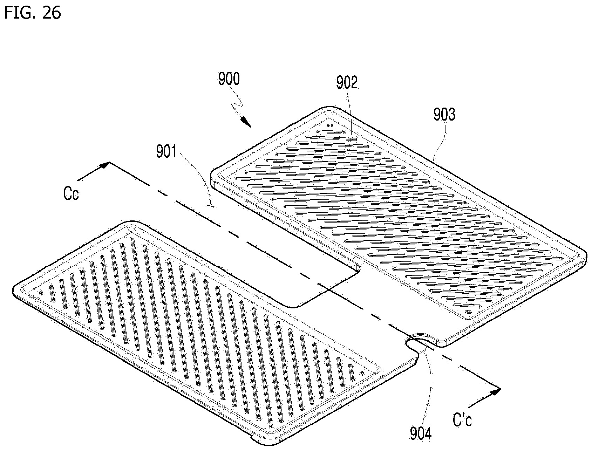

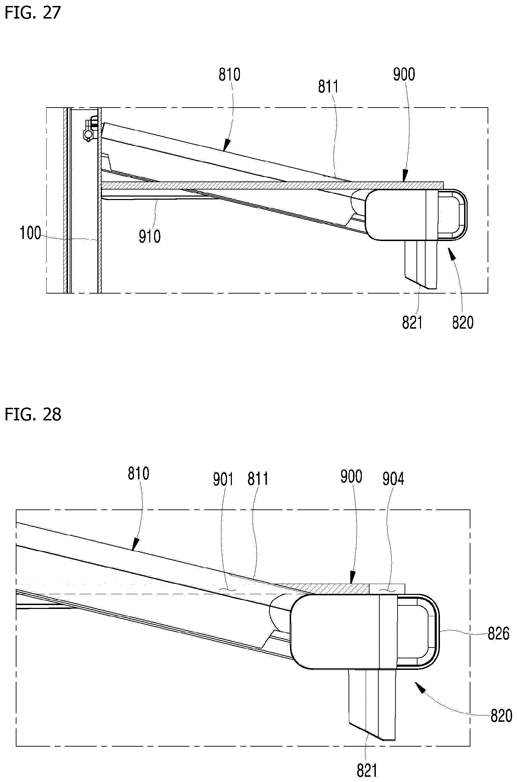

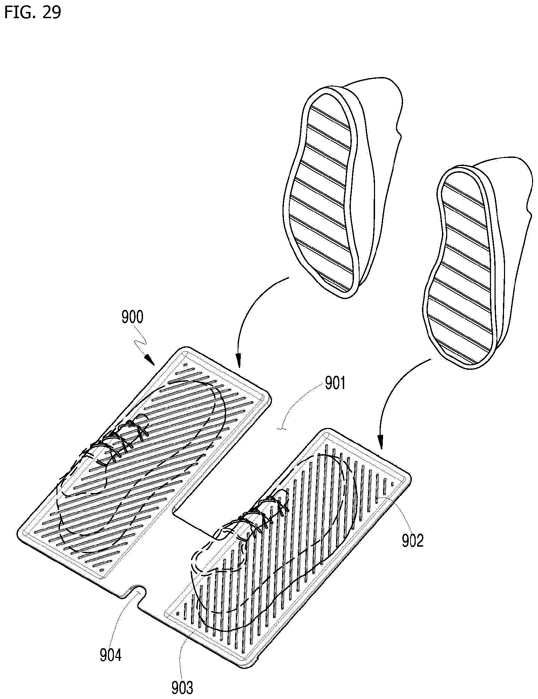

FIG. 24 is a view illustrating a state in which the auxiliary shelf 900 is installed in the shoes care device 1 according to one embodiment of the present invention. FIG. 25 is a cross-sectional view illustrating an inner surface of the door 30 illustrated in FIG. 23. FIG. 26 is a perspective view illustrating the auxiliary shelf 900 in the shoes care device 1 according to one embodiment of the present invention. FIG. 27 is a view illustrating the auxiliary shelf 900 and the nozzle duct 810 in the shoes care device 1 according to one embodiment of the present invention. FIG. 28 is a view illustrating a state in which the nozzle duct 810 is coupled to a cross section taken along line Cc-C'c of the auxiliary shelf 900 illustrated in FIG. 26. FIG. 29 is a view exemplarily illustrating a state in which the shoes are settled on the auxiliary shelf 900 in the shoes care device 1 according to one embodiment of the present invention.

The shoes care device 1 according to one embodiment of the present invention may include the inner cabinet 100, the outlet 203, the connection path F10, the blowing part 310, the dehumidifying part 330, and the auxiliary shelf 900.

As illustrated in FIG. 24, a shelf holder 910 is formed in the inner cabinet 100, and the auxiliary shelf 900 may be mounted on the shelf holder 910.

In this case, the auxiliary shelf 900 is installed to be mounted on the shelf holder 910, which is a part where the shoes can be settled on the upper surface thereof, and may spatially partition the accommodation space 101 of the inner cabinet 100.

Accordingly, the shoes may be accommodated in each space partitioned by the auxiliary shelf 900 in the inner cabinet 100 and then treated.

As described above, since the shoes care device 1 according to one embodiment of the present invention is used by mounting the auxiliary shelf 900 on the shelf holder 910 formed in the inner cabinet 100, a plurality of shoes can be efficiently treated even in one inner cabinet 100.

In the shoes care device 1 according to one embodiment of the present invention, the auxiliary shelf 900 may be installed to be detachable from the shelf holder 910. That is, the auxiliary shelf 900 may be configured to be separated from the shelf holder 910 in an outer direction of the inner cabinet 100.

Accordingly, when the foreign substances are accumulated on the auxiliary shelf 900 and needs to be removed, or when contaminants on the auxiliary shelf 900 needs to be removed, the user can separate the auxiliary shelf 900 from the inner cabinet 100 and perform cleaning and cleaning.

In addition, even if the auxiliary shelf 900 needs to be inspected and replaced, a predetermined operation may be performed after the auxiliary shelf 900 is separated from the inner cabinet 100.

As described above, in the shoes care device 1 according to one embodiment of the present invention, since the auxiliary shelf 900 is detached from the shelf holder 910, the auxiliary shelf 900 may be easily managed.

In the shoes care device 1 according to one embodiment of the present invention, the auxiliary shelf 900 may have an auxiliary isolation rib 902 formed to protrude upwards from an upper surface thereof.

That is, as illustrated in FIG. 26, the auxiliary isolation rib 902 protrudes from the upper surface of the auxiliary shelf 900, and the bottom surfaces of the shoes may be settled on the auxiliary isolation rib 902.

When the shoes are settled on the upper surface of the planar auxiliary shelf 900, the upper surface of the auxiliary shelf 900 and the bottom surfaces of the shoes are mostly in close contact with each other. In this case, since it is difficult for air to get in contact with the bottom surfaces of the shoes, there may be any difficulty treating the bottom surfaces of the shoes. Further, when water is present between the auxiliary shelf 900 and the shoes, it may be difficult to move or evaporate such water.

Therefore, preferably, a separation space through which air or water may move between the upper surface of the auxiliary shelf 900 and the bottom surfaces of the shoes.

As described above, in the shoes care device 1 according to one embodiment of the present invention, since the auxiliary isolation rib 902 is formed on the upper surface of the auxiliary shelf 900, the separation space for a movement of air or water may be formed between the upper surface of the auxiliary shelf 900 and the bottom surfaces of the shoes.

In the shoes care device 1 according to one embodiment of the present invention, the auxiliary isolation rib 902 may be formed in an oblique direction between the front surface and the rear surface of the inner cabinet 100.

That is, as illustrated in FIGS. 26 and 29, the auxiliary isolation ribs 902 may be formed in the oblique direction so as not to be parallel to the horizontal or vertical direction of the planar auxiliary shelf 900.

In general, the protrusion structure for preventing slipping may be formed on the bottom surfaces of the shoes, and such a protrusion structure may be formed on the bottom surfaces of the shoes along a transverse direction and/or a vertical direction.

Accordingly, when the auxiliary isolation rib 902 is formed horizontally or vertically on the auxiliary shelf 900, a relatively large part may be in parallel with a direction of the protrusion structure formed on the bottom surfaces of the shoes.

In this case, when the protrusion structure formed on the bottom surfaces of the shoes engages with the auxiliary isolation rib 902, the shoes and the upper surface of the auxiliary shelf 900 come into relatively more contact, and as described above, the larger the contact area may result in some disadvantages in terms of the function and maintenance of the shoes care device 1.

Accordingly, the auxiliary isolation rib 902 may be preferably formed in the oblique direction such that the protrusion structure formed on the bottom surfaces of the shoes are not parallel to the auxiliary isolation rib 902 as much as possible.

As described above, in the shoes care device 1 according to one embodiment of the present invention, since the auxiliary isolation rib 902 is formed in the oblique direction, an area in which the upper surface of the auxiliary shelf 900 and the bottom surfaces of the shoes come into contact can be further reduced.

In the shoes care device 1 according to one embodiment of the present invention, the auxiliary shelf 900 may be provided with an auxiliary order rib 903 continuously protruding upwards along the circumferential surface.

That is, as illustrated in FIGS. 26 and 29, the circumferential surface of the auxiliary shelf 900 may be formed to protrude upwards from the remaining parts. Accordingly, water staying on the upper surface of the auxiliary shelf 900 may be blocked by the auxiliary order rib 903, and may be blocked from moving outside the circumferential surface of the auxiliary shelf 900.

In this way, in the shoes care device 1 according to one embodiment of the present invention, since the auxiliary order rib 903 is formed along the circumferential surface of the auxiliary shelf 900, water moving on the auxiliary shelf 900 may be prevented from flowing to an unintended part

In the shoes care device 1 according to one embodiment of the present invention, the auxiliary shelf 900 may be formed in a convex cross-sectional shape from the central part of the inner cabinet 100 to opposite sides. That is, similarly to the main shelf 40, the auxiliary shelf 900 may be formed in a convex shape such that the central part thereof is higher than the opposite sides.

Accordingly, since water staying in the central part of the auxiliary shelf 900 may flow to opposite sides, the occurrence of residual water can be minimized on the auxiliary shelf 900.

In addition, considering the bottom surfaces of shoes formed mostly flat, the upper surface of the auxiliary shelf 900 may have a convex cross-sectional shape, which can minimize a contact area between the upper surface of the auxiliary shelf 900 and the bottom surfaces of the shoes.

In this way, since the central part of the auxiliary shelf 900 is convexly formed, the shoes care device 1 according to one embodiment of the present invention prevents water from accumulating in the central part of the auxiliary shelf 900, and minimizes an area where the upper surface of the auxiliary shelf 900 is in contact with the bottom surfaces of the shoes.

The shoes care device 1 according to one embodiment of the present invention may further include the nozzle duct 810 and the nozzle 820.

The nozzle duct 810 is a part installed inside the inner cabinet 100 and forming a passage of air, and one end thereof is coupled to the inner cabinet 100 and the other thereof end is coupled to the nozzle 820 to form a passage through which air moves to the nozzle 820.

In this case, the nozzle duct 810 may have an upper discharge port 811 opened upwards. For example, since the upper discharge port 811 is formed on a part of the upper surface of the nozzle duct 810, some of the air moving into the nozzle duct 810 may be discharged to the accommodation space 101 through the upper discharge port 811.

Meanwhile, since the nozzle duct 810 is installed to protrude inside the inner cabinet 100, the position where the nozzle 820 is disposed inside the inner cabinet 100 may vary.

The nozzle 820 is a part coupled to the end of the nozzle duct 810 such that the shoes can be inserted in the inner cabinet 100, and air passing through the connection passage F10 may be sprayed into the shoes inside the inner cabinet 100 through the nozzle 820.

Specifically, the nozzle 820 may inject air smoothly into the shoes by injecting air in a state where the nozzle is inserted into the shoes.

In this case, the nozzle 820 may have a lower discharge port 821 opened downwards so as to spray air into the shoes. For example, the lower discharge port 821 is formed in the end of the nozzle 820, and air may be discharged downwards through the lower discharge port 821 in a state where the end of the nozzle 820 is inserted into the shoe.

The nozzle duct 810 may be hinge-coupled to the inner cabinet 100. That is, a nozzle duct hinge axis 813 formed on one end of the nozzle duct 810 is coupled to the inner cabinet 100, and the nozzle duct 810 may be rotated around the nozzle duct hinge axis 813.

The nozzle duct 810 may be installed to protrude ahead from a rear wall of the inner cabinet 100.

Accordingly, depending on whether the nozzle 820 is used or not, the nozzle duct 810 is hinge-rotated inside the inner cabinet 100 and the angle is adjusted such that the nozzle 820 can be displaced in various positions on an inner space of the inner cabinet 100.

The nozzle 820 may be hinge-coupled to the nozzle duct 810. That is, the nozzle 820 may be coupled to a nozzle hinge axis 825 formed in the other end of the nozzle duct 810, and the nozzle 820 may be rotated around the nozzle hinge axis 825.

When the angle of the nozzle duct 810 is changed and the nozzle duct 810 is inclinedly disposed, it may be difficult for the nozzle 820 to get inserted into the upper surfaces of the shoes if the nozzle 820 is also inclined integrally with the nozzle duct 810.

Accordingly, even if the nozzle duct 810 is inclinedly disposed, the angle may be preferably adjusted such that the nozzle 820 is not inclined by more than a predetermined angle.

In this case, the auxiliary shelf 900 may have a cutout portion 901 with a width corresponding to an outer diameter of the nozzle duct 810. That is, as illustrated in FIG. 26, the central part of the auxiliary shelf 900 may be provided with a cutout portion 901 of which one surface is opened and inserted inside.

Further, as illustrated in Figs. 24 and 28, the nozzle duct 810 may be inserted into the cutout portion 901 when the auxiliary shelf 900 is mounted on the shelf holder 910.

That is, in the cutout portion 901 of the auxiliary shelf 900, since a part facing the rear wall of the inner cabinet 100 is opened, the auxiliary shelf 900 may be pushed from the front surface of the inner cabinet 100 to the rear wall.

In this case, since the nozzle duct 810 is inserted into the cutout portion 901 of the auxiliary shelf 900 and the width of the incision 901 corresponds to an outer diameter of the nozzle duct 810, interference by the nozzle duct 810 may not occur during the mounting process of the auxiliary shelf 900.

In the shoes care device 1 according to one embodiment of the present invention, when the auxiliary shelf 900 is mounted in the inner cabinet 100, since the nozzle duct 810 is inserted into the incision 901 of the auxiliary shelf 900, the auxiliary shelf 900 may be mounted while minimizing the interference with the nozzle duct 810.

In the shoes care device 1 according to one embodiment of the present invention, the upper discharge port 811 may be disposed on an upper part of the auxiliary shelf 900 and the lower discharge port 821 may be disposed below the auxiliary shelf 900 based on a state in which the nozzle duct 810 is inserted into the cutout portion 901 and an upper surface of the nozzle 820 is in contact with the auxiliary shelf 900.

That is, as illustrated in FIGS. 24 and 27, when the auxiliary shelf 900 is mounted on the shelf holder 910 to spatially partition the accommodation space 101 of the inner cabinet 100, the upper discharge port 811 may be disposed in an upper space thereof, and the lower discharge port 821 may be disposed in the lower space thereof.

Accordingly, when a plurality of shoes are accommodated in the accommodation space 101 of the inner cabinet 100, the shoes accommodated in the upper shelf 900 may be treated by the air discharged from the upper outlet 811, and the shoes accommodated in the lower shelf 900 may be treated by the air discharged from the lower outlet 821.

As described above, with the auxiliary shelf 900 mounted in the inner cabinet 100, the upper discharge port 811 discharges air to the upper part of the auxiliary shelf 900 and the lower discharge port 821 discharges air to the lower part of the auxiliary shelf 900. Accordingly, the shoes care device 1 according to one embodiment of the present invention can efficiently treat the shoes by placing the shoes above and below the auxiliary shelf 900.

In the shoes care device 1 according to one embodiment of the present invention, the nozzle 820 may include a nozzle body 822 hinge-coupled to the nozzle duct 810 and a nozzle protrusion 823 protruding downwards from the nozzle body 822 and having the lower discharge port 821 formed in the end thereof.

In this case, the upper surface of the nozzle body 822 can be attached to the lower surface of the auxiliary shelf 900 by magnetic force.

The nozzle 820 may be dropped downwards when an unintended external force is applied to the nozzle 820, or the performance of the part supporting the nozzle is degraded due to long-term use.

Accordingly, the upper surface of the nozzle body 822 may be attached to the lower surface of the auxiliary shelf 900 by the magnetic force, and the nozzle 820 may be held on the lower surface of the auxiliary shelf 900.

In this case, a magnetic body may be disposed in at least one of the nozzle body 822 and the auxiliary shelf 900, and the nozzle body 822 and the auxiliary shelf 900 may be made of a material that can be attached by the magnetic force.

In the shoes care device 1 according to one embodiment of the present invention, since the upper surface of the nozzle body 822 and the lower surface of the auxiliary shelf 900 are magnetically attached to each other, the lower surface of the auxiliary shelf 900 and the upper surface of the nozzle body 822 may be attached to each other.

In the shoes care device 1 according to one embodiment of the present invention, the auxiliary shelf 900 may be in close contact with the upper surface of the nozzle duct 810 by inclinedly forming a front lower surface of the cutout portion 901.

That is, as illustrated in FIG. 28, in order to closely adhere the upper surface of the nozzle duct 810 and the lower surface of the auxiliary shelf 900, the front lower surface of the cutout portion 901 may be inclined in a shape corresponding to the upper surface of the nozzle duct 810.

Accordingly, the auxiliary shelf 900 and the nozzle duct 810 can maintain a more stable contact state by minimizing a gap between the lower surface of the auxiliary shelf 900 and the upper surface of the nozzle duct 810.

In this way, in the shoes care device 1 according to one embodiment of the present invention, since the front lower surface of the cutout portion 901 in close contact with the upper surface of the nozzle duct 810 is formed to be inclined, the lower surface of the auxiliary shelf 900 and the upper surface of the nozzle duct 810 can be in stable contact with each other.

In the shoes care device 1 according to one embodiment of the present invention, the nozzle 820 may further include a nozzle handle 826 formed on a front surface of the nozzle body 822, and the auxiliary shelf 900 may have a handle groove 904 formed in a part corresponding to an upper part of the nozzle handle 826.

That is, as illustrated in FIGS. 24 and 28, since the nozzle handle 826 is formed in the nozzle body 822, the user can easily adjust the angle of the nozzle duct 810 by holding the nozzle handle 826 and applying force without touching the nozzle duct 810.

In this case, when the part where the nozzle handle 826 is formed is covered by the auxiliary shelf 900, it may be difficult for the user to recognize the position of the nozzle handle 826 or grasp the nozzle handle 826.

Accordingly, since the handle groove 904 is formed in a part of the auxiliary shelf 900 corresponding to the upper part where the nozzle handle 826 is formed, the user can more easily recognize the position the nozzle handle 826, or grasp the nozzle handle 826.

In this way, in the shoes care device 1 according to one embodiment of the present invention, since the handle groove 904 is formed in the part of the auxiliary shelf 900 corresponding to the upper part of the nozzle handle 826, the angle of the nozzle can be more easily changed through the nozzle handle 826.

The shoes care device 1 according to one embodiment of the present invention may further include the door 30, and the door 30 may include an accommodation groove 35 formed on the inner surface to correspond to the planar shape of the auxiliary shelf 900 and capable of accommodating the auxiliary shelf 900.

That is, as illustrated in FIG. 25, since the accommodation groove 35 is formed on the inner surface of the door 30, the auxiliary shelf 900 in an unused state may be accommodated in the accommodation groove 35.

As described above, the auxiliary shelf 900 is a component configured to simultaneously perform treatment on several shoes in one inner cabinet 100, and may not be used during the treatment of one shoe.

In this case, since storing the auxiliary shelf 900 in a separate space may be considerably cumbersome from the user's point of view, the auxiliary shelf 900 can be preferably accommodated in the shoes care device 1.

Accordingly, since the shoes care device 1 according to one embodiment of the present invention has the accommodation groove 35 formed on the inner surface of the door 30 to accommodate the auxiliary shelf 900, the auxiliary shelf 900 in the unused state can be conveniently and effectively stored.

In the shoes care device 1 according to one embodiment of the present invention, the accommodation groove 35 may be formed such that the lower part thereof is spaced apart from the auxiliary shelf 900 in a state where the auxiliary shelf 900 is accommodated.

That is, as illustrated in FIG. 25, a predetermined gap may be formed between the auxiliary shelf 900 and the upper part of the accommodation groove 35.

As described above, when the condensed water flows down along the inner surface of the door 30, the condensed water may remain in the accommodation groove 35. Specifically, when both the auxiliary shelf 900 and the accommodation groove 35 are in close contact with each other, bacteria and mold may be generated by the condensed water remaining therebetween.

Accordingly, preferably, as the lower part of the accommodation groove 35 is spaced apart from the auxiliary shelf 900 by the predetermined gap, the condensed water flowing down to the lower part of the accommodation groove 35 is made to evaporate when air is circulated.

In addition, when the user wants to use the auxiliary shelf 900 accommodated in the accommodation groove 35, it may be difficult for the user to separate the auxiliary shelf 900 from the accommodation groove 35 if both the auxiliary shelf 900 and the accommodation groove 35 are in close contact with each other.

Accordingly, as the lower part of the accommodation groove 35 is spaced apart from the auxiliary shelf 900 by the predetermined gap, when the auxiliary shelf 900 is pushed into the corresponding gap, the upper part of the auxiliary shelf 900 is made to protrude from the accommodation groove 35 so that the user can easily separate the auxiliary shelf 900 from the accommodation groove 35.

Since the lower part of the accommodation groove 35 is spaced apart from the auxiliary shelf 900 by the predetermined gap, the shoes care device 1 according to one embodiment of the present invention may prevent moisture from remaining and causing contamination between the auxiliary shelf 900 and the accommodation groove 35, and may easily detach the auxiliary shelf 900 from the accommodation groove 35.

In the shoes care device 1 according to one embodiment of the present invention, the auxiliary shelf 900 may be attached to the accommodation groove 35 by the magnetic force.

The auxiliary shelf 900 in the unused state needs to be stably accommodated in the accommodation groove 35, and needs to be prevented from being unintentionally detached from the accommodation groove 35 even in vibration and impact caused by opening and closing the door 30.

Accordingly, the auxiliary shelf 900 may be attached to the accommodation groove 35 by the magnetic force, and the auxiliary shelf 900 in the unused state may be held in the accommodation groove 35.

In this case, a magnetic material may be disposed in at least one of the accommodation groove 35 and the auxiliary shelf 900, and the accommodation groove 35 and the auxiliary shelf 900 may be made of a material that can be attached by the magnetic force.

In this way, in the shoes care device 1 according to one embodiment of the present invention, since the auxiliary shelf 900 and the accommodation groove 35 are attached to each other by the magnetic force, the auxiliary shelf 900 can be stably attached to the accommodation groove 35.

In the shoes care device 1 according to one embodiment of the present invention, the auxiliary shelf 900 may be accommodated in the accommodation groove 35 in a shape where the cutout portion 901 is disposed in the lower part thereof.

That is, the auxiliary shelf 900 having a 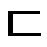 -shaped plane by forming cutout portion 901 may be accommodated in the accommodation groove 35 in a shape where an open surface of the cutout portion 901 is disposed in the lower part thereof. Accordingly, the auxiliary shelf 900 may be accommodated in the accommodation groove 35 in a shape where the handle groove 904 formed on the auxiliary shelf 900 is disposed in the upper part thereof.

As described above, preferably, the lower part of the accommodation groove 35 is spaced apart from the auxiliary shelf 900 by the predetermined gap such that the condensed water flowing down to the lower part of the accommodation groove 35 is evaporated when air is circulated.

Accordingly, the cutout portion 901 with a relatively large open area is disposed in the lower part of the accommodation groove 35 so that the lower part of the accommodation groove 35 can has a larger separation part that that of the auxiliary shelf 900.

In addition, when the user wants to take out the auxiliary shelf 900 from the accommodation groove 35, a handle portion that is easy to grip may be disposed on the upper part, which can be more conveniently used in the user's point of view.

As described above, since the shoes care device 1 according to one embodiment of the present invention accommodates the auxiliary shelf 900 in the accommodation groove 35 such that the cutout portion 901 is disposed downwards, a more separation part is secured between the lower part of the accommodation groove 35 and the auxiliary shelf 900, and the auxiliary shelf 900 can be easily separated from the accommodation groove 35 through the handle groove 904.

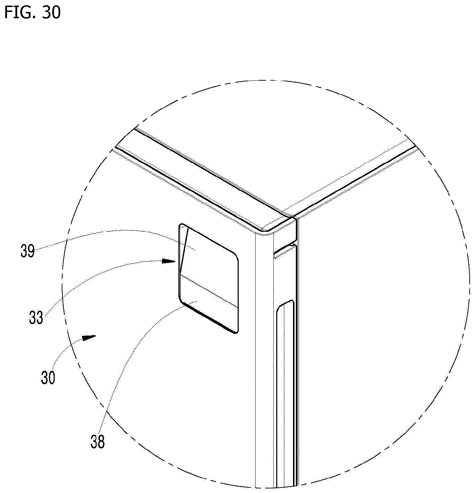

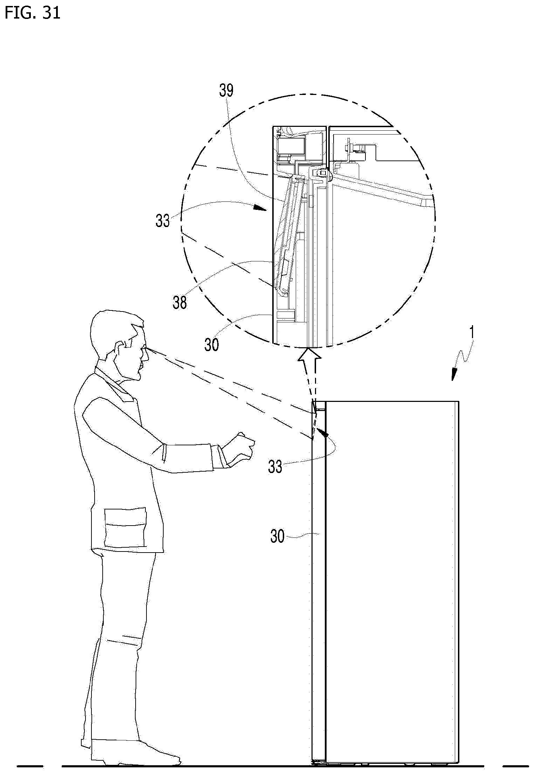

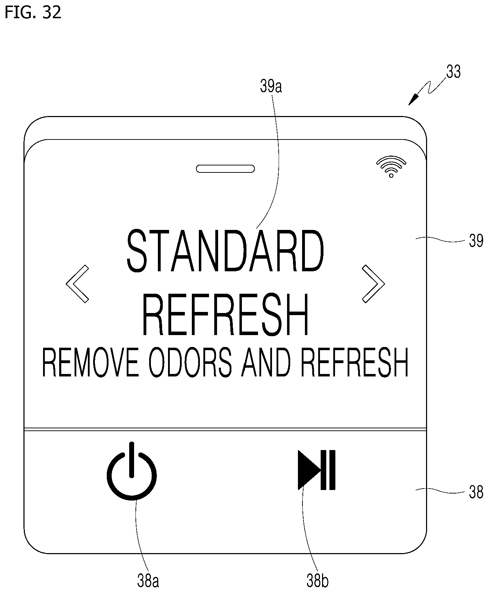

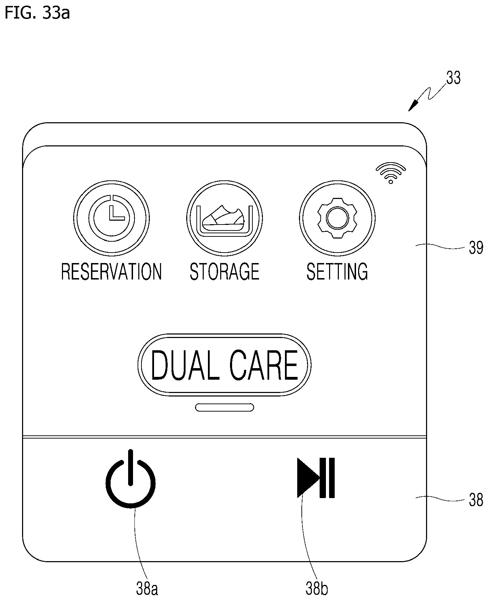

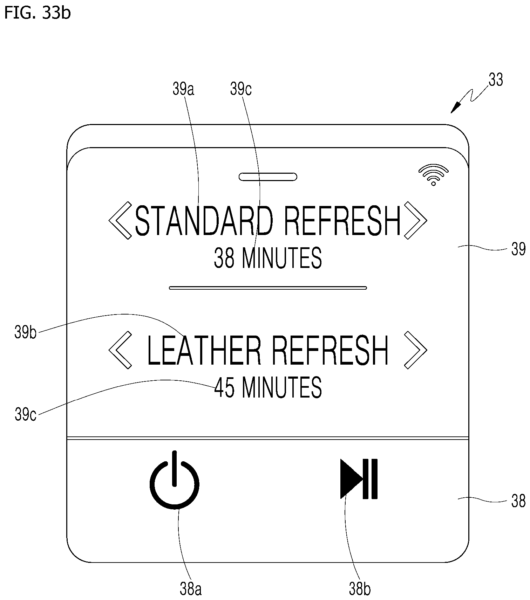

FIG. 30 is a view illustrating a control panel 33 in more detail in the shoes care device 1 according to one embodiment of the present invention. FIG. 31 is a view exemplarily illustrating a state in which a user uses the control panel 33 in the shoes care device 1 according to one embodiment of the present invention. FIG. 32 is a view exemplarily illustrating a fixed UI section 38 and a variable UI section 39 of the control panel 33 illustrated in FIG. 30. FIGS. 33a and 33b are views exemplarily illustrating a modified example of the control panel 33 illustrated in FIG. 32.

The shoes care device 1 according to one embodiment of the present invention may include the inner cabinet 100, the connection path F10, the blowing part 310, the dehumidifying part 330, the door 30, and the control panel 33. In this case, the control panel 33 may include the fixed UI section 38 and the variable UI section 39.

Specifically, the control panel 33 is a part formed outside the door 30 such that a control signal for the shoes care device 1 can be input by the user, and the user can manipulate the control panel 33 to input an operation signal for each component of the shoes care device 1.

In this case, the control panel 33 may include a touch screen. A control unit (the controller 10) that controls each component of the shoes care device 1 in connection with the control panel 33 is provided in the inner space of the door 30. The controller 10 may be provided inside the machine room 50.

In addition, a user interface (UI) is disposed on the control panel 33, and the user can input an operation signal for each component of the shoes care device 1 by manipulating the UI disposed on the control panel 33.

In this case, although the UIs are essentially used due to their characteristics, there may be the UIs whose types do not need to be changed. Alternatively, the UIs may be present that need to be used while changing to various types due to their characteristics.

In this way, since a way for the user to manipulate the UIs disposed on the control panel 33 varies according to the characteristics, preferably, each of the UIs may be separated and disposed in order for the user to use them more conveniently in consideration of the corresponding characteristics.

To this end, the fixed UI section 38 may be formed parallel to the front surface of the door 30, and first control UIs 38a and 38b capable of changing whether to perform display may be disposed. In this case, the first control UIs 38a and 38b are used necessarily due to their characteristics, but the type does not need to be changed, and the user can recognize that they are always disposed in that position on the control panel 33.

Accordingly, even if the fixed UI section 38 is formed parallel to the front surface of the door 30, the user may not have much difficulty checking of the position or manipulating.

In addition, the variable UI section 39 may be formed to have an inclination angle with the front surface of the door 30, and second control UIs 39a, 39b and 39c capable of changing whether to perform display and types may be disposed. In this case, the second control UI 39a, 39b and 39c need to be used by changing to various types due to their characteristics, and the user needs to operate then while checking the status and type of UIs currently being implemented.

Accordingly, when the variable UI section 39 is also formed parallel to the front surface of the door 30, inconvenience for the user, such as having to bend or lower the posture to check or manipulate its position, may occur.

Therefore, the variable UI section 39 is formed to have the inclination angle with the front surface of the door 30 such that the user can check or manipulate the position more comfortably.

In this way, since the shoes care device 1 according to one embodiment of the present invention is arranged according to the characteristics of the UIs in the fixed UI section 38 and the variable UI section 39 formed in the control panel 33, the user can more conveniently input the control signal for the shoes care device 1.

In the shoes care device 1 according to one embodiment of the present invention, the variable UI section 39 may be disposed above the fixed UI section 38. That is, as illustrated in FIGS. 30 and 31, the fixed UI section 38 may be formed in the lower end of the control panel 33, and the variable UI section 39 may be formed in the upper end of the control panel 33.

As described above, considering that the user recognizes that the first control UIs 38a and 38b disposed in the fixed UI section 38 are always disposed in that position on the control panel 33, the user may not experience any special inconvenience even if they are disposed relatively lower.

On the other hand, preferably, the second control UI 39a, 39b, and 39c disposed in the variable UI section 39 may be disposed in an upper part close to the user's eye level in that it is necessary for the user to manipulate the UI while checking the status and type of the UI being implemented.

In this way, in the shoes care device 1 according to one embodiment of the present invention, since the control panel 33 is formed to place the variable UI section 39 above the fixed UI section 38, the variable UI section 39 relatively complicated to manipulate can be placed in a more convenient position for the user.

In the shoes care device 1 according to one embodiment of the present invention, the variable UI section 39 may be inclined to move away from the front surface of the door 30 in an upward direction.

Referring to FIG. 31, the shoes care device 1 may be mainly used while being disposed on the floor, and the user approaching the shoes care device 1 may have an eye level at a relatively higher position than the control panel 33.

Accordingly, in order for the user to more conveniently check and manipulate the variable UI section 39, preferably, an inclined surface of the variable UI section 39 is formed to retreat in the upward direction.

In this way, in the shoes care device 1 according to one embodiment of the present invention, since the upper part of the variable UI section 39 is formed to have an inclined surface in the shape of retreating from the front surface of the door 30, the user can more conveniently check and operate the variable UI section 39 relatively complicated to manipulate.

In the shoes care device 1 according to one embodiment of the present invention, the fixed UI section 38 and the variable UI section 39 may be formed in an integral shape in which a connection part therebetween is bent.

As mentioned above, the connection part between the fixed UI section 38 and the variable UI section 39 needs to be bent such that the fixed UI section 38 is formed parallel to the front surface of the door 30, and the variable UI section 39 has the inclination angle with the front surface of the door 30

In this case, if the fixed UI section 38 and the variable UI section 39 are separately manufactured and coupled with each other, a separate connection frame, etc. needs to be intervened in the bent connection part between the fixed UI section 38 and the variable UI section 39.

In other words, since separately manufacturing the fixed UI section 38 and the variable UI section 39 makes the configuration relatively complicated, the manufacturing may be cumbersome, and the separate connection frame may give an aesthetically untidy appearance.

Therefore, the fixed UI section 38 and the variable UI section 39 may be preferably formed in an integral shape without separately manufacturing them.

In this way, in the shoes care device 1 according to one embodiment of the present invention, since the fixed UI section 38 and the variable UI section 39 are integrally formed, which makes the structure simpler, the control panel 33 can be easily manufactured and aesthetics can be improved in appearance.

In the shoes care device 1 according to one embodiment of the present invention, the first control UIs 38a and 38b may include at least one of a power source UI capable of selecting whether to power the shoes care device 1 and an operation UI capable of selecting whether to operate the shoes care device 1.

The power UI is meant to operate a power on/off of the shoes care device 1, and corresponds to an UI essential for use of the shoes care device 1. However, the type of the power UI does not need to be changed, and it may be convenient for the user to dispose the power UI in a specific position all the times.

Similarly, the operation UI is meant to manipulate whether or not the shoes care device 1 starts or stops operating, and also corresponds to an UI essential for the use of the shoes care device 1. However, the type of operation UI also does not need to be changed, and it may be convenient for the user to dispose the operation UI in a specific position all the times.

Accordingly, preferably, the power UI and the operation UI are disposed in the fixed UI section 38 so as to change whether to perform display.

In this way, in the shoes care device 1 according to one embodiment of the present invention, since at least one of the power UI and operation UI is disposed in the fixed UI section 38, the power UI and operation UI with a relatively simple operation may be disposed in a way of changing whether to perform display.

In the shoes care device 1 according to one embodiment of the present invention, the second control UIs 39a, 39b and 39c may include at least one of a stroke UI capable of selecting the stroke type of the shoes care device 1, a material UI capable of selecting the material type of shoes accommodated in the shoes care device 1 and a time UI capable of selecting the type of operation time of the shoes care device 1.

The stroke UI is meant to set what stroke to perform on the shoes in the shoes care device 1, and needs to be manipulated while checking the state and type of the UI currently being implemented.

Similarly, the material UI is meant to select the material of the shoes treated by the shoes care device 1 and perform an optimal treatment accordingly, and also needs to be manipulated while checking the status and type of the UI currently being implemented.

In addition, similarly, the time UI is meant to set the treatment time for shoes performed by the shoes care device 1, and needs to be manipulated while checking the status and type of UI currently being implemented.

Accordingly, preferably, the stroke UI, the material UI, and the time UI may be disposed in the variable UI section 39 so that whether to perform display and the type thereof are changed.

In this way, in the shoes care device 1 according to one embodiment of the present invention, since at least one of the stroke UI, the material UI, and the time UI is disposed in the variable UI section 39, the stroke UI, the material UI, and the time UI, which are relatively complicated in manipulation, may be placed in a way of changing whether to perform display and the type thereof.

In the shoes care device 1 according to one embodiment of the present invention, the fixed UI section 38 may change whether display the first control UIs 38a and 38b in a touch manner.

As described above, since the display of the first control UIs 38a and 38b is changed in the fixed UI section 38, it may be convenient for the user to display the first control UI 38a and 38b when the user touches them.

Meanwhile, the first control UIs 38a and 38b disposed in the fixed UI section 38 may be formed in a separate physical button manner in addition to the touch screen, and may be configured to be manipulated by appropriately combining the touch and physical pressure.

In this way, in the shoes care device 1 according to one embodiment of the present invention, since the fixed UI section 38 is operated in the touch manner, the fixed UI section 38 in the way of changing whether to perform display can be effectively manipulated.

In the shoes care device 1 according to one embodiment of the present invention, the variable UI section 39 may change whether to display the second control UIs 39a, 39b and 39c in the touch manner, and the type of the second control UIs 39a, 39b, and 39c may be changed in a sliding manner.

As described above, since the variable UI section 39 needs to change whether to display the second control UIs 39a, 39b and 39c, it may be convenient for the user to display the second control UIs 39a, 39b and 39c when the user touches them.

In addition, since the type of the second control UIs 39a, 39b and 39c is also changed in the variable UI section 39, it may be convenient to change several types of UI in the sliding manner on a screen of a certain area.

In this way, in the shoes care device 1 according to one embodiment of the present invention, since the variable UI section 39 is manipulated by the touch manner and the sliding manner, the variable UI section 39 in a manner of changing whether to perform display and the type thereof can be effectively operated.

In the shoes care device 1 according to one embodiment of the present invention, a plurality of inner cabinets 100 are disposed, and the connection paths F10 may be formed for each of the inner cabinets 100. That is, the plurality of inner cabinets 100 may be installed spatially separated from each other.

In this case, although a configuration in which the inner cabinet 100 is vertically disposed is illustrated, the present invention is not limited thereto, and the inner cabinet 100 may be disposed in a front-rear direction, a left-right direction, an up- and-down direction, or a combination thereof.

In addition, since the connection paths F10 are installed individually in the inner cabinets 100 arranged respectively, the shoes can be treated independently in each of the inner cabinets 100.

In this way, in the shoes care device 1 according to one embodiment of the present invention, since the plurality of inner cabinets 100 are arranged and the connection path F10 is formed for each inner cabinet 100, each shoes care device 1 can independently treat the shoes for each of the inner cabinets 100.

In the shoes care device 1 according to one embodiment of the present invention, the variable UI section 39 may be disposed to display the second control UIs 39a, 39b and 39c for each of the inner cabinets 100.

That is, as illustrated in FIG. 32, the variable UI section 39 may be configured to display an overall setting state of the shoes care device 1. In addition, as illustrated in FIG. 33B, the variable UI section 39 may be configured to display setting states for each of the inner cabinets 100 as needed.

Accordingly, all the setting states of the plurality of inner cabinets 100 can be confirmed in one variable UI section 39.

In this way, in the shoes care device 1 according to one embodiment of the present invention, since the UIs for each of the inner cabinets 100 is displayed in the variable UI section 39, the states of each of the inner cabinets 100 can be easily confirmed.

In the shoes care device 1 according to one embodiment of the present invention, the second control UIs 39a, 39b and 39c for each inner cabinet 100 can be independently changed.

That is, as illustrated in FIG. 33A, the user can select the UI displayed in the variable UI section 39 to perform independent control (which may be referred to as 'dual care') for each of the inner cabinets 100.

In addition, as illustrated in FIG. 33b, in a state where the second control UIs 39a, 39b and 39c for each of the inner cabinets 100 are displayed, the type of UIs can be changed by the sliding manner such that each of the inner cabinets 100 can treat the shoes independently.

In this way, since the UIs for each of the inner cabinets 100 displayed in the variable UI section 39 are independently operated, the independent treatment can be easily performed in each of the inner cabinets 100.

In the shoes care device 1 according to one embodiment of the present invention, a plurality of inner cabinets 100 may be stacked vertically, and the variable UI section 39 may be divided vertically to display the second controls UI 39a, 39b and 39c for each of the inner cabinets 100.

That is, as illustrated in FIG. 33b, the second control UIs 39a, 39b and 39c displayed on an upper part of the variable UI section 39 may display a set state of the inner cabinet 100 disposed upwards and the second control UIs 39a, 39b and 39c displayed on a lower part of the variable UI section 39 may display a set state of the inner cabinet 100 disposed downwards.

Accordingly, the user can easily grasp and manipulate the states of each of the inner cabinets 100 by checking a display content divided up and down on the variable UI section 39.

In this way, in the shoes care device 1 according to one embodiment of the present invention, since the plurality of inner cabinets 100 are arranged vertically, and the variable UI section 39 is divided vertically to display the UIs for each of the inner cabinets 100, each of inner cabinets 100 disposed vertically may be intuitively identified through the variable UI section 39.

The shoes care device 1 according to one embodiment of the present invention may further include the steam generator 700 configured to supply steam to the inner cabinet 100.

In other words, since the steam can be supplied into the inner cabinet 100 through the steam generator 700 to perform a steam treatment on the shoes, in addition to high temperature sterilization of the steam, a refreshing effect can be exerted by swelling of a shoe material.

In the shoes care device 1 according to one embodiment of the present invention, the stroke UI can select a drying stroke and a steam stroke.

As described above, the shoes care device 1 may supply dry air or steam to the accommodation space 101 of the inner cabinet 100. Further, depending on the type of shoes, shoes of a specific material may be disadvantageous in contact with steam.

Accordingly, the user may be preferably allowed to select the dry stroke and the steam stroke by manipulating the stroke UI disposed in the variable UI section 39.

In this way, since the shoes care device 1 according to one embodiment of the present invention can select the drying stroke and the steam stroke in the variable UI section 39, various treatments for the shoes can be easily performed.

In the shoes care device 1 according to one embodiment of the present invention, the control panel 33 may be disposed on the upper end of the door 30. That is, as illustrated in FIG. 31, the control panel 33 may be disposed on the upper end of the door 30 relatively close to the user's eye level.

Accordingly, the user may easily check and manipulate the control panel 33 even if the posture is bent or lowered relatively low.

In this way, in the shoes care device 1 according to one embodiment of the present invention, since the control panel 33 is disposed in the upper end of the door 30, the control panel 33 for the user's manipulation may be disposed closer to the user.

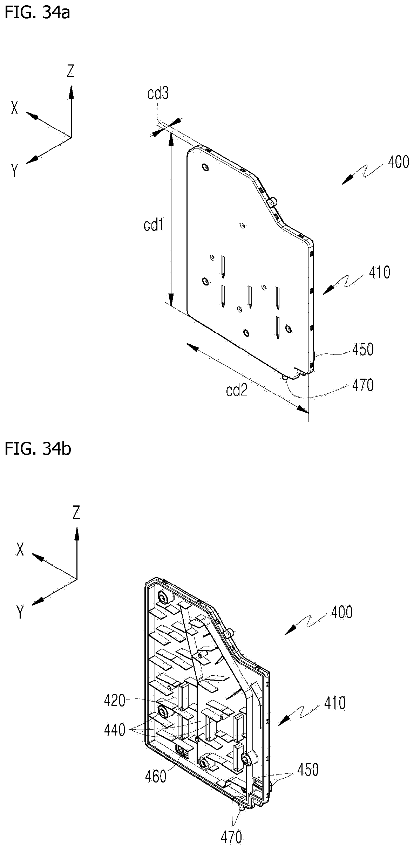

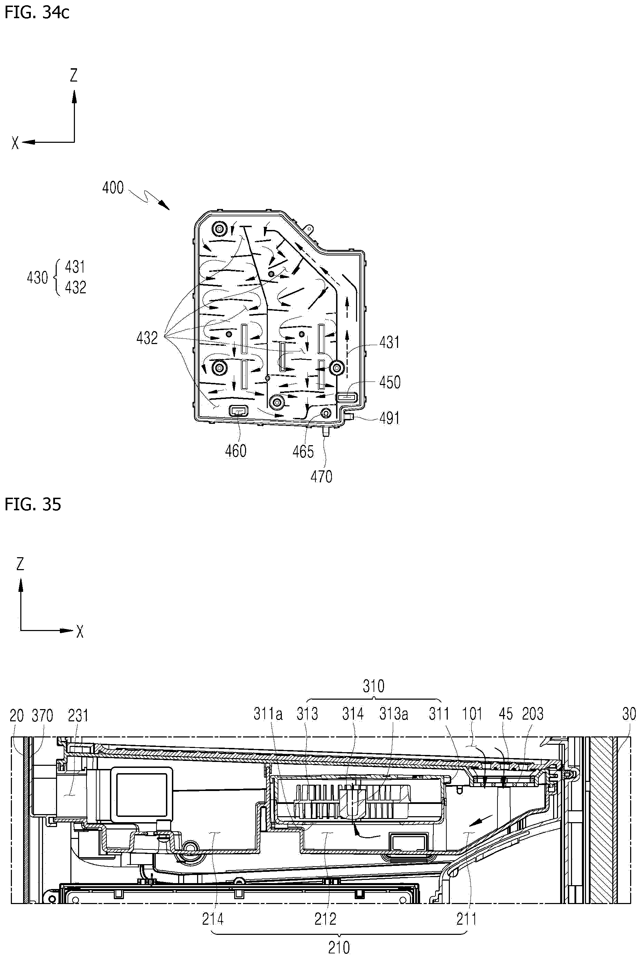

FIG. 34a is an exploded perspective view illustrating the condenser 400 according to one embodiment of the present invention. FIG. 34b is a view illustrating an internal state of the condenser 400 of FIG. 34a. FIG. 34c is a front view illustrating the condenser 400 of FIG. 34b.

The condenser 400 forms a part of the regeneration path F20.

The condenser 400 may be made of or include a material such as synthetic resin, metal, ceramic, etc. The condenser 400 may be configured by injection molding, press molding, etc.

The condenser 400 may be made of a material having excellent thermal conductivity. The condenser 400 may be made of a metal with excellent thermal conductivity, and can be made of a material such as aluminum, an aluminum alloy, copper, a copper alloy, etc.

The condenser 400 may include a condenser housing 410, a flow path guide wall 440, a condenser inlet 450, and a condensed water outlet 470. The condenser 400 may include a condenser communication port 465.

In one embodiment of the present invention, the condenser 400 is disposed between the inner cabinet 100 and the outer cabinet 20. The condenser 400 may be disposed between the inner rear plate 110 and the outer rear plate 21, between the first inner side plate 120 and the first outer side plate 22, or between the second inner side plate 130 and the second outer side plate 23.

In the drawing in accordance with one embodiment of the present invention, the condenser 400 is illustrated to be disposed between the first inner side plate 120 and the first outer side plate 22.

A thickness cd3 of the condenser 400 (the size of the condenser 400 in the second direction (Y direction)) may be 0.5 to 1 times a distance between the first inner side plate 120 and the first outer side plate 22. A width cd2 of the condenser 400 (the size of the condenser 400 in the first direction (X direction)) and a height cd1 in the vertical direction may be 10 times or more and 40 times or less of the thickness cd3 of the condenser 400 (the size of the condenser 400 in the second direction (Y direction)), respectively.

The condenser 400 may be in close contact with the first inner side plate 120 and/or the first outer side plate 22. That is, the condenser 400 may be in close contact with an outer surface of the first inner side plate 120 or in close contact with an inner surface of the first outer side plate 22.

The first outer side plate 22 may be made of a material having excellent thermal conductivity. The first outer side plate 22 may be made of a metallic material having excellent thermal conductivity. The condenser 400 is disposed in close contact with or very close to the first outer side plate 22.

Accordingly, the water vapor moving through the condenser 400 may be effectively condensed.

In the shoes care device 1 according to one embodiment of the present invention, the machine room 50 is provided below the inner cabinet 100, and the condenser 400 is disposed between the inner cabinet 100 and the outer cabinet 20. That is, the condenser 400 is not provided inside the machine room 50 and is disposed outside a restricted space of the machine room 50.

In addition, the condenser 400 is disposed between the inner cabinet 100 and the outer cabinet 20 and can fully utilize the space between the inner cabinet 100 and the outer cabinet 20, and may be configured to have a large area on the whole.

In the shoes care device 1 according to one embodiment of the present invention, the condenser 400 may be disposed between the first inner side plate 120 of the inner cabinet 100 and the first outer side plate 22 of the outer cabinet 20, and the dry air duct 370 may be disposed between the inner rear plate 110 of the inner cabinet 100 and the outer rear plate 21 of the outer cabinet 20.

The condenser 400 may be formed to have a size corresponding to an area of one wall surface of the inner cabinet 100 (e.g., an area of the first inner side plate 120), and accordingly, the condenser 400 may be configured to have a relatively large area.

In one embodiment, the vertical height cd1 of the condenser 400 may be 0.5 to 1 times the vertical height h11 or h12 of the inner cabinet 100, and the width cd2 of the condenser 400 may be 0.5 to 1 times the width (the size in the first direction (X direction)) in the first inner side plate 120 of the inner cabinet 100.

According to one embodiment of the present invention, the vertical height of the machine room 50 in the shoes care device 1 may be formed relatively low, and the vertical heights h2 of the machine room 50 may be formed smaller than the vertical height h11 or h12 of the inner cabinet 100.

In this case, since the space inside the machine room 50 becomes relatively small, and the machine room 50 accommodates several components that constitute the shoes care device 1, the arrangement and design of each component may be restricted. Furthermore, unlike the present invention, when the condenser 400 is disposed inside the machine room 50, the shape and position of the condenser 400 may be limited, and the heat exchange efficiency of the condenser 400 may be reduced.

In one embodiment of the present invention, since the condenser 400 is disposed between the inner cabinet 100 and the outer cabinet 20, each component can be effectively arranged even when the vertical height of the machine room 50 needs to be formed relatively low.

In addition, by placing the condenser 400 between the inner cabinet 100 and the outer cabinet 20, the condenser 400 can be formed in a relatively thin and wide shape, the heat exchange area of the condenser 400 can be expanded, and the heat exchange between an external air of the shoes care device 1 and the condenser 400 can be easily performed. Accordingly, water vapor passing through the condenser 400 may be effectively condensed.

While the condenser 400 is disposed between the first inner side plate 120 and the first outer side plate 22, the condenser 400 may be disposed to extend upwards from the module housing 200. Further, the condenser 400 is disposed higher than the sump 600 disposed inside the machine room 50.

Accordingly, steam in the third module chamber 214 of the module housing 200 naturally rises and flows into the condenser 400, and the condensed water in the condenser 400 naturally descends by gravity and flows into the sump 600, and when the dehumidifying material 331 is regenerated, the steam can be effectively condensed and discharged.

The condenser housing 410 forms an overall appearance of the condenser 400. A condensation space 420, which is a space inside the condenser 400, is provided inside the condenser housing 410.

The condenser inlet 450 forms an inlet of the condenser 400 through which air flows into the condenser 400, and forms an inlet of the condensation space 420.

The condenser inlet 450 is connected and in communication with the wet air outlet 232. In the shoes care device 1 according to one embodiment of the present invention, the wet air outlet 232 and the condenser inlet 450 may be directly connected to each other while being disposed inside and outside based on the first inner side plate 120 of the inner cabinet 100.

While wet air introduced into the condenser 400 through the condenser inlet 450 moves along the flow path inside the condenser 400, the wet air is condensed by heat exchange and moves below the condenser 400.

The condensed water outlet 470 forms an outlet through which the condensed water in the condenser 400 is discharged, and also forms an outlet of the condensation space 420.

The condensed water outlet 470 of the condenser 400 is connected to the sump 600, and the condensed water inside the condenser 400 moves to the sump 600.

The condensed water outlet 470 may be formed in the lower end of the condenser 400. That is, the condensed water outlet 470 may be formed in the lowest part of the condensation space 420. The lower end of the condensation space 420 may be inclined downwardly toward the condensed water outlet 470.

The condensed water outlet 470 is connected to the sump 600. The condensed water outlet 470 for the movement of the condensed water and the sump 600 may be connected by a pipe, a hose, etc.

The flow path guide wall 440 is formed in the shape of a plate having a narrow width and a long length. The flow path guide wall 440 may be provided in the inside (the condensation space 420) of the condenser housing 410 and may be formed in a plural form.

A plurality of flow path guide walls 440 are disposed to be spaced apart from each other to form a path through which water vapor and condensed water move. The plurality of flow path guide walls 440 may be disposed to cross each other.

The flow path guide wall 440 forms a condenser flow path 430 which is a path extending from the condenser inlet 450 to a condenser outlet 460 in the condensation space 420. The condenser flow path 430 is a movement passage of air (or water) provided inside the condenser 400, and connects the condenser inlet 450 and the condenser outlet 460. In addition, the condenser flow path 430 is a movement passage of air (or water) provided inside the condenser 400, and connects the condenser inlet 450 and the condensed water outlet 470.

The condenser flow path 430 may include an introduction section 431 and a condensation section 432.

As the plurality of flow path guide walls 440 are spaced apart from each other, the condensation section 432 may be provided between the flow path guide walls 440.

The condensation section 432 may be formed in a zigzag shape extending from the uppermost side of the condensation space 420 to the lower side.

In one embodiment of the present invention, an angle formed by the flow path guide wall 440 forming the condensation section 432 with a horizontal plane may be 1 to 10°. The flow path guide wall 440 constituting the condensation section 432 is formed substantially along the first direction (X direction), and may be disposed to be inclined upwards or downwards along the first direction (X direction).

In one embodiment of the present invention, the length of the flow path guide wall 440 constituting the condensation section 432 may be 30 to 400 mm, the width of the flow path guide wall 440 may be 5 to 20 mm, and an interval between the flow path guide walls 440 may be 10 to 50 mm.

In the arrangement explained above, the contact area and contact time between the steam and the condenser 400 can be increased while the water vapor moves along the zigzag-shaped condensation section 432 in the condensation space 420, and the heat exchange between the water vapor and condenser 400 and the condensation of the water vapor can be effectively performed.

In addition, by inclinedly forming the flow path guide wall 440, the condensed water formed in the condensation space 420 can move downwards along the surface of the flow path guide wall 440, and can move smoothly in the direction of gravity without accumulating inside the condenser 400, and residual water can be effectively prevented from occurring inside the condenser 400.

The introduction section 431 may extend upwards from the condenser inlet 450 to an upper start part of the condensation section 432.

The condenser outlet 460 forms an outlet of the condenser 400 through which air inside the condenser 400 is discharged.

The condenser outlet 460 may be formed in the lower part of the condenser 400. The condenser outlet 460 may be formed in a lower side of the condensation section 432.

However, the condenser outlet 460 may be formed in an area higher than the condensed water outlet 470. Accordingly, the condensed water inside the condenser 400 may be discharged through the condensed water outlet 470, and the air inside the condenser 400 may be discharged through the condenser outlet 460.

Meanwhile, in the shoes care device 1 according to one embodiment of the present invention, the inside of the condenser 400 and the inside of the module housing 200 may be configured to communicate with each other through the condenser outlet 460.

In the shoes care device 1 according to one embodiment of the present invention, the condenser outlet 460 may be formed in a position and a height corresponding to the first condensed water discharge hole 233, and the condenser outlet 460 and the first condensed water discharge hole 233 may be coupled to each other.

In the air introduced into the condenser 400, the condensed water moves to the sump 600 through the condensed water outlet 470, and uncondensed air may flow back into the module housing 200 through the condenser outlet 460.

High-temperature and humid air inside the module housing 200 forming the conversion flow path F10a flows into the condenser 400 of the regeneration path F20, the condensed water inside the condenser 400 moves to the sump 600, the uncondensed air can be resupplied into the module housing 200 forming the conversion flow path F10a, and the air is condensed while being circulated in the module housing 200 and the condenser 400.

The condenser communication port 465 communicates with the third module chamber 214 of the module housing 200 at a lower side of the condensation space. That is, the inside of the condenser 400 and the inside of the module housing 200 may be configured to communicate with each other through the condenser communication port 465.

The condenser communication port 465 may be formed directly above the condensed water outlet 470.

In the shoes care device 1 according to one embodiment of the present invention, the condenser communication hole 465 may be formed in a position and a height corresponding to the second condensed water discharge hole 234, and the condenser communication hole 465 and the second condensed water discharge hole 234 may be coupled to each other.

The condensed water inside the third module chamber 214 flows into the condenser 400 through the second condensed water discharge hole 234 and the condenser communication port 465, and moves to the sump 600 through the condensed water outlet 470.

The shoes care device 1 according to one embodiment of the present invention may include a condenser connection portion 490.

The condenser connection portion 490 may form a flow path directly connecting the condenser 400 of the first management device 2a and the condenser 400 of the second management device 2b.

The condenser connection portion 490 may be formed in the form of a pipe, a hose, etc. In the shoes care device 1 according to one embodiment of the present invention, when the condenser 400 of the first management device 2a is disposed on the upper side of the condenser 400 of the second management device 2b and the sump 600 is disposed on the lower side of the condenser 400 of the second management device 2a, the condensed water inside the condenser 400 of the first management device 2a may move into the condenser 400 of the second management device 2b through the condenser connection portion 490 and then move the sump 600.

For connection with the condenser connection portion 490, a condenser connector 491 may be formed in the condenser 400. The condenser connector 491 forms an inlet through which condensed water flows into the condenser 400.

The condenser connector 491 may be formed adjacent to the condensation space 420 of the condenser 400. The condenser connector 491 may be formed adjacent to the condensed water outlet 470 of the condenser 400.

When the condenser 400 of the first management device 2a is disposed above the condenser 400 of the second management device 2b and the sump 600 is disposed below the condenser 400 of the second management device 2b, the condenser connection portion 490 may be configured to connect the condensed water outlet 470 of the condenser 400 of the first management device 2a and the condenser connector 491 of the condenser 400 of the second management device 2b. In this case, the condenser connector 491 is not formed in the condenser 400 of the first management device 2a, or the condenser connector 491 of the condenser 400 of the first management device 2a may be blocked with a separate stopper.

The condensed water inside the condenser 400 of the first management device 2a may move into the condenser 400 of the second management device 2b through the condenser connection portion 490 and then move to the sump 600.

Accordingly, when the first management device 2a and the second management device 2b are arranged vertically in the shoes care device 1, a flow path (such as a hose) connecting the condenser 400 of the second management device 2b and the sump 600 may be used as a flow path configured to discharge the condensed water of the first management device 2a. Hence, the length of the flow path (hose, etc.) required for discharging the condensed water can be formed short on the whole, and only the condenser 400 of the second management device 2b may be directly connected to the sump 600 to discharge the condensed water of all the condensers 400. As a result, since an overall coupling structure can be simplified, which makes it easy to assemble and maintain the shoes care device 1, and the condensed water may be easily discharged.

Most of the water vapor in the air passing through the condenser 400 is condensed and moves to the sump 600 through the condensed water outlet 470. However, some small droplets condensed in the air may not be discharged through the condensed water outlet 470.

FIG. 35 is a cross-sectional view illustrating a part of the first module chamber 212 of the module housing 200 in the shoes care device 1 according to the present invention.

The blowing part 310 forms a part of the connection path F10. The blowing part 310 is configured to generate an air flow in the connection path F10.

The blowing part 310 may include the blowing fan 313, a blowing housing 311, and a blowing motor 314.

The blowing fan 313 may be configured to rotate around a rotation axis 313a in the third direction (Z direction) perpendicular to the first direction (X direction). The blowing motor 314 of the blowing part 310 rotates the blowing fan 313.

The blowing housing 311 is configured to accommodate the blowing fan 313. The blowing housing 311 may have a substantially round shape around the rotation axis 313a of the blowing fan 313. The blowing housing 311 may be formed in a circular shape or a spiral shape around the rotation axis 313a of the blowing fan 313.

An inlet 311a of the blowing part 310 is formed on a bottom surface of the blowing housing 311, and air flows into the blowing housing 311 below the bottom surface of the blowing housing 311.

The size of the blowing housing 311 in the horizontal direction may be formed larger than the size of the blowing housing 311 in the vertical direction. The size of the blowing housing 311 in the horizontal direction may be made more than twice the size of the blowing housing 311 in the vertical direction.

In the shoes care device 1 according to one embodiment of the present invention, when the blowing fan 313 rotates around a vertical rotation axis to allow the module housing 200 to have a relatively low vertical height, the diameter of the blowing housing 311 and the diameter of the blowing fan 313 can be formed sufficiently large, and the flow rate of air transported by the blowing part 310 can be sufficiently increased.

The blowing housing 311 may communicate with the first module chamber 212 on the rotating axis 313a of the blowing fan 313, and the edge thereof may be connected to and communicate with the blowing duct 312. The blowing duct 312 forms an outlet 311b of the blowing part 310.

Accordingly, the air below the blowing housing 311 in the first module chamber 212 moves upwards near the rotation axis 313a of the blowing fan 313 and flows into the blowing housing 311, and the air inside the blowing housing 311 is pressurized to the edge of the blowing housing 311 depending on the rotation of the blowing fan 313 and moves along the circumferential direction of the blowing housing 311 toward the blowing duct 312.

The blowing housing 311 includes the blowing duct 312 forming the outlet 311b of the blowing housing 311. The blowing duct 312 may extend in a horizontal direction from the blowing housing 311. The blowing duct 312 may be formed substantially along the second direction (Y direction). The blowing duct 312 may extend to the second module chamber 213, and all air discharged from the blowing part 310 through the blowing duct 312 may move to the second module chamber 213.

The blowing housing 311 and the blowing duct 312 may form a spiral passage around the rotating axis 313a of the blowing fan 313 together such that, when the blowing fan 313 rotates, the air inside the blowing housing 311 naturally moves to the blowing duct 312.

Along the rotation direction of the blowing fan 313, the distance in the radial direction from the rotation axis 313a of the blowing fan 313 may be sequentially increased from the blowing housing 311 to the blowing duct 312. In one embodiment, as illustrated in FIG. 9, the distance from the rotation axis 313a of the blowing fan 313 to the outer edges of the blowing housing 311 and the blowing duct 312 may be configured to increase sequentially in the clock direction.

In addition, the second module chamber 213 may be configured to extend from an end of the blowing duct 312.

In addition, the direction of sequentially connecting the first module chamber 212, the second module chamber 213, the third module chamber 214 and the dry air outlet 231 may be configured to correspond to the direction of the movement of air around the rotation axis of the blowing fan 313 in the blowing housing 311.

Based on FIG. 9, the rotation direction of the blowing fan 313 may be configured to be clockwise, the distance from the rotation axis 313a of the blowing fan 313 to the outer edges of the blowing housing 311 and the blowing duct 312 may be configured to sequentially increase in the clockwise direction and have a spiral form, and the direction of sequentially connecting the first module chamber 212, the second module chamber 213, the third module chamber 214 and the dry air outlet 231 may be configured to be clockwise.

Accordingly, the air passing through the blowing housing 311 and the blowing fan 313 may move in the clockwise direction inside the module housing 200 toward the dry air outlet 231 or the wet air outlet 232, and the air may move naturally along the formation direction of the flow path (the convention flow path F10a) inside the module housing 200.

In addition, by performing the blowing part 310 and the blowing duct 312 as described above, the flow rate of the air supplied to the third module chamber 214 can be stably secured, and sufficient air can be supplied to the dehumidifying part 330.

In addition, while the air moving through the third module chamber 214 smoothly passes through the dehumidifying part 330, a flow path resistance of the air passing through the dehumidifying part 330 can be prevented from unnecessarily increasing.

In the shoes care device 1 according to one embodiment of the present invention, the blowing part 310 may be spaced apart from the bottom of the first module chamber 212 such that air in the first module chamber 212 flows into the blowing part 310 from the lower side of the first module chamber 212. Accordingly, even if condensed water is generated inside the first module chamber 212, the condensed water can move along the bottom surface of the first module chamber 212 and be discharged to the outside of the module housing 200, and the residual water can be prevented or minimized in the blowing part 310.

In the shoes care device 1 according to one embodiment of the present invention, the direction of sequentially connecting the first module chamber 212, the second module chamber 213, the third module chamber 214 and the dry air outlet 231 may correspond to the direction of movement of air around the rotation axis of the blowing fan 313 in the blowing housing 311. Accordingly, the air introduced into the blowing housing 311 can naturally move into the module housing 200 in a predetermined direction from the blowing housing 311 to the second module chamber 213, the third module chamber 214 and the dry air outlet 231 with respect to the rotation axis of the blowing fan 313. Accordingly, a smooth air flow may be achieved in the module housing 200, and an unintentional increase in flow resistance can be prevented.

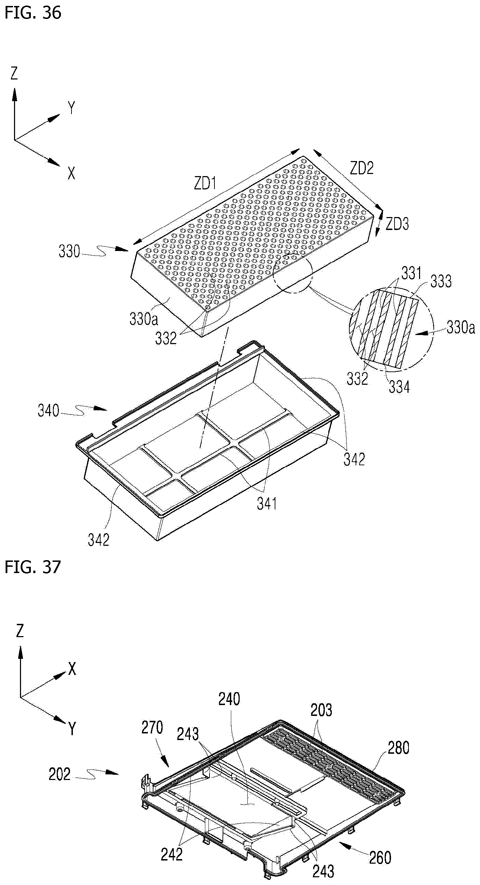

FIG. 36 is a perspective view illustrating the dehumidifying part 330 and a dehumidifying material housing 340 according to one embodiment of the present invention.

FIG. 37 is a bottom perspective view illustrating the module cover 202 according to one embodiment of the present invention.

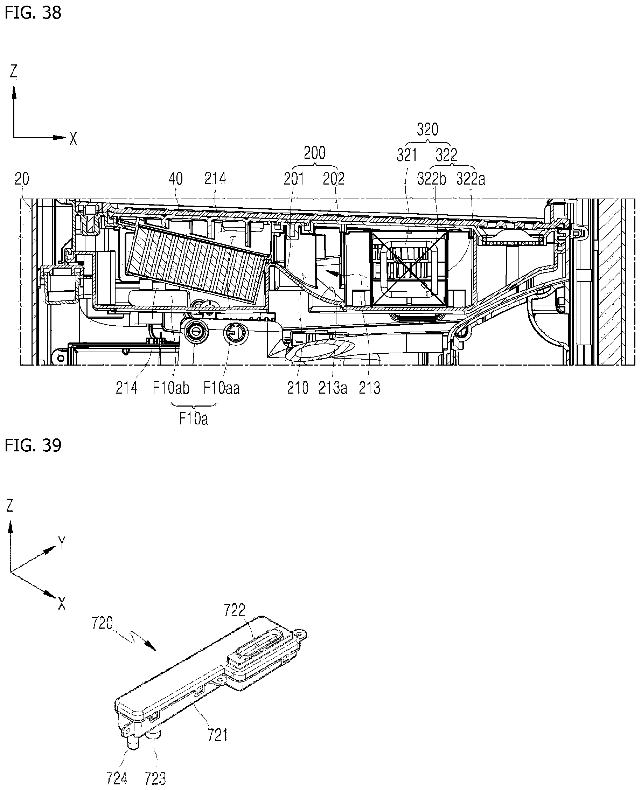

FIG. 38 is a cross-sectional view illustrating the third module chamber 214 of the module housing 200 in the shoes care device 1 according to the present invention.

The dehumidifying part 330 according to one embodiment of the present invention may be configured to have a predetermined thickness and length. In one embodiment, as described above, the dehumidifying part 330 may be configured to have a substantially hexahedral shape.

Accordingly, the dehumidifying part 330 may have a predetermined length, width, and thickness.

Each of a length ZD1 and a width ZD2 of the dehumidifying part 330 may be formed longer than a thickness ZD3 of the dehumidifying part 330. In one embodiment, the length ZD1 and the width ZD2 of the dehumidifying part 330 be made more than twice the thickness ZD3 of the dehumidifying part 330. In addition, the length ZD1 of the dehumidifying part 330 may be formed longer than the width ZD2 of the dehumidifying part 330.

The dehumidifying part 330 includes an upper surface 333 and a lower surface 334 facing each other. Here, the upper surface 333 of the dehumidifying part 330 and the lower surface 334 of the dehumidifying part 330 are opposite surfaces facing each other in the thickness direction of the dehumidifying part 330.

As described above, the dehumidifying part 330 is provided with the plurality of dehumidification through holes 332. The dehumidifying through hole 332 may be configured to penetrate the dehumidifying part 330 in a direction in which the upper surface 333 and the lower surface 334 of the dehumidifying part 330 are connected to each other.

In the shoes care device 1 according to one embodiment of the present invention, the dehumidifying part 330 may be accommodated inside the module housing 200 while being accommodated in the dehumidifying material housing 340.

The dehumidifying material housing 340 is formed in the form of a container capable of accommodating the dehumidifying part 330. In a state where the dehumidifying part 330 is accommodated in the dehumidifying material housing 340, an edge wall of the dehumidifying material housing 340 may be in close contact with the dehumidifying part 330. Accordingly, the dehumidifying part 330 can be prevented from being separated inside the dehumidifying material housing 340.

The dehumidifying material housing 340 has an upper side opened and a lower side opened. However, the lower side of the dehumidifying material housing 340 is provided with a support 341 that supports the dehumidifying part 330 such that the dehumidifying part 330 does not deviate downwards. The supports 341 may be disposed to cross each other in the form of a grid or a net. The gap (opening) between the supports 341 is formed sufficiently larger than the dehumidifying through hole 332 so as not to interfere with the flow of the air passing through the dehumidifying part 330.

In the shoes care device 1 according to one embodiment of the present invention, when the dehumidifying part 330 is disposed inside the module housing 200, the dehumidifying part 330 may be disposed so that the upper surface 333 and the lower surface 334 are inclined without being parallel to the horizontal direction.

The dehumidifying part 330 may be disposed in the third module chamber 214 in a form inclined downwardly toward the second module chamber 213. That is, the upper surface 333 and the lower surface 334 of the dehumidifying part 330 may be disposed to be inclined downwardly toward the second module chamber 213.

Since the dehumidifying part 330 is inclined downwardly toward the second module chamber 213 in the third module chamber 214, the air before penetrating the dehumidifying part 330 is disposed in an upper space of the dehumidifying part 330 in the third module chamber 214, and the air after penetrating the dehumidifying part 330 is disposed in a lower space of the dehumidifying part 330 in the third module chamber 214. Here, the upper space of the dehumidifying part 330 in the third module chamber 214 is defined as 'a first flow path F10aa', and the lower space of the dehumidifying part 330 in the third module chamber 214 is defined as 'a second flow path F10ab.

In addition, since the dehumidifying part 330 is disposed to be inclined in the third module chamber 214, the dehumidifying through hole 332 is also disposed to be inclined.

A cover partition wall 242 may be formed in the module cover 202.

The cover partition wall 242 is formed in a plate shape extending downwards from the edge of the dehumidifying material exit 240. The cover partition wall 242 may be formed in a substantially triangular plate shape.

The cover partition wall 242 may be configured such that a lower edge thereof is inclined downwards from the third module chamber 214 to the second module chamber 213. A pair of cover partition walls 242 may be provided to be spaced apart from each other in the left-right direction. The distance between the pair of cover partition walls 242 may be configured to correspond to the length of the dehumidifying part 330.

A lower locking portion 243 may be formed in a lower edge of the cover partition wall 242 and a front edge of the cover partition wall 242 in the first direction (X direction), and an upper locking portion 342 may be formed in an upper edge of the dehumidifying material housing 340 to get settled and locked in an upper side of the lower locking portion 243. Accordingly, when the dehumidifying material housing 340 is settled in the cover partition wall 242 in a state where the dehumidifying part 330 is accommodated in the dehumidifying material housing 340, the upper locking portion 342 of the dehumidifying material housing 340 is settled and assembled in an upper side of the lower locking portion 243 of the cover partition wall 242.

In addition, in this case, the cover partition wall 242 may block direct communication between the first flow path F10aa and the second flow path F10ab while the lower edge thereof is in close contact with the upper edge of the dehumidifying part 330.

By providing the cover partition wall 242 in the module cover 202, the air in the first flow path F10aa may pass through the dehumidifying part 330 over the entire area of the dehumidifying part 330 and move to the second flow path F10ab. In addition, a plurality of dehumidifying through holes 332 penetrating the dehumidifying part 330 in the thickness direction may be formed in the dehumidifying part 330, thereby increasing the contact area between the air passing through the third module chamber 214 and the dehumidifying material 331.

In one embodiment of the present invention, the dehumidifying part 330 is not disposed parallel to the horizontal direction and is inclined downwardly toward the second module chamber 213. Comparing this with the case where the dehumidifying part 330 is disposed horizontally, the size of the upper surface of the dehumidifying part 330 may be formed larger, and the entire volume of the dehumidifying part 330 may be increased. Accordingly, the amount of dehumidification of air by the dehumidifying part 330 may be increased.

As described above, as the dehumidifying part 330 is disposed to be inclined, the direction from the first flow path F10aa to the second flow path F10ab through the dehumidifying part 330 is configured not to be vertical but to be inclined. A rapid change in the direction of air in a path through which the air moves to the second module chamber 213, the first flow path F10aa, and the second flow path F10ab can be reduced to achieve a smooth movement of air.

Accordingly, the natural movement of air moving through the dehumidifying part 330 can be achieved, and an unnecessary flow path resistance can be minimized in the third module chamber 214.

In addition, while the air of the second module chamber 213 moves from the first flow path F10aa to the second flow path F10ab, moisture (small droplets or condensed water) may enter or occur in the third module chamber 214. The bottom surface of the dehumidifying part 330 may be naturally separated from the bottom of the third module chamber 214, and the air inside the third module chamber 214 may move downwards through the dehumidifying part 330 from the upper side of the third module chamber 214. Accordingly, the small droplets or the condensed water can move along the bottom surface of the third module chamber 214 without remaining or seeping into the dehumidifying part 330, and can be easily discharged toward the condenser 400.

In one embodiment, the dehumidifying part 330 may have a constant cross-section along the second direction (Y direction).

In another embodiment, the dehumidifying part 330 may be formed in a form where the thickness thereof is deformed along the second direction (Y direction).

In the shoes care device 1 according to one embodiment of the present invention, the first module chamber 212 and the second module chamber 213 are formed in front of the third module chamber 214 with respect to the first direction (X direction). That is, when the longitudinal direction of the dehumidifying part 330 is formed along the second direction (Y direction), the first module chamber 212 or the second module chamber 213 does not interfere with securing the length of the third module chamber 214, and the blowing part 310 or the heating part 320 does not interfere with securing the length of the dehumidifying part 330. Accordingly, a total length ZD1 of the dehumidifying part 330 may be formed longer than the length of each of the blowing part 310 and the heating part 320 with respect to the second direction (Y direction).

Accordingly, the length of the dehumidifying part 330 can be secured sufficiently long, the entire volume of the dehumidifying part 330 can be formed relatively large, and the dehumidifying amount per unit time of the dehumidifying part 330 can be improved.

In the shoes care device 1 according to one embodiment of the present invention, the length ZD1 of the dehumidifying part 330 may be longer than 1/2 of the length of each of the inner cabinet 100 and the module housing 200 with respect to the second direction (Y direction).

The dehumidifying part 330 may be spaced apart from the bottom of the third module chamber 214 so that the air inside the third module chamber 214 moves downwards through the dehumidifying part 330 from the upper side of the third module chamber 214. Since the dehumidifying part 330 is spaced apart from the bottom of the third module chamber 214, the air inside the third module chamber 214 can smoothly pass through the dehumidifying part 330, and the condensed water generated by penetrating the dehumidifying part 330 moves along the bottom surface of the third module chamber 214 and can be discharged outside the module housing 200.

In the shoes care device 1 according to one embodiment of the present invention, the dehumidifying part 330 provided in the third module chamber 214 may be disposed to be inclined. The dehumidifying part 330 may be disposed to be inclined downwardly toward the second module chamber 213. Accordingly, when the air moves from the second module chamber 213 to the third module chamber 214, the air can easily pass through the dehumidifying part 330. In addition, compared to the case where the dehumidifying part 330 is horizontally arranged, the area or volume of the dehumidifying part 330 can be further expanded, and the dehumidifying amount per unit time of the dehumidifying part 330 can be improved.

FIG. 39 is a perspective view illustrating the steam separator 720 according to one embodiment of the present invention.

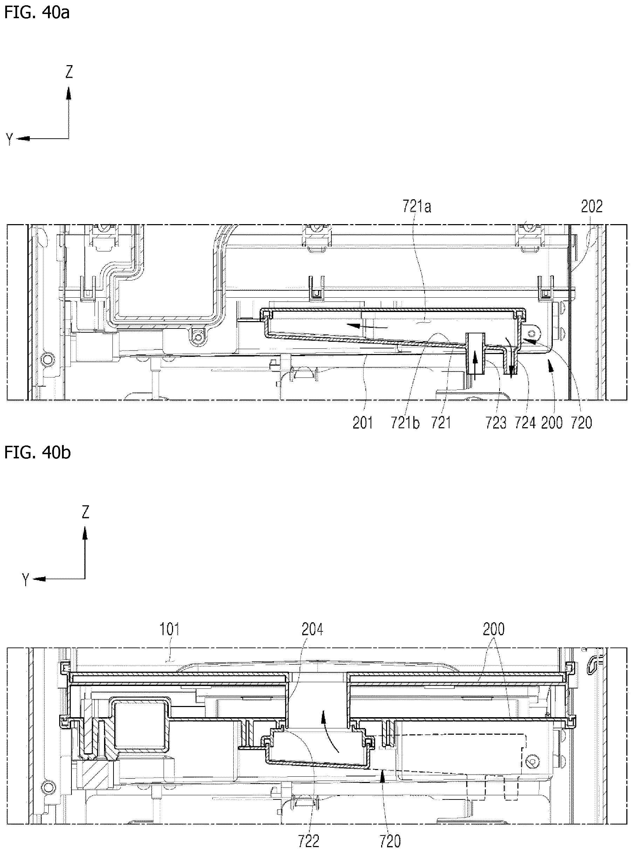

FIG. 40a is a cross-sectional view illustrating a part of a separating inlet 723 of the steam separator 720 in the shoes care device 1 according to one embodiment of the present invention.

FIG. 40b is a cross-sectional view illustrating a part of a separating connector 722 of the steam separator 720 in the shoes care device 1 according to one embodiment of the present invention.

The shoes care device 1 according to one embodiment of the present invention may further include the steam separator 720 installed adjacent to the steam inlet 204. The steam separator 720 is provided in each of the first management device 2a and the second management device 2b.

Most of the steam supplied from the steam generator 700 to the accommodation space 101 of the inner cabinet 100 is in a gaseous state, but the stream can be condensed during the movement to generate condensed water in a liquid state.

As described above, measures are needed to prevent moisture from leaving at an unintended part inside the shoes care device 1.

This is applicable to the flow path through which steam is supplied, and the condensed water in the steam needs to be prevented from being adsorbed and remaining on an inner surface of the flow path through which steam is supplied.

In addition, even if the condensed water in steam is supplied to the inside of the inner cabinet 100 without change, since the condensed water is not supplied to the shoes in the form of steam, it is difficult to properly perform the management on the shoes (sterilization treatment by high-temperature steam, swelling of shoe materials, etc.).

Therefore, preferably, the condensed water in the steam is removed through the steam separator 720 installed in the steam inlet 204 before the condensed water in the steam flows into the steam inlet 204.

In the shoes care device 1 according to one embodiment of the present invention, the steam separator 720 may include a separating base 721, the separating connector 722, the separating inlet 723, and a separating outlet 724.

The separating base 721 is formed in a housing shape forming a predetermined inner space 721a. The separating base 721 may have an inner area relatively larger than the hole formed by the steam inlet 204 in a plan view, and the separating base 721 may be fixed to the module housing 200 while covering the steam inlet 204 in the lower side of the steam inlet 204.

The separating inlet 723 forms an inlet through which steam flows into the steam separator 720. The separating inlet 723 may be connected to the steam valve 710, and the steam generated by the steam generator 700 may flow into the steam separator 720 through the separating inlet 723 after passing through the steam valve 710. The separating inlet 723 may be disposed lower than the separating connector 722. The separating inlet 723 may be formed in a shape opened vertically from the lower side of the separating base 721.

The separating inlet 723 may have a vertical tubular shape, and an upper end thereof may be formed higher than a bottom surface 721b of the separating base 721. The separating inlet 723 may be formed to protrude upward from the bottom surface 721b of the separating base 721. Accordingly, even if the condensed water occurs inside the separating base 721 and flows to the bottom surface 721b of the separating base 721, the condensed water may be prevented from flowing into the separating inlet 723, and all the condensed water may be discharged to the separating outlet 724, as described below.

The separating connector 722 forms an outlet through which steam inside the steam separator 720 is discharged. The steam inside the steam separator 720 may move to the steam inlet 204 through the separating connector 722. The separating connector 722 may be formed above the separating base 721. The separating connector 722 can be formed in a form opened from the upper side of the separating base 721 to the upper side in the vertical direction, and can be directly connected to and communicate with the steam inlet 204.

The separating outlet 724 forms an outlet through which condensed water inside the steam separator 720 is discharged. The separating outlet 724 is connected to the sump 600, and the condensed water inside the steam separator 720 may move to the sump 600 through the separating outlet 724. The separating outlet 724 may be disposed lower than the separating connector 722. The separating outlet 724 may be formed in a form opened from the lower side of the separating base 721 to the lower side in the vertical direction.

The separating outlet 724 may be formed in a lower end of the bottom of the separating base 721. That is, the separating outlet 724 may be formed at the lowest part among the bottom surfaces of the separating base 721. The bottom surface of the separating base 721 may be inclined downwardly toward the separating outlet 724. In one embodiment, the bottom surface of the separating base 721 may be inclined downward in the second direction (Y direction) or a direction opposite to the second direction (Y direction), and the separating outlet 724 may be formed on the bottom surface of the separating base 721 at a front end or a rear end of the second direction (Y direction).

The steam flowing into the steam separator 720 may be heated to a predetermined temperature, and may be above ordinary temperature (e.g., 20±5°C). Since this steam has a strong tendency to rise, it can move naturally through the separating connector 722 formed upwards from the upper side of the separating base 721.

Some of the steam flowing into the steam separator 720 may be cooled and condensed inside the separating base 721, and the condensed water may flow along the bottom surface of the separating base 721, and may be discharged to the outside of the steam separator 720 through the separating outlet 724 and move to the sump 600.

As described above, in the shoes care device 1 according to one embodiment of the present invention, since the steam separator 720 is provided with the separating connector 722 and the separating outlet 724 separately, when the steam and condensed water are present together, each of them can move through an individual path

In addition, the separating connector 722 is formed in the upper side of the steam separator 720, while the separating outlet 724 is formed in the lower side of the steam separator 720. Accordingly, according to the properties of the steam and the condensed water, the water vapor and condensed water are discharged in opposite directions, and residual water can be prevented from occurring in the flow path through which the steam is supplied.

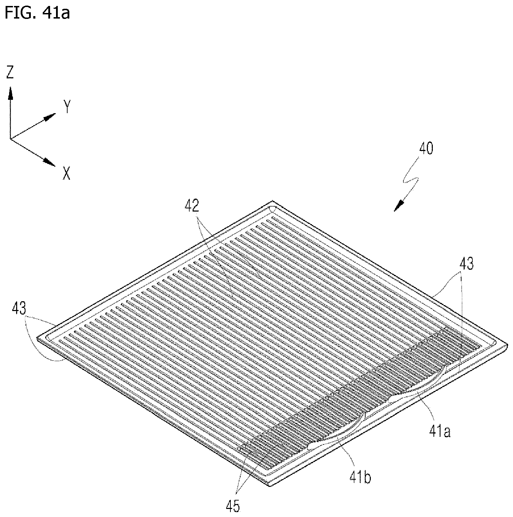

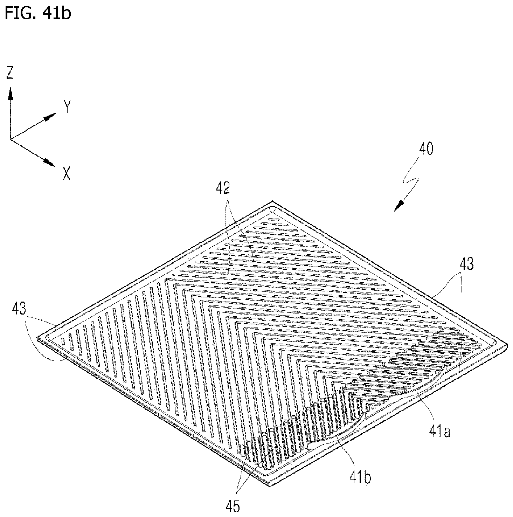

FIGS. 41a and 41b are perspective views illustrating the main shelf 40 according to one embodiment of the present invention.

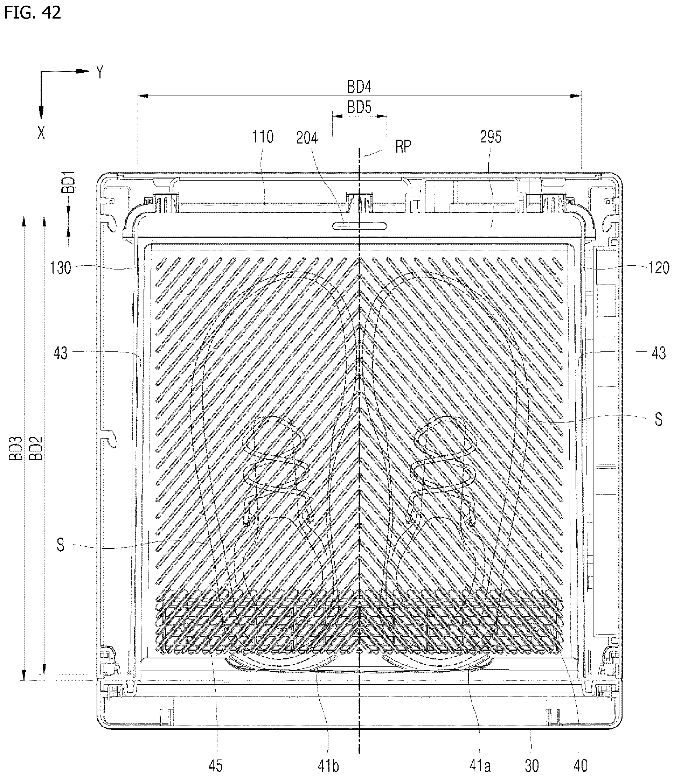

FIG. 42 is a cross-sectional view illustrating the shoes care device 1 according to one embodiment of the present invention, and schematically illustrating a state in which the shoes S are settled on an upper side of the main shelf 40.

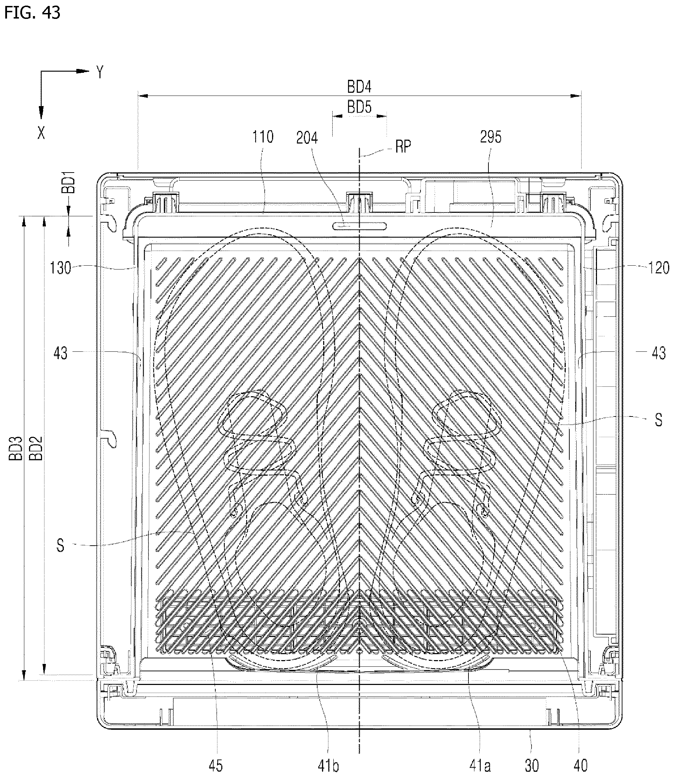

FIG. 43 is a cross-sectional view illustrating the shores care device 1 according to one embodiment of the present invention and is a view schematically illustrating a state in which the shoes S having a larger size than that of FIG. 42 is settled on the upper side of the main shelf 40.

As mentioned above, the main shelf 40 is placed and used on the bottom of the inner cabinet 100, and when the main shelf 40 is placed on the bottom of the inner cabinet 100, the upper surface of the main shelf 40 forms the bottom surface of the accommodation space 101. In addition, the module cover 202 of the module housing 200 may form the bottom surface of the inner cabinet 100. In this case, the main shelf 40 is settled on the upper side of the module housing 200 (the upper surface of the module cover 202.

The upper surface of the main shelf 40 forms the bottom surface of the accommodation space 101 and is configured to be inclined downwardly toward the first direction (X direction).

The main shelf 40 may be formed such that a pair of shoes S are settled on the upper surface thereof. The main shelf 40 is substantially formed in a thin and wide plate shape.

The shoes care device 1 according to one embodiment of the present invention may include a pair of first guide rib 41a and second guide rib 41b such that the position where the shoes are placed is guided to the user.

The first guide rib 41a and the second guide rib 41b may guide the settling position and direction of the shoes S such that the shoes S can be placed on the main shelf 40 to match or substantially match the front and rear directions of the shoes S to the first direction (X direction) when the user places the shoes S on the bottom of the accommodation space 101.

The first guide rib 41a and the second guide rib 41b are formed on the bottom surface of the inner cabinet 100. The first guide rib 41a and the second guide rib 41b may be formed on the upper surface of the main shelf 40.

Each of the first guide rib 41a and the second guide rib 41b are formed to support the front end or the rear end of the shoes S. Each of the first guide rib 41a and the second guide rib 41b may correspond to a front edge shape or a rear edge shape of the shoes S. Each of the first guide rib 41a and the second guide rib 41b may be formed in a curved shape that is concave in the first direction (X direction). Each of the first guide rib 41a and the second guide rib 41b may be formed in an arc shape.

Each of the first guide rib 41a and the second guide rib 41b is formed to protrude upwards from the bottom surface of the accommodation space 101. When the first guide rib 41a and the second guide rib 41b are formed in the main shelf 40, the first guide rib 41a and the second guide rib 41b protrude upwards from the upper surface of the main shelf 40.

The protruding heights of the first guide rib 41a and the second guide rib 41b may be variously formed according to embodiments. The protruding heights of the first guide rib 41a and the second guide rib 41b may be 3 to 20 mm.

The first guide rib 41a and the second guide rib 41b are positioned at different sides based on the reference plane RP. The first guide rib 41a and the second guide rib 41b may be symmetrical with respect to the reference plane RP.

The first guide rib 41a and the second guide rib 41b are formed in front of the accommodation space 101 based on the first direction (X direction). Based on the first direction (X direction), when the first guide rib 41a and the second guide rib 41b are formed relatively ahead, the steam inlet 204 may be formed relatively in the rear.

In the shoes care device 1 according to one embodiment of the present invention, the steam inlet 204 is formed on the bottom of the accommodation space 101 or formed adjacent to the bottom. The steam inlet 204 may be formed in the rear center of the accommodation space 101 based on the first direction (X direction). When the reference plane RP is a center surface that bisects the left and right sides of the shoes care device 1, the reference plane RP crosses the steam inlet 204.

Opposite sides of the accommodation space 101 may be symmetrical with respect to the reference plane RP.

Based on the rear end of the bottom surface of the accommodation space 101 in the first direction (X direction), when a distance to the steam inlet 204 is BD1, a distance to the first guide rib 41a and the second guide rib 41b is BD2, a distance to the front end of the bottom surface of the accommodation space 101 is BD3, the BD1 may be 1/10 or less of the BD3, and the BD2 may be 9/10 to 10/10 of the BD3.

When the steam inlet 204 is formed at the rearmost portion of the bottom surface of the accommodation space 101 based on the first direction (X direction), the first guide rib 41a and the second guide rib 41b may be formed in the frontmost part.

According to the shoes care device 1 according to one embodiment of the present invention, first of all, since the shoes are placed and managed on the bottom of the accommodation space 101, it is convenient to enter or withdraw the shoes from the accommodation space 101, and the shoes of various sizes may be easily managed. In addition, the steam generated by the steam generator 700 may be dispersed into the inner cabinet 100 to refresh the shoes S, and the air inside the inner cabinet 100 may be circulated through the connection path F10 to dehumidify the air.

Usually, since the shoes are made in a round shape in the front and back, or the width thereof becomes narrower toward the front and back, when a pair of shoes (S) is placed side by side, a space is formed between the front or rear ends of the pair of shoes S. In one embodiment of the present invention, since the steam inlet 204 is located in the rear center of the accommodation space 101 based on the first direction (X direction), when the pair of shoes is placed on the bottom of the accommodation space 101, the possibility of covering the steam inlet 204 by the shoes may be minimized, and the steam may be uniformly supplied to each of the shoes.

Unlike the embodiment of the present invention, when the steam inlet 204 is formed by being biased to the left or right in the accommodation space 101, more steam supplied through the steam inlet 204 may be further supplied to any one shoe, and accordingly, uniform shoe management may not be achieved.

According to one embodiment of the present invention, even when high-temperature steam is discharged through the steam inlet 204, the steam is directly discharged to the bottoms of the shoes S, thereby minimizing the possibility of ununiformly treating the pair of shoes or damaging the shoes.

According to the shoes care device 1 according to one embodiment of the present invention, when the user places the pair of shoes on the bottom of the accommodation space 101, the pair of shoes are on the bottom of the accommodation space 101 in a state where one shoe is supported by the first guide rib 41a and the other shoe is supported by the second guide rib 41b. Accordingly, two shoes are located on different sides based on the reference plane RP, and the steam inlet 204 is located at one part of the reference plane RP (see FIG. 42).

When the shoe size is very large (e.g., when the shoe length is longer than the length of the main shelf 40 with respect to the first direction (X direction)), a pair of shoes may be placed diagonally apart toward the back of the first direction (X direction) (see FIG. 43). In this case, the steam inlet 204 is located between the pair of shoes S. As such, according to one embodiment of the present invention, when the size of shoes is very large, the possibility of covering the steam inlet 204 by the shoes or of placing the shoes very close to the steam inlet 204 can be minimized.

Unlike the embodiment of the present invention, when the steam inlet 204 is formed to be biased to the left or right in the accommodation space 101, if the size of the managed shoes is very large, more steam supplied through the steam inlet 204 may be supplied to any one shoe, or the steam inlet 204 may be covered by the shoes, and when high-temperature steam is discharged from the steam inlet 204, this can damage the shoes.

In the shoes care device 1 according to one embodiment of the present invention, as described above, the outlet 203 may be formed in front of the upper surface of the module housing 200 in the first direction (X direction). In this case, a shelf hole 45 vertically penetrated directly above the outlet 203 is formed in the main shelf 40.

A net such as a grid shape or a mesh shape may be formed in the shelf hole 45.

Accordingly, a plurality of shelf holes 45 are provided.

The plurality of shelf holes 45 may be repeatedly arranged along the second direction (Y direction). The shape formed by the plurality of shelf holes 45 on the whole may be formed in a long shape along the second direction (Y direction) in the main shelf 40.

An area in which the shelf hole 45 is formed in a plan view may be formed to coincide with or substantially coincide with an area in which the outlet 203 is formed.

The first guide rib 41a and the second guide rib 41b are formed ahead of the shelf hole 45 in the first direction (X direction).

As described above, the entire area of the main shelf 40 in the front-rear direction may be used as an area where the shoes are settled, based on the first direction (X direction), and the condensed water on the upper surface of the main shelf 40 may flow along the upper surface of the main shelf 40 and flow into the shelf hole 45 and the outlet 203.

The main shelf 40 may include a plurality of main isolation ribs 42. The plurality of main isolation ribs 42 protrude upwards from the upper surface of the main shelf 40 and are spaced apart from each other. The main isolation rib 42 may be formed along the first direction (X direction) and may be formed in a direction inclined with the first direction (X direction).

The protruding height of the main isolation rib 42 may be formed lower than the protruding heights of the first guide rib 41a and the second guide rib 41b.

As each of the main isolation ribs 42 is spaced apart from each other, a gap (space or valley) is repeatedly formed between the main isolation ribs 42.

The main isolation rib 42 may be formed in most areas of the upper surface of the main shelf 40. The main isolation rib 42 may be formed in most areas of the upper surface of the main shelf 40, except the edge of the main shelf 40 (the main water stop rib 43).

When the shoes are settled on the upper side of the main shelf 40, one part of the bottom surface of the shoes S comes into contact with the main isolation rib 42, and the other part does not come into contact with the main isolation rib 42. A plurality of gaps are formed between the main isolation ribs 42, and steam or air may be supplied to the bottom of the shoes through such gaps during the management process of the shoes S. In addition, with the formation of the gaps, the condensed water on the upper surface of the main shelf 40 may flow without blocking the flow of the condensed water by the shoes.

The main shelf 40 is configured to include the main water stop rib 43 that forms an edge and protrudes upwards. The main water stop rib 43 may be formed on the entire edge of the main shelf 40. When the main shelf 40 in a plan view is formed in a rectangular shape, the main water stop rib 43 is formed in a rectangular shape.

The main water stop rib 43 may be formed in close contact with or close to the inner cabinet 100 at both left and right sides in the first direction (X direction).

With the formation of the main water stop rib 43, the condensed water on the upper side of the main shelf 40 may remain on the upper side of the main shelf 40 or flow toward the shelf hole 45 without departing from the main water stop rib 43 to the outside of the main self 40.

The steam inlet 204 may be located behind the main shelf 40 in the first direction (X direction). The steam inlet 204 may penetrate in the vertical direction, and the upper end thereof may be higher than the bottom of the main shelf 40.

The shoes care device 1 may include a bottom bar 295. The steam inlet 204 is formed on an upper surface of the bottom bar 295.

The bottom bar 295 may form the rearmost portion of the bottom surface of the accommodation space 101. The bottom bar 295 may be formed in a long shape along the second direction (Y direction).

The bottom bar 295 is interposed between the inner cabinet 100 and the main water stop rib 43 at the rear in the first direction (X direction). The bottom bar 295 may be in close contact between the inner cabinet 100 and the main water stop rib 43.

In one embodiment, the bottom bar 295 may be formed integrally with the module housing 200.

In another embodiment, the bottom bar 295 may be formed separately from the module housing 200 and then coupled to the module housing 200. The bottom bar 295 may be coupled to the module cover 202 of the module housing 200 at the rear in the first direction (X direction).

The steam inlet 204 may be formed at the rear of the module housing 200 based on the first direction (X direction), and can be formed in a form that vertically penetrates the module housing 200 and the bottom bar 295.

The upper surface of the bottom bar 295 may be formed to be higher than or equal to the upper surface of the main water stop rib 43.

The first inner side plate 120 of the inner cabinet 100 forms a wall surface on the side where the first guide rib 41a is located based on the reference plane RP.

The second inner side plate 130 of the inner cabinet 100 forms a wall surface on the side where the second guide rib 41b is located based on the reference plane RP.

The inner rear plate 110 of the inner cabinet 100 forms a wall surface at the rear in the first direction (X direction) and connects the first inner side plate 120 and the second inner side plate 130.

In the shoes care device 1 according to one embodiment of the present invention, the first guide rib 41a may be formed to be more biased toward the reference plane RP than the first inner side plate 120, and the second guide rib 41b may be formed to be more biased toward the reference plane RP than the second inner side plate 130.

Accordingly, for ordinary-sized shoes, a pair of shoes S may be disposed side by side around the reference plane RP, and for very large-sized shoes, a pair of shoes S may be placed in a form opened to the rear in the first direction (X direction).

A length BD5 of the steam inlet 204 in the second direction (Y direction) may be configured to be 1/15 to 1/5 of a distance BD4 between the first inner side plate 120 and the second inner side plate 130. The length BD5 of the steam inlet 204 in the second direction (Y direction) may be configured to be 1/5 to 1/3 of the distance BD4 between the first inner side plate 120 and the second inner side plate 130.

According to the shoes care device 1 according to one embodiment of the present invention, foreign substances separated from the shoes during the shoe management process can be settled on the upper surface of the main shelf 40. Since the user can separate the main shelf 40 from the inner cabinet 100 to wash the main shelf 40, the shoes care device 1 can be easily maintained.

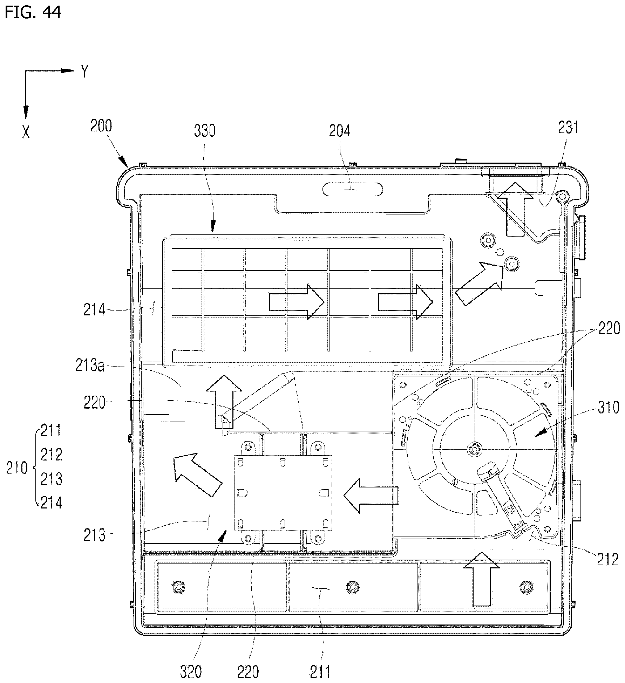

In the shoes care device 1 according to one embodiment of the present invention, the main shelf 40 is inclined downwards in the first direction (X direction), the shelf hole 45 is formed in the main shelf 40, and the condensed water on the upper surface of the main shelf 40 may flow into the module housing 200 through the shelf hole 45 and then move to the regeneration path F20. In addition, the shoes care device 1 may include the bottom bar 295, and the main shelf 40 may include the main water stop rib 43. In this case, the steam inlet 204 is formed on the upper surface of the bottom bar 295, the upper surface of the bottom bar 295 is formed higher than or equal to the upper surface of the main water stop rib 43, and the main water stop rib 43 is in close contact with to or close to the inner cabinet 100 on both left and right sides in the first direction (X direction). Accordingly, since the direction in which steam is discharged from the steam inlet 204 is not a direction directly facing the shoe, the stream is prevented from damaging the shoes. In addition, the condensed water condensed in the accommodation space 101 is located inside the main water stop rib 43 as the edge of the main shelf 40, or moves through a regeneration path, and the condensed water is easily managed in the shoes care device 1. FIG. 44 is a view illustrating the inside of the module housing 200 according to one embodiment of the present invention. FIG. 44 illustrates that a schematic flow direction of air inside the module housing 200 is indicated by an arrow.

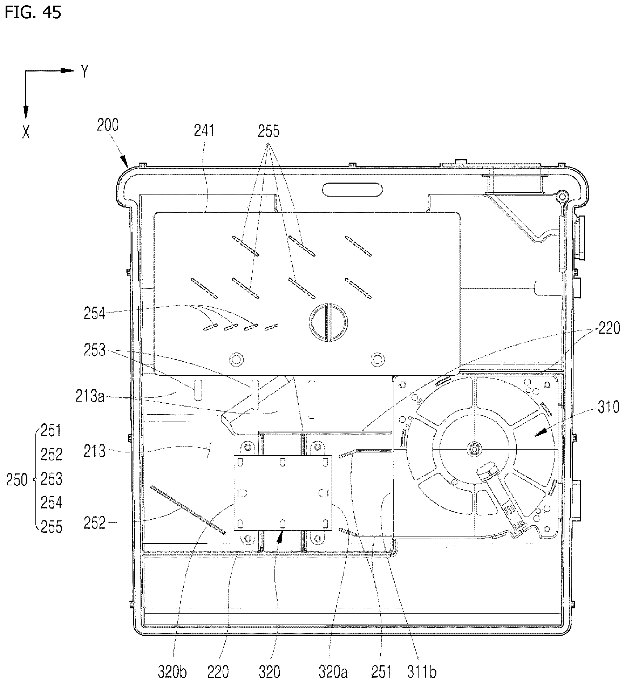

FIG. 45 is a view illustrating the inside of the module housing 200 according to one embodiment of the present invention. FIG. 45 illustrates the dehumidifying material cover 241 together.

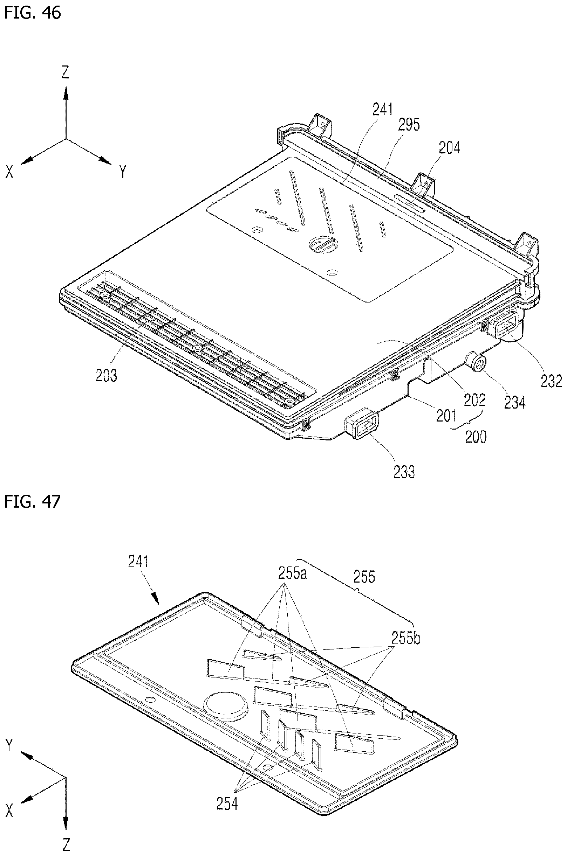

FIG. 46 is a perspective view illustrating the drying module DM according to one embodiment of the present invention.

FIG. 47 is a bottom perspective view illustrating the dehumidifying material cover 241 illustrated in FIG. 46.

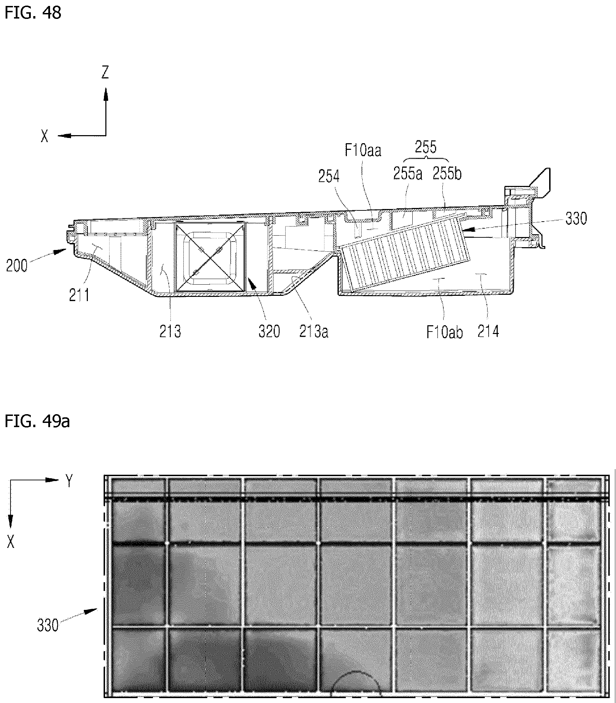

FIG. 48 is a cross-sectional view illustrating the drying module DM of FIG. 46.

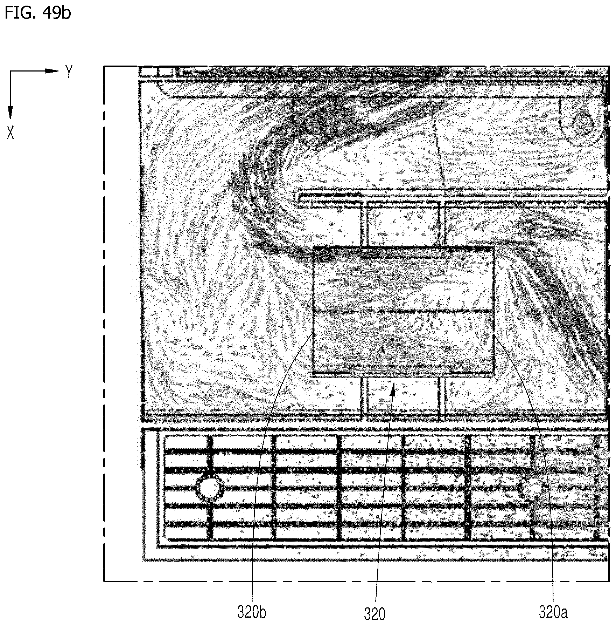

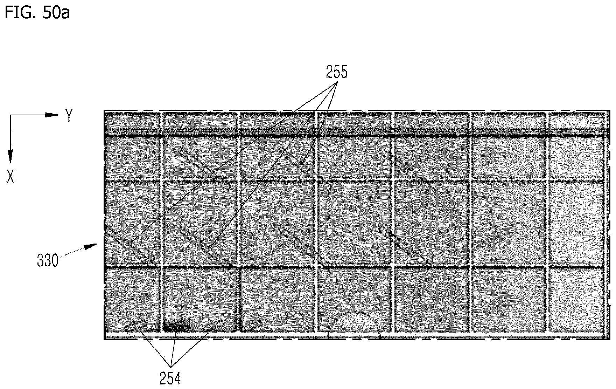

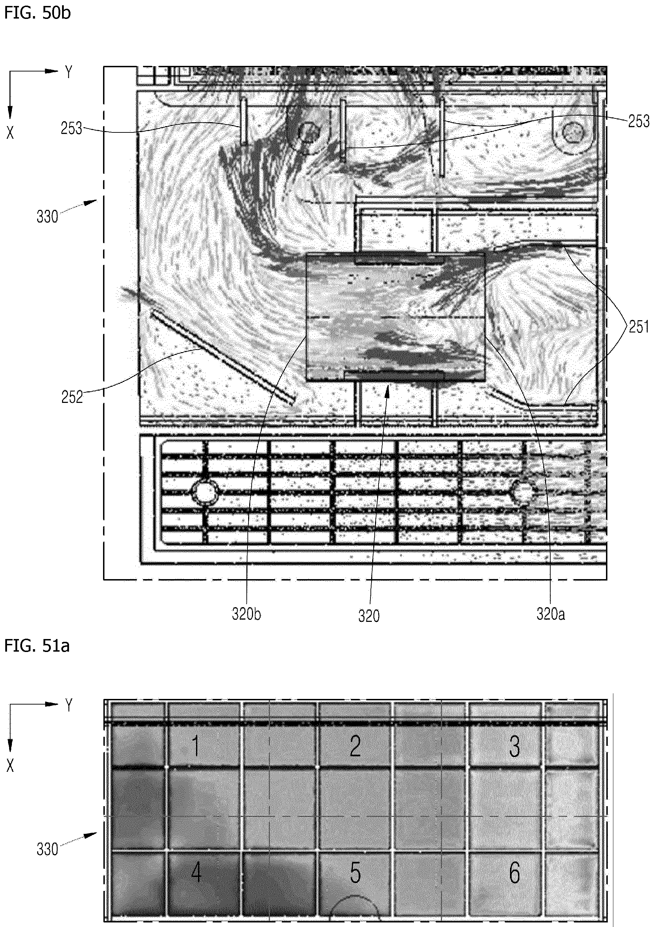

FIGS. 49a and 49b are views illustrating simulation results of air speed distribution when an inner rib 250 is not formed in the module housing 200 according to one embodiment of the present invention, and FIGS. 50a and 50b are diagrams illustrating simulation results of the air speed distribution when the inner rib 250 is formed in the module housing 200 according to another embodiment of the present invention.

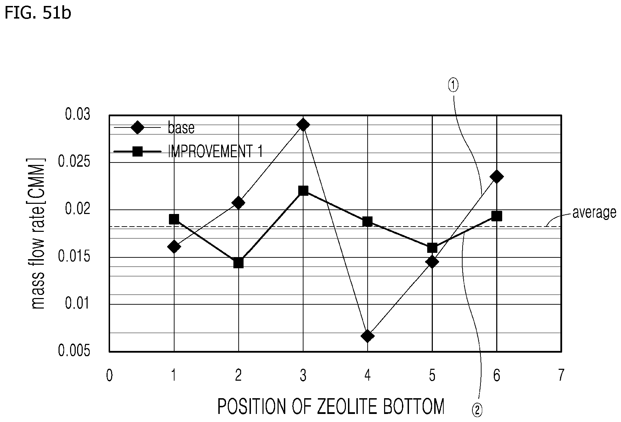

FIG. 51a is a view illustrating the entire area of the dehumidifying part divided into six areas (which are indicated by Nos. 1 to 6, and each of which is identified by one dotted line) in a plan view in the shoes care device 1 according to one embodiment of the present invention, and FIG. 51b is a view illustrating a mass flow rate in each area of FIG. 51a. In Fig. 51b, graph ① is a case where a fourth inner rib 254 and a fifth inner rib 255 are not formed, and graph ② is a case where the fourth inner rib 254 and the fifth inner rib 255 are formed.

As described above, the module housing 200 includes at least one module partition wall 220. The module partition 220 mutually partitions the first module chamber 212, the second module chamber 213, and the third module chamber 214 such that air moves sequentially through the first module chamber 212, the second module chamber 213, and the third module chamber 214.

In the shoes care device 1 according to one embodiment of the present invention, the module housing 200 may include at least one inner rib 250. The inner rib 250 is provided inside the module housing 200.

Each of the inner ribs 250 may be formed integrally with the module housing 200. Some or all of the inner ribs 250 may be formed integrally with the module case 201. Some or all of the inner ribs 250 may be formed integrally with the module cover 202. Some of the inner ribs 250 may be formed integrally with the dehumidifying material cover 241.

Each of the inner ribs 250 is formed in a plate shape inside the module housing 200 and is configured to guide the movement of air. That is, the inner rib 250 is configured such that the air inside the module housing 200 moves along the surfaces formed by each of the inner ribs 250.

Each of the inner ribs 250 may form a vertical surface. That is, the surface of the inner rib 250 may be formed in a vertical direction.

The inner rib 250 includes a first inner rib 251. The first inner rib 251 extends from the edge of the outlet 311b of the blowing part 310 to the edge of an inlet 320a of the heating part 320.

As described above, the outer flange 322a of the heater flange 322 may be formed in a tubular shape along the second direction (Y direction). In this case, in the outer flange 322a, the front in the second direction (Y direction) forms the inlet 320a of the heating part 320, and the rear in the second direction (Y direction) forms the outlet 320b of the heating part 320.

Two first inner ribs 251 can be provided, and the two first inner ribs 251 may be spaced apart from each other and formed on opposite sides of the outlet 311b of the blowing part 310. The two first inner ribs 251 may be formed in a shape where a gap between the first inner ribs 251 becomes narrow toward the inlet 320a of the heating part 320.

When air moves from the blowing part 310 to the heating part 320, the first inner rib 251 may prevent a vortex flow and avoid or minimize a head loss.

The inner rib 250 includes a second inner rib 252. The second inner rib 252 is formed in front of the outlet 320b of the heating part 320. The second inner rib 252 forms an inclined surface inclined from the second direction (Y direction) to the first direction (X direction). Accordingly, since the air passing through the outlet of the heating part 320 hits the second inner rib 252, the flow direction of the air can be naturally changed to the third module chamber 214 and the dehumidifying part 330.

Accordingly, with the formation of the second inner rib 252 in the module housing 200, the flow direction of the air from the heating part 320 to the dehumidifying part 330 can be effectively changed and the head loss can be minimized.

The module housing 200 may include a chamber inclined surface 213a. The chamber inclined surface 213a forms a bottom surface inclined upwards from the bottom surface of the second module chamber 213 to the dehumidifying part 330. The air that hits the second inner rib 252 and moves to the third module chamber 214 may move upwards along the chamber inclined surface 213a, and may also move to the upper side of the dehumidifying part 330.

The inner rib 250 includes a third inner rib 253. A plurality of third inner ribs 253 may be provided.

The third inner rib 253 may be formed between the second module chamber 213 and the third module chamber 214. The third inner rib 253 may be formed on the chamber inclined surface 213a.

The third inner rib 253 is formed parallel to the first direction (X direction), and the plurality of third inner ribs 253 are spaced apart from each other in the second direction (Y direction). Each of the third inner ribs 253 may have a length (a length in the first direction (X direction)) formed longer toward the second direction (Y direction).

The third inner rib 253 helps the air moving from the second module chamber 213 to the third module chamber 214 move parallel to the first direction (X direction), and allows the air to uniformly flow to the dehumidifying part 330 in the entire area of the dehumidifying part 330 the second direction (Y direction).

As described above, the dehumidifying part 330 may be disposed to be inclined downwardly toward the second module chamber 213. In this case, the dehumidifying part 330 and the dehumidifying material cover 241 are spaced apart from each other, and a space (the first flow path F10aa) is provided in the upper side of the dehumidifying part 330.

The inner rib 250 may include a fourth inner rib 254. The fourth inner rib 254 is disposed above the dehumidifying part 330 in the third module chamber 214. That is, the fourth inner rib 254 is disposed in the first flow path F10aa.

The fourth inner rib 254 is inclined from the first direction (X direction) to a direction opposite to the second direction (Y direction).

A plurality of fourth inner ribs 254 are provided and are repeatedly spaced apart from each other in the second direction (Y direction).

The inner rib 250 may include a fifth inner rib 255. The fifth inner rib 255 is disposed above the dehumidifying part 330 in the third module chamber 214. That is, the fifth inner rib 255 is disposed in the first flow path F10aa.

The fifth inner rib 255 is inclined from the first direction (X direction) to the second direction (Y direction).

A plurality of fifth inner ribs 255 are provided and are repeatedly spaced apart from each other in the second direction (Y direction).

The fifth inner rib 255 is positioned behind the fourth inner rib 254 with respect to the first direction (X direction). That is, the air moving from the second module chamber 213 to the third module chamber 214 may first pass through the fourth inner rib 254 and then through the fifth inner rib 255.

The vertical length of the fourth inner rib 254 may be formed longer than the vertical length of the fifth inner rib 255.

With respect to the second direction (Y direction), the fifth inner rib 255 positioned at the frontmost is positioned ahead of the fourth inner rib 254 at the frontmost, and the fifth inner rib 255 positioned at the rearmost is positioned behind or equal to the fourth inner rib 254 positioned at the rearmost.

In a plan view, the length of the fifth inner rib 255 is formed longer than the length of the fourth inner rib 254.

The fifth inner rib 255 may be divided into a fifth-first inner rib 255a and a fifth-second inner rib 255b. The fifth-first inner ribs 255a form a first row. The fifth-second inner rib 255b is positioned behind the fifth-first inner rib 255a with respect to the first direction (X direction) and forms a second row.

With respect to the second direction (Y direction), a length from the rear end of the dehumidifying part 330 to the fifth inner rib 255 positioned at the frontmost may be 1/2 to 4/5 of a length from the rear end of the dehumidifying part 330 to the front end thereof.

In the shoes care device 1 according to one embodiment of the present invention, the fourth inner rib 254 and the fifth inner rib 255 may be formed on the bottom surface of the module cover 202.

As described above, in the shoes care device 1 according to the embodiment of the present invention, the module cover 202 may include the dehumidifying material exit 240 and the dehumidifying material cover 241. In this case, the fourth inner rib 254 and the fifth inner rib 255 may be integrally formed on the bottom surface of the dehumidifying material cover 241.

In the shoes care device 1 according to one embodiment of the present invention, the fourth inner rib 254 forms a surface resistant to the air moving in the opposite direction to the first direction (X direction) from the upper side of the dehumidifying part 330. The fourth inner rib 254 may reduce the deviation of the flow rate between the air moving through the front part of the dehumidifying part 330 and the air moving through the rear part of the dehumidifying part 330 with respect to the first direction (X direction).

In addition, the fifth inner rib 255 forms a surface resistant to the air moving in a direction opposite to the first direction (X direction) from the upper side of the dehumidifying part 330. The fifth inner rib 255 may further reduce the deviation of the flow rate between the air moving through the front part of the dehumidifying part 330 and the air moving through the rear part of the dehumidifying part 330 with respect to the first direction (X direction).

In addition, the air moving from the second module chamber 213 to the third module chamber 214 may be widely dispersed along the second direction (Y direction) by the fifth inner rib 255. Accordingly, the air of the third module chamber 214 may pass through the dehumidifying part 330 at a uniform speed over the entire area of the dehumidifying part 330.

In FIGS. 49a and 50a, a blue (a dark color when an image is displayed in black and white) portion indicates a portion having a relatively low flow rate. In FIG. 49a, it can be seen that the flow rate of the lower left part is lower than other parts, and the deviation of the flow rate of the dehumidifying part 330 is slightly larger for each area on the plane. In FIG. 50a, it can be seen that the deviation of the flow rate of the dehumidifying part 330 is smaller for each area on the plane as compared to FIG 49a.

In FIGS. 49b and 50b, a red (dark color when an image is displayed in black and white) portion indicates a portion having a relatively high flow rate.

As a result of simulation of the shoes care device 1 according to one embodiment of the present invention, as compared to FIGS. 49a and 49b (when the inner rib 250 is not formed inside the module housing 200), FIGS 50a and 50b (when the inner rib 250 is formed inside the module housing 200) has confirmed that the wind volume is improved by about 0.7% inside the module housing 200. That is, it can be seen that the wind volume of the air moving in the module housing 200 in a case in which the inner rib 250 is formed is improved by about 0.7% as compared to a case in which the inner rib 250 is not formed, and the air inside the module housing 200 is smoother circulated.

Also, as a result of checking a pressure loss of the inlet (the first flow path F10aa) of the dehumidifying part 330 compared to the outlet 311b of the blowing part 310, as compared to the pressure loss of FIG. 49b (when the inner rib 250 is not formed inside the module housing 200), FIG. 50b (when the inner rib 250 is formed inside the module housing 200) has confirmed that the pressure loss was reduced by about 9%. That is, it can be seen that the pressure loss of air flowing inside the module housing 200 in a case in which the first inner rib 251, the second inner rib 252, and the third inner rib 253 are formed is reduced by about 9% as compared to a case in which the first inner rib 251, the second inner rib 252, and the third inner rib 253 are not formed.

In addition, as a result of checking a pressure loss of the outlet (the first flow path F10ab) of the dehumidifying part 330 compared to the inlet (the first flow path F10aa) of the dehumidifying part 330, as compared to the pressure loss of FIG. 49a (when the inner rib 250 is not formed inside the module housing 200), FIG. 50a (when the inner rib 250 is formed inside the module housing 200) has confirmed that the pressure loss was increased by about 27%. That is, it can be seen that the pressure loss of air flowing inside the module housing 200 in a case in which the fourth inner rib 254 and the fifth inner rib 255 are formed is increased by about 9% as compared to a case in which the fourth inner rib 254 and the fifth inner rib 255 are not formed, and the flow rate decreases when the air penetrates the dehumidifying part 330 in the third module chamber 214.

In addition, as shown in FIG. 51b in this case, it can be seen that when the fourth inner rib 254 and the fifth inner rib 225 are not formed (CD), the deviation of the mass flow rate of the dehumidifying part 330 for each area is relatively large, and when the fourth inner rib 254 and the fifth inner rib 225 are formed (②), the deviation of the mass flow rate of the third module chamber 214 for each area is relatively small.

When the fourth inner rib 254 and the fifth inner rib 255 are not formed (①), the mass flow rate is relatively very high in a third region of the dehumidifying part 330 and the mass flow rate is relatively very low in a fourth area. However, when the fourth inner rib 254 and the fifth inner rib 255 are formed (②), the mass flow rate is decreased in the third area of the dehumidifying part 330 and the mass flow rate is increased in the fourth area. The deviation for each area can be seen to decrease.

As such, it can be seen that when the fourth inner rib 254 and the fifth inner rib 255 are formed compared to when the fourth inner rib 254 and the fifth inner rib 255 are not formed, flow uniformity is excellent in each area of the dehumidifying part 330, and the air passes uniformly through each area of the dehumidifying part 330 in the third module chamber 214.

According to one embodiment of the present invention, with the formation of the fourth inner rib 254 and the fifth inner rib 255, the dehumidifying part 330 can be efficiently utilized.

As described above, according to one embodiment of the present invention, air may smoothly move in the module chamber by the inner rib 250, and an unnecessary head loss, a vortex flow, etc., may be prevented from occurring in the module chamber.

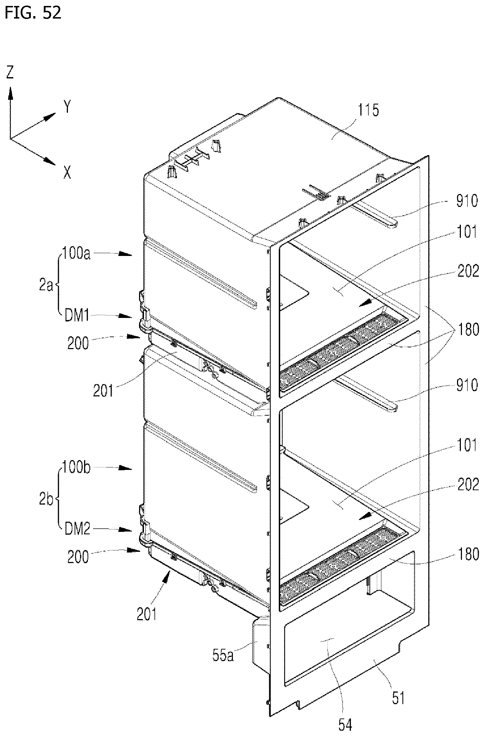

FIG. 52 is a perspective view illustrating a state in which inner cabinets 100a and 100b and the module housing 200 are coupled according to one embodiment of the present invention.

FIG. 53 is a perspective view illustrating the inner cabinets 100a and 100b illustrated in FIG. 52.

FIG. 54 is a perspective view of the inner cabinets 100a and 100b illustrated in FIG. 53 when viewed from the back.

As described above, it will be understood that the 'inner cabinet 100' described in one embodiment of the present invention means each of the 'first inner cabinet 100a' and the 'second inner cabinet 100b', except for a particularly limited case.

In the shoes care device 1 according to one embodiment of the present invention, the module housing 200 may be detachably coupled to the inner cabinet 100. The module housing 200 may be coupled to a lower side of the inner cabinet 100 while sliding in a horizontal direction. The module cover 202 is coupled to the lower end of the inner cabinet 100 so as to slide in the horizontal direction.

The module housing 200 may be coupled to a lower side of the inner cabinet 100 while moving in the first direction (X direction), and the module housing 200 may be separated from the inner cabinet 100 while moving an opposite direction to the first direction (X direction) in a state where the module housing 200 and the inner cabinet 100 are coupled to each other.

The module housing 200 may be coupled to the inner cabinet 100 while sliding in the first direction (X direction). To this end, the inner cabinet 100 may include sliding guides 160 and 170, and the module housing 200 may include sliders 260 and 270.

The sliders 260 and 270 are slidably moved along the formation direction of the sliding guides 160 and 170, and the sliders 260 and 270 and the sliding guides 160 and 170 are slidably coupled to each other.

According to one embodiment of the present invention, the module housing 200 may be formed directly under the inner cabinet 100 to prevent the connection path F10 from being unnecessarily elongated, and air in the accommodation space 101 may be rapidly dehumidified by the dehumidifying part 330. In addition, the inner cabinet 100 and the module housing 200 may be stably coupled to each other to form the shoes care device 1 with a simple structure.

In the shoes care device 1 according to one embodiment of the present invention, the blowing part 310, the heating part 320, and the dehumidifying part 330 are accommodated together in the module chamber 210 inside the module housing 200, and the module housing 200 is detachably coupled to the lower side of the inner cabinet 100. That is, the drying module DM is detachably coupled to the lower side of the inner cabinet 100. The drying module A (DM1) of the first management device 2a is detachably coupled to a lower side of the first inner cabinet 100a, and the drying module B (DM2) of the second management device 2b is detachably coupled to a lower side of the second inner cabinet 100b.

In the module housing 200, the module case 201 may be formed by injection molding, and the module case 201 may be integrally formed. The air inside the inner cabinet 100 moves to the module chamber 210 of the module housing 200, and the blowing part 310, the heating part 320, and the dehumidifying part 330, which are the main means for drying the air inside the inner cabinet 100 and the main means for regenerating the dehumidifying part 330, are disposed together in the module chamber 210 of the module housing 200. Accordingly, the condensed water generated inside the inner cabinet 100 may flow into the module chamber 210 inside the module housing 200, and the condensed water generated through the blowing part 310, the heating part 320, and/or the dehumidifying part 330 can be effective prevented from leaking from the module housing 200.

In addition, according to one embodiment of the present invention, the shoes care device 1 can be provided with a structure capable of easily managing and replacing the module housing 200 and the components accommodated therein.

Unlike one embodiment of the present invention, when the blowing part 310, the heating part 320, and the dehumidifying part 330 are accommodated in separate chambers physically separated from each other, and the chambers are coupled to each other, a potential leak at the coupling part of each chamber may not be excluded, and the coupling with the inner cabinet 100 may be cumbersome.

In the shoes care device 1 according to one embodiment of the present invention, the module housing 200 is coupled to the lower side of the inner cabinet 100 while sliding in the horizontal direction. Accordingly, when the module housing 200 is coupled to or separated from the lower side of the inner cabinet 100, there is no need to invade other spaces of the inner cabinet 100 or the machine room 50, and assembly and separation are facilitated. Further, there is no need to have a separate space for coupling or separation of the module housing 200 inside the inner cabinet 100 or inside the machine room 50. Furthermore, an increase in the vertical height of the machine room 50 can be prevented by the module housing 200.

In the shoes care device 1 according to one embodiment of the present invention, the module chamber 210 of the module housing 200 includes a first module chamber 212 accommodating the blowing part 310, a second module chamber 213 accommodating the heating part 320, and a third module chamber 214 accommodating the dehumidifying part 330. The first module chamber 212, the second module chamber 213, and the third module chamber 214 are formed at different positions in a plan view. Therefore, the shoes care device 1 can be formed which has a structure capable of minimizing the vertical height of the module housing 200 and securing the space in the inner cabinet 100 and/or the machine room 50.

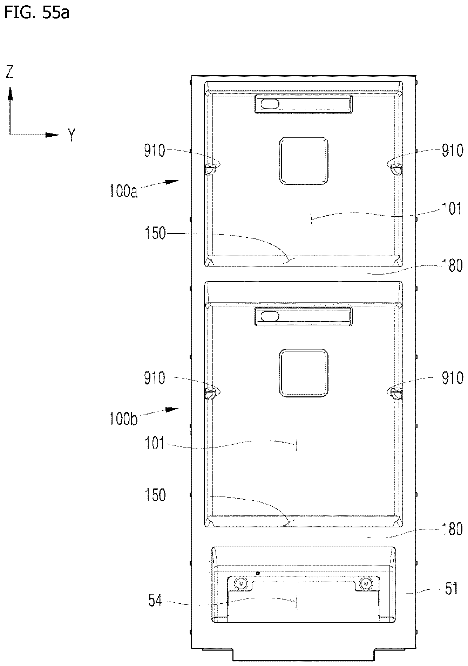

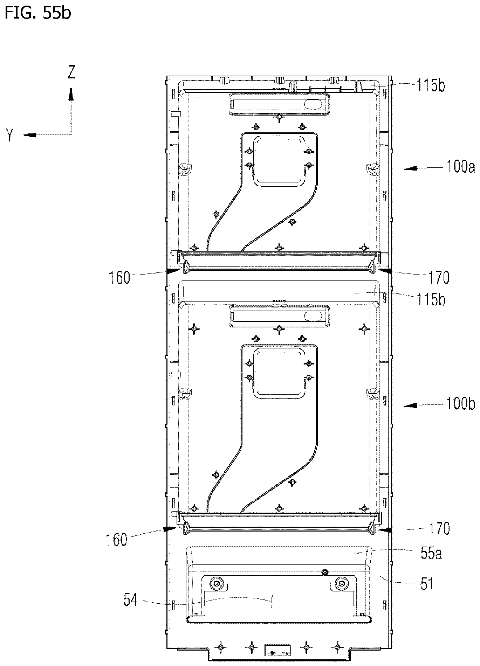

FIG. 55a is a front view illustrating the inner cabinets 100a and 100b illustrated in FIG. 53, and FIG. 55a is a rear view illustrating the inner cabinets 100a and 100b illustrated in FIG. 53.

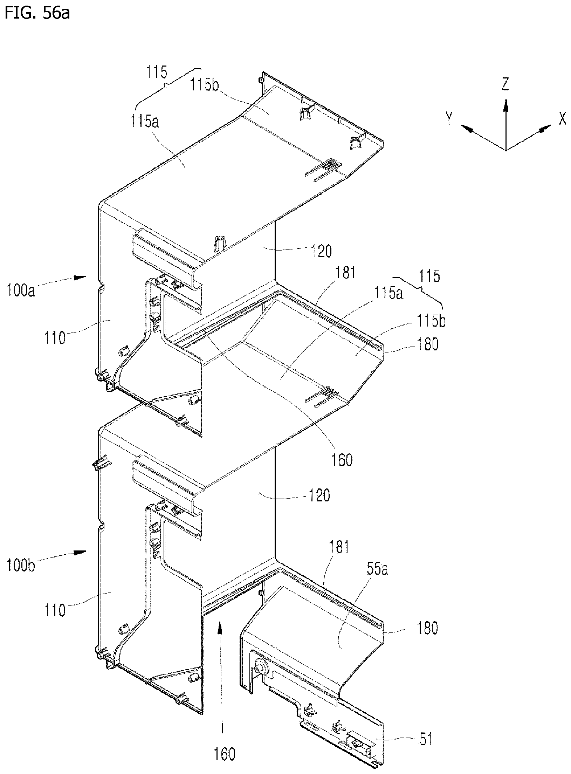

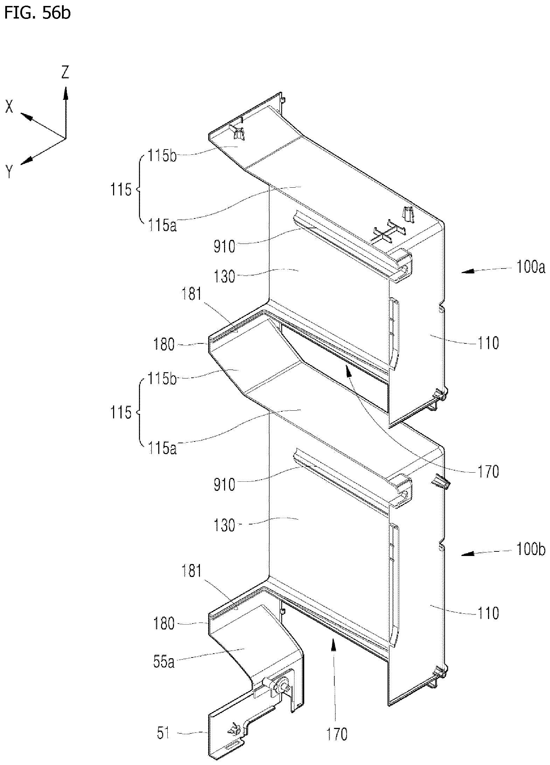

FIGS. 56a and 56b are cross-sectional perspective views illustrating the inner cabinets 100a and 100b illustrated in FIG. 53.

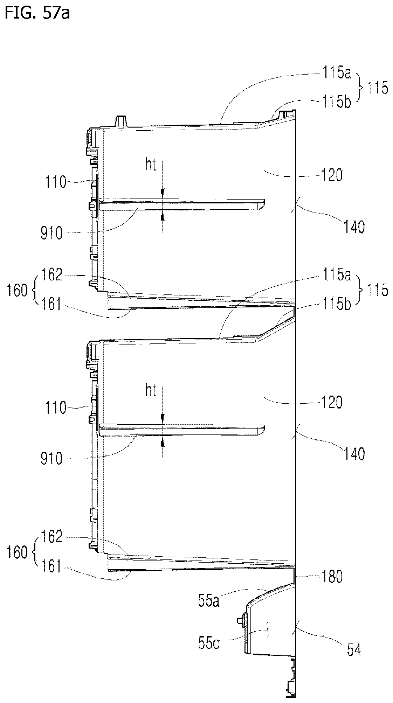

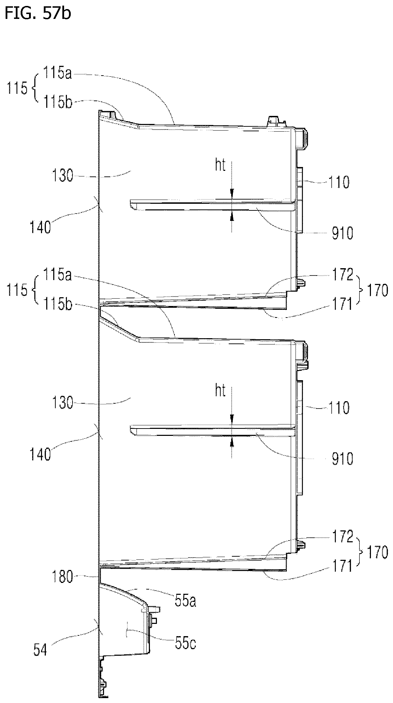

FIGS. 57a and 57b are side cross-sectional views illustrating the inner cabinets 100a and 100b illustrated in FIG. 53.

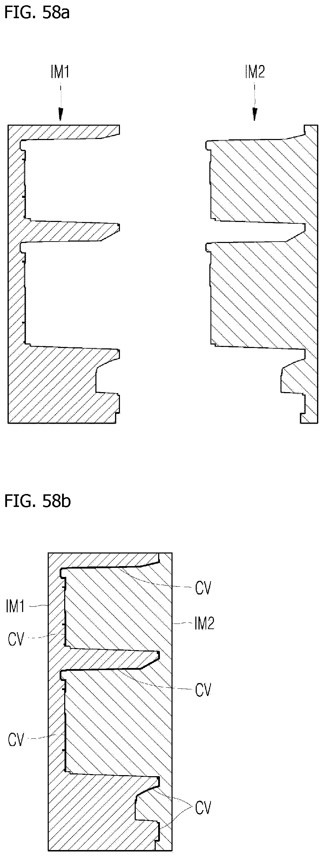

FIG. 58a is a cross-sectional view schematically illustrating a molds IM1 and IM2 used in manufacturing the inner cabinets 100a and 100b according to one embodiment of the present invention, and FIG. 58b is a schematic view illustrating a state in which the molds IM1 and IM2 of FIG. 58a is closed.

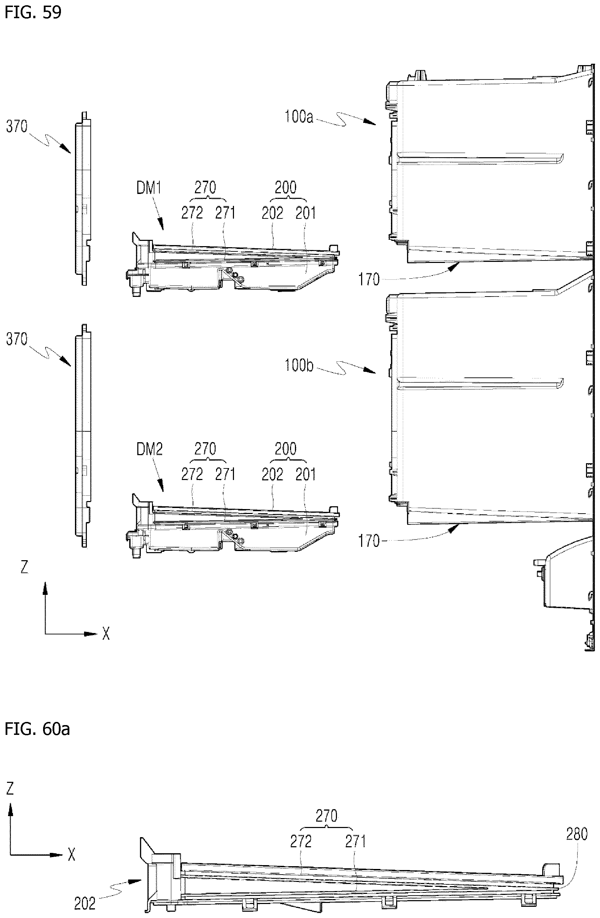

FIG. 59 is a side view illustrating a state in which the inner cabinets 100a and 100b, the module housing 200, and a drying air duct 370 are separated from each other in the shoes care device 1 according to one embodiment of the present invention.

FIG. 60a is a side view illustrating the module cover 202 according to one embodiment of the present invention.

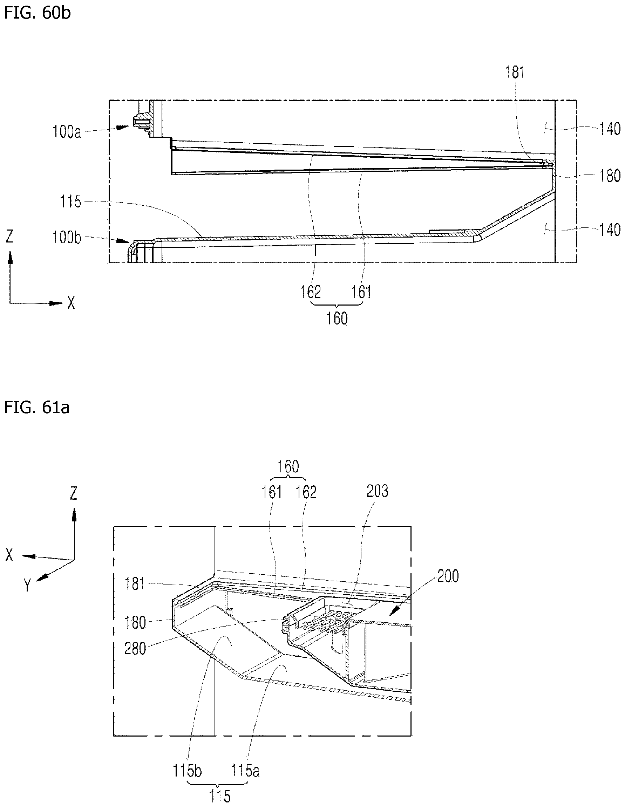

FIG. 60b is a cross-sectional view illustrating the inner cabinets 100a and 100b according to one embodiment of the present invention.

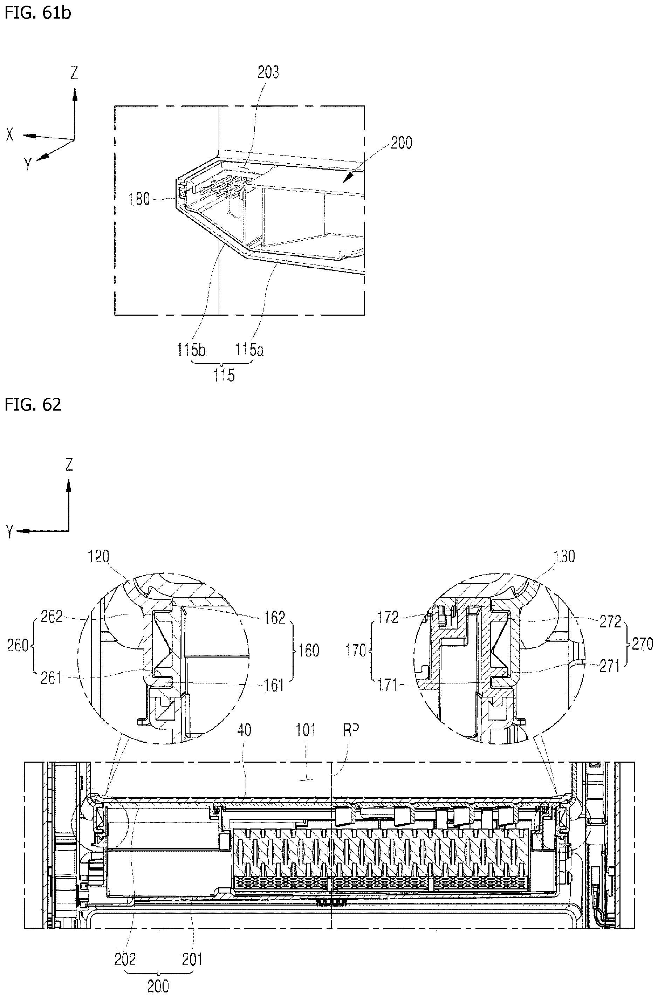

FIG. 61a is a cross-sectional perspective view of a part of the shores care device 1 before the coupling of the module housing 200 and the inner cabinet 100 is completed, and FIG. 61b is a cross-sectional perspective view of a part of the shores care device 1 where the coupling of the module housing 200 and the inner cabinet 100 is completed.

FIG. 62 is a cross-sectional view illustrating a state in which the inner cabinet 100 and the module housing 200 are coupled to each other in the shores care device 1 according to one embodiment of the present invention.

The sliding guides 160 and 170 are formed in the first direction (X direction) as the horizontal direction. The sliding guides 160 and 170 may be formed integrally with the inner cabinet 100. In the shoes care device 1 according to one embodiment of the present invention, the inner cabinet 100 may be formed by injection molding, and the inner cabinet 100 including the sliding guides 160 and 170 may be integrally formed by injection molding.

For injection molding of an injection-molded article with an undercut, when separating the injection-molded article from the mold, the injection-molded article may be elastically deformed and separated, a plurality of divided molds moving in different directions may be included, or a sliding core may be provided in the mold.

When the inner cabinet 100 is integrally molded by injection molding, when the undercut (an outer undercut or an inner undercut) is formed in the structure of the inner cabinet 100, manufacturing costs may increase or injection molding may be difficult.

In the shoes care device 1 according to one embodiment of the present invention, the inner cabinet 100 has a structure having no undercut or a structure with a minimized undercut structure. Accordingly, the inner cabinet 100 can be easily manufactured by injection molding, and further, the inner cabinet 100a of the first management device 2a and the inner cabinet 100b of the second management device 2b can be integrally formed.

When the inner cabinets 100a and 100b according to one embodiment of the present invention are manufactured by injection molding, a first mold IM1 having a shape corresponding to outer surfaces of the inner cabinets 100a and 100b and a second mold IM2 having a shape corresponding to inner surfaces of the inner cabinets 100a and 100b may be used.

The first mold IM1 and the second mold IM2 are closed, and then a molding material is supplied to a cavity CV between the first mold IM1 and the second mold IM2 and is injection-molded. Then, the injection-molded article (the inner cabinet 100) can be separated from the first mold IM1 and the second mold IM2 while opening the first mold IM1 and the second mold IM2.

Since the inner cabinets 100a and 100b have a main opening 140 opened in the first direction (X direction), the first mold IM1 and the second mold IM2 may be moved relative to each other in a direction parallel to the first direction (X direction).

Since the sliding guides 160 and 170 are formed in the inner cabinet 100 in the first direction (X direction), which is a formation direction of the main opening 140, the undercut can be excluded when the sliding guides 160 and 170 are formed. Accordingly, the sliding guide 100 may be easily formed in an injection-molded manner, and the first inner cabinet 100a of the first management device 2a and the second inner cabinet 100b of the second management device 2b may be integrally formed (see FIG. 53).

The sliders 260 and 270 may be fixed to the module case 201 or the module cover 202. The sliders 260 and 270 may be formed integrally with the module case 201 or the module cover 202.

As shown in the drawing according to one embodiment of the present invention, when the sliders 260 and 270 are formed integrally with the module cover 202, since the module cover 202 is located above the module case 201, the inner cabinet 100 and the module housing 200 may be more easily and stably coupled. In the shoes care device 1 according to one embodiment of the present invention, the module cover 202 may be formed by injection molding, and the module cover 202 including the sliders 260 and 270 may be integrally formed by injection molding.

The sliders 260 and 270 may be formed on both left and right edges of the module cover 202 with respect to the first direction (X direction), respectively. That is, a pair of sliders 260 and 270 may be provided in the module cover 202. The slider 260 formed on the left side of the module cover 202 and the slider 270 formed on the right side of the module cover 202 can be symmetrical to each other with respect to the reference plane RP.

The sliding guides 160 and 170 may be formed on left and right sides in the first direction (X direction) below the inner cabinet 100. That is, a pair of sliding guides 160 and 170 may be provided in the inner cabinet 100. In this case, the sliding guide 160 formed on the left side of the inner cabinet 100 and the sliding guide 170 formed on the right side of the inner cabinet 100 may be symmetrical to each other with respect to the reference plane RP.

The sliding guides 160 and 170 may be formed to extend from the rear to the front side of the inner cabinet 100. The sliding guides 160 and 170 may include lower rails 161 and 171 and upper rails 162 and 172.

The lower rails 161 and 171 are formed in a form parallel to or upwardly inclined along the first direction (X direction). The lower rails 161 and 171 may have a straight line shape.

The upper rails 162 and 172 are located above the lower rails 161 and 171 and are inclined downwards in the first direction (X direction). The upper rails 162 and 172 may have a straight line shape.

As the lower rails 161 and 171 and the upper rails 162 and 172 are formed in this way, the undercut can be excluded when the lower rails 161 and 171 and the upper rails 162 and 172 are molded, and the sliding guides 160 and 170 may easily injection-molding the integrally formed inner cabinet 100.

The sliding guides 160 and 170 in which the lower rails 161 and 171 and the upper rails 162 and 172 are combined, may have a tapered shape in which the vertical width thereof gets narrow toward the front side in the first direction (X direction).

This structure of the sliding guides 160 and 170 makes it easier to open the mold IM1 and IM2 and separate the injection-molded article (the inner cabinet 100) from the mold IM1 and IM2 when the inner cabinet 100 is molded by injection molding.

When the sliding guides 160 and 170 are formed on the left and right sides under the inner cabinet 100 in the first direction (X direction), respectively, as described above, the sliders 260 and 270 are also formed on the left and right sides of the module housing 200 in the first direction (X direction), respectively.

The sliders 260 and 270 may be formed integrally with the module cover 202, and in this case, the sliders 260 and 270 may be formed to extend from the rear to the front of the module cover 202. The sliders 260 and 270 is configured to include lower sliders 261 and 271 and upper sliders 262 and 272.

The sliders 260 and 270 in which the lower sliders 261 and 271 and the upper sliders 262 and 272 are combined may have a tapered shape in which the vertical width thereof gets narrow toward the front side in the first direction (X direction).

The lower sliders 261 and 271 may be formed parallel to the lower rails 161 and 171. The lower sliders 261 and 271 may have a straight line shape. The lower sliders 261 and 271 may be in contact with the upper sides of the lower rails 161 and 171 and may slide in the first direction (X direction) or a direction opposite to the first direction (X direction).

The upper sliders 262 and 272 may be formed parallel to the upper rails 162 and 172. The upper sliders 262 and 272 may have a straight line shape. When the module housing 200 including the module cover 202 moves to the frontmost of the first direction (X direction) and when the coupling of the inner cabinet 100 and the module housing 200 is completed, the upper sliders 262 and 272 are in contact with the lower side of the upper rails 162 and 172. That is, before the coupling is completed by locating the module housing 200 behind the inner cabinet 100, the upper sliders 262 and 272 are spaced apart from the upper rails 162 and 172.

The sliders 260 and 270 are slidably coupled along the sliding guides 160 and 170, and when the module housing 200 is completely coupled to the inner cabinet 100, the module cover 202 completely shields the lower opening 150 of the inner cabinet 100.

As described above, in one inner cabinet 100, a pair of sliding guides 160 and 170 may be provided and formed on both left and right sides of the inner cabinet 100. In this case, the pair of sliding guides 160 and 170 may be divided into a first sliding guide 160 and a second sliding guide 170.

The first sliding guide 160 may be formed in a lower end of the first inner side plate 120 in the first direction (X direction). The second sliding guide 170 may be formed in a lower end of the second inner side plate 130 in the first direction (X direction).

In addition, the lower opening 150 of the inner cabinet 100 has a hole shape opened between the first sliding guide 160 and the second sliding guide 170.

In addition, as described above, a pair of sliders 260 and 270 may be provided and formed on both left and right sides of the module housing 200 (the module cover 202). In this case, the pair of sliders 260 and 270 may be divided into a first slider 260 and a second slider 270.

The first sliding guide 160 and the second sliding guide 170 are symmetrical with respect to the reference plane RP, and the first slider 260 and the second slider 270 are symmetrical with respect to the reference plane RP.

In the shoes care device 1 according to one embodiment of the present invention, when the inner cabinets 100a and 100b and the module housing 200 are coupled, if the module housing 200 moves to the frontmost part in the first direction (X direction), the upper rails 162 and 172 and the upper sliders 262 and 272 come into contact with each other, and the upper rails 162 and 172 serve as stoppers. Therefore, with the movement of the module housing 200 in the first direction (X direction), stable assembly between the module housing 200 and the inner cabinet 100 is achieved, and vertical and horizontal gaps between the module housing 200 and the inner cabinet 100 are effectively prevented.

Each of the sliding guides 160 and 170 and the sliders 260 and 270 is provided in pairs and is symmetrically formed on both left and right sides of the inner cabinet 100 with respect to the first direction (X direction), thereby stably and easily coupling and separation of the module housing 200 and the inner cabinet 100.

The inner cabinet 100 includes a front frame 180 and a fixing guide 181. The front frame 180 and the fixing guide 181 may be formed integrally with the inner cabinet 100.

The front frame 180 is formed in the inner cabinet 100 ahead in the first direction (X direction). The front frame 180 may be bent outwards from the front end of the inner cabinet 100 with respect to the first direction (X direction). The front frame 180 may form a surface orthogonal to the first direction (X direction). The front surface of the front frame 180 may be orthogonal to the first direction (X direction).

The front frame 180 is configured to shield the front end of the module housing 200 with respect to the first direction (X direction).

The front frame 180 connects a front end of the first inner side plate 120 and a front end of the second inner side plate 130 to each other. The front frame 180 connects a lower side of the front end of the first inner side plate 120 and a lower side of the front end of the second inner side plate 130 to each other. The front frame 180 may be formed along the entire frame of the inner cabinet 100 ahead in the first direction (X direction), and the area surrounded by the front frame 180 corresponds to the main opening 140.

The inner cabinet 100a of the first management device 2a and the inner cabinet 100b of the second management device 2b are connected to each of front frames 180. When the first inner cabinet 100a and the second inner cabinet 100b are integrally formed by injection molding, the front frame 180 of the first inner cabinet 100a and the front frame 180 of the second inner cabinet 100b are formed integrally with each other.

The front frame 180 of the second inner cabinet 100b may be formed integrally with the first wall 51.

The fixing guide 181 is formed on a rear surface of the front frame 180 with respect to the first direction (X direction). The fixing guide 181 may protrude to the opposite side of the first direction (X direction). The fixing guide 181 may be formed in a shape extending in the second direction (Y direction). The fixing guide 181 may have a constant cross-section in the second direction (Y direction).

The module cover 202 includes a fixing rib 280.

The fixing rib 280 may protrude from the front end of the module cover 202 in the first direction (X direction) to the first direction (X direction). Further, the fixing rib 280 may be formed to extend in the second direction (Y direction). The fixing rib 280 may have a constant cross-section in the second direction (Y direction).

The fixing guide 181 and the fixing rib 280 may be engaged with each other. When one of the fixing guide 181 and the fixing rib 280 is formed in a protrusion shape, the other may be formed in a groove shape into which the protrusion is inserted. When the fixing rib 280 is formed in a protrusion shape protruding in the first direction (X direction), the fixing guide 181 may be formed in a groove shape that is concave in the first direction (X direction), and the fixing rib 280 may be inserted into the fixing guide 181 and be coupled to each other.

In one embodiment of the present invention, when the sliders 260 and 270 of the module housing 200 move in the first direction (X direction) along the sliding guides 160 and 170 of the inner cabinet 100, and the coupling of the module housing 200 and the inner cabinet 100 is completed, the coupling the fixing rib 280 and the fixing guide 181 may are made to be completed.

In the shoes care device 1 according to one embodiment of the present invention, when the door 30 is opened, the inner cabinet 100 is exposed from the frontmost part of the front frame 180, and there is no need to form any division line (e.g., a division line where the inner cabinets 100 are coupled to each other) in the front frame 180. Accordingly, the shoes care device 1 can be form which is a structurally stable and has excellent aesthetics.

The inner cabinet 100 includes an inner upper plate 115 forming a ceiling.

The inner cabinet 100b of the second management device 2b may include the inner upper plate 115 which forms the ceiling and is connected to the front frame 180, where a front part thereof in the first direction (X direction) forms an upwardly inclined surface.

The inner upper plate 115 of the inner cabinet 100 may include a first region 115a and a second region 115b. In each of the inner cabinet 100a of the first management device 2a and the inner cabinet 100b of the second management device 2b, the inner upper plate 115 may include the first region 115a and the second region 115b.

In the first direction (X direction), the first region 115a forms a relatively rear part and a middle part, and the second region 115b forms a relatively front part.

The first region 115a forms a flat surface. The first region 115a may form a substantial part of the inner upper plate 115.

In one embodiment, the first region 115a may be parallel to the horizontal direction.

In another embodiment, the first region 115a may be inclined upwards in the first direction (X direction). The first region 115a may be formed smaller than an inclination angle formed by the second region 115b and inclined upwards in the first direction (X direction).

The second region 115b extends ahead from the front of the first region 115a with respect to the first direction (X direction). The second region 115b forms a surface upwardly inclined in the first direction (X direction) and forms a region connected to the front frame 180.

The second region 115b of the second management device 2b may have a greater inclination angle formed by a horizontal surface than the second region 115b of the first management device 2a.

Accordingly, even if the width of the front frame 180 is not increased when viewed from the front, a space in which the module housing 200 is coupled to the lower side of the inner cabinet 100 can be secured in the first management device 2a.

In addition, the structure of the inner upper plate 115 including the first region 115a and the second region 115b does not act as an undercut when the inner cabinet 100 is molded by injection molding, and is easily used to open the molds IM1 and IM2 and separate the injection-molded article (the inner cabinet 100) from the molds IM1 and IM2).

The front frame 180 of the first inner cabinet 100a may be connected to the inner upper plate 115 of the second inner cabinet 100b. The front frame 180 of the first inner cabinet 100a and the inner upper plate 115 of the second inner cabinet 100b may be connected to each other in the entire section in the second direction (Y direction).

The first inner side plate 120 may extend downwards from one end of the inner upper plate 115, and the second inner side plate 130 may extend downwards from the other end of the inner upper plate 115.

In one embodiment, the first inner side plate 120 and the second inner side plate 130 may be parallel to each other in the first direction (X direction).

In another embodiment, the first inner side plate 120 and the second inner side plate 130 may be inclined in a direction moving away from each other along the first direction (X). In one embodiment, an angle formed between the first inner side plate 120 and a vertical surface and an angle formed between the second inner side plate 130 and the vertical surface may be 5 degrees or less, respectively.

Each of the first inner side plate 120 and the second inner side plate 130 includes a shelf holder 910 protruding inwards from the inner side and formed in the first direction (X direction).

The shelf holder 910 may be formed such that the vertical width ht becomes narrower toward the first direction (X direction).

The shelf holder 910 formed on the first inner side plate 120 and the shelf holder 910 formed on the second inner side plate 130 are formed integrally with the inner cabinet 100.

Since the shelf holder (910) is formed parallel to the first direction (X direction) and the vertical widths ht thereof becomes narrower toward the first direction (X direction), the shelf holder 910 does not act as an undercut when the inner cabinet 100 is injection-molded, this makes it easy to open the molds IM1 and IM2 and separate the inner cabinet 100 from the molds IM1 and IM2.

As described above, the first management device 2a and the second management device 2b may be vertically disposed, and in this case, the first inner cabinet 100a and the second inner cabinet 100b may be formed integrally with each other. Accordingly, no separate process to couple the first inner cabinet 100a and the second inner cabinet 100b is required, the sense of unity between the first inner cabinet 100a and the second inner cabinet 100b can be improved, and the overall aesthetics and productivity of the shoes care device 1 can be improved.

In the shoes care device 1 according to one embodiment of the present invention, the inner cabinet 100 of the first management device 2a is disposed above the inner cabinet 100 of the second management device 2b. The hoes care device 1 may be formed where the inner cabinet 100 and the module housing 200 of the first management device 2a, and the inner cabinet 100 and the module housing 200 of the second management device 2b are arranged in order from top to bottom, and the shoes care device 1 may be formed which can utilize a space in the vertical direction. In this case, the module housing 200 is coupled to a lower side of the inner cabinet 100 while sliding in the first direction (X direction). In other words, the module housing 200 of the first management device 2a is slidably coupled between the first inner cabinet 100a and the second inner cabinet 100b, and the module housing 200 of the second management device 2b is slidably coupled to the lower end of the second inner cabinet 100b.

The structure of coupling the inner cabinet 100 and the module housing 200 allows a plurality of inner cabinets 100 to be integrally formed by injection molding, and allows a stable coupling between the inner cabinet 100 and the module housing 200 without wasting a space.

As the module housing 200 slides in the first direction (X direction) and is coupled to the inner cabinet 100, the inner cabinet 100 and the module housing 200 can be easily and stably coupled to each other. In addition, when the inner cabinet 100 or its internal components need to be repaired or replaced, the module housing 200 can be easily separated from the inner cabinet 100 by moving the module housing 200 behind the first direction (X direction).

In the shoes care device 1 according to one embodiment of the present invention, the module cover 202 constituting the module housing 200 is provided an inlet 203 formed through which air of the accommodation space 101 is sucked, and the inner cabinet 100 includes a lower opening 150 formed by opening an upper part thereof. When the module housing 200 is coupled to the inner cabinet 100, the module cover 202 is formed to close the lower opening 150. That is, the module cover 202 of the module housing 200 forms a bottom surface of the inner cabinet 100.

When a complex structure is required on the bottom of the inner cabinet 100, unlike the embodiment of the present invention, assuming that the entire inner cabinet 100 including the bottom of the inner cabinet 100 is formed by injection molding, manufacturing costs may increase or manufacturing thereof may be difficult. This is because the structure of the bottom of the inner cabinet 100 and the outlet 203 act as undercuts. When the first inner cabinet 100a and the second inner cabinet 100b are arranged vertically and integrally with each other, such difficulties may be further increased.

Unlike the embodiment of the present invention, assuming that the module cover 202 is formed integrally with the inner cabinet 100 and the inner cabinet 100 is formed by injection molding, the molds (the first mold IM1 and the second mold IM2) for manufacturing the inner cabinet 100 needs to mold the inner cabinet 100 while being closed and opened with a relatively movement in a direction in parallel with the first direction (X direction), but there is any difficulty forming what structure is formed on the bottom of the module cover 202, and also forming a part of the outlet 203 vertically penetrating the module cover 202. In particular, it is more difficult to form the inner cabinet 100 of the first management device 2a and the inner cabinet 100 of the second management device 2b integrally by injection molding.

As described above, in the shoes care device 1 according to one embodiment of the present invention, as the module cover 202 of the module housing 200 formed separately from the inner cabinet 100 forms the bottom surface of the inner cabinet 100, the module cover 202 may be easily formed even when a relatively complex structure for forming a connection path is required on the lower surface of the module cover 202, the inner cabinet 100 may also be easily formed by injection molding, and then, by coupling the module housing 200 to the inner cabinet 100, the bottom surface of the inner cabinet 100 may be easily formed.

Even when the first inner cabinet 100a and the second inner cabinet 100b are vertically arranged and integrally formed, the inner cabinet 100a and 100b may be first manufactured by injection molding and then the inner cabinet 100a and 100b may be coupled to the module housing 200, thereby easily forming the bottom of the inner cabinet 100a and 100b.

In addition, the inner upper plate 115, the first inner side plate 120, and the second inner side plate 130 are formed in the first direction (X direction), which is the formation direction of the main opening 140, the sliding guides 160 and 170 formed integrally with the inner cabinets 100a and 100b and the shelf holder 910 are formed in the first direction (X direction), and the inner upper plate 115, the first inner side plate 120, the second inner side plate 130, the sliding guides 160 and 170, and the shelf holder 910 may all be formed without undercut based on the first mold IM1 and the second mold IM2 that move relative to each other in the first direction (X direction). Accordingly, the inner cabinet 100 may be integrally manufactured by injection molding.

In the shoes care device 1 according to one embodiment of the present invention, the inner cabinet 100 of the first management device 2a and the inner cabinet 100 of the second management device 2b may be integrally formed by injection molding to form the shoes care device 1 with a structure advantageous for rigidity. In addition, it is possible to reduce the components necessary for manufacturing the shoes care device 1 and reduce material costs and manufacturing costs.

In addition, the front part of the module housing 200 is covered by the front frame 180 and the front frame 180 integrated with the inner cabinet 100 is exposed from the front when the door 30 is opened. Accordingly, the module housing 200 and the inner cabinet 100 can be integrally coupled to each other, and the shoes care device 1 with an excellent esthetic sense on the whole can be formed.

The shoes care device 1 according to one embodiment of the present invention includes a dry air duct 370 that connects the module housing 200 and the inner cabinet 100 in communication with each other. In the module cover 202, the outlet 203 is formed in the front side in the first direction (X direction), and the dry air duct 370 connects the module housing 200 and the inner cabinet 100 in communication with each other in the rear side in the first direction (X direction). Accordingly, it is possible to easily form a shoes care device 1 with a structure in which the module housing 200 can be first coupled to the inner cabinet 100 and the dry air duct 370 can be coupled to the inner cabinet 100 and the inside of the module housing 200, and air can be circulated inside the inner cabinet 100 and inside the module housing 200.

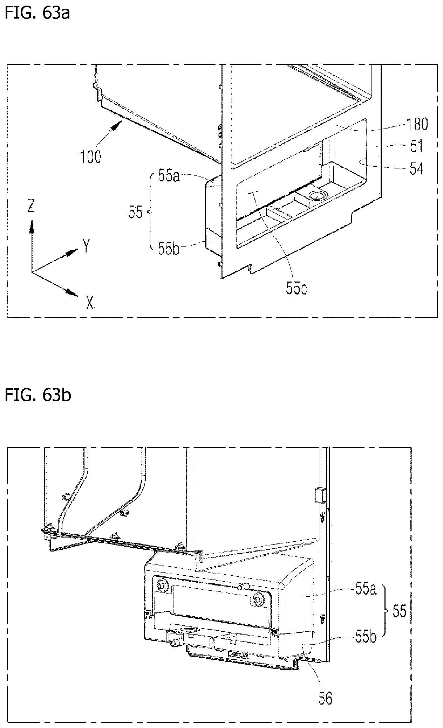

FIG. 63a is a perspective view illustrating the lower side of the inner cabinet 100 and the first wall 51, and FIG. 63b is a rear perspective view illustrating the lower side of the inner cabinet 100 and the first wall 51.

A water tank opening 54, which is a hole penetrated in the first direction (X direction), is formed in the first wall 51.

Each of the water supply tank 60 and the drain tank 70 may be disposed in the water tank opening 54. When the water supply tank 60 and the drain tank 70 are disposed in the water tank opening 54, the water tank opening 54 may be shielded by the water supply tank 60 and the drain tank 70.

The front frame 180 and the first wall 51 may form the same plane and be formed integrally with each other.

The shoes care device 1 may include an upper accommodation housing 55a.

The upper accommodation housing 55a is integrally connected to the first wall 51 in an edge of the water tank opening 54. The upper accommodation housing 55a extends behind the first wall 51 with respect to the first direction (X direction), and forms a water tank accommodation space 55c for accommodating the water supply tank 60 and the drain tank 70.

The upper part of the upper accommodation housing 55a is inclined downwardly toward the direction opposite to the first direction (X direction). The upper accommodation housing 55a is formed to cover the upper sides of the water supply tank 60 and the drain tank 70.

The shoes care device 1 includes a lower accommodation housing 55b.

The lower accommodation housing 55b is formed to cover the lower sides of the water supply tank 60 and the drain tank 70. The lower accommodation housing 55b and the upper accommodation housing 55a are coupled to form the accommodation housing 55. The lower accommodation housing 55b is coupled to a lower side of the upper accommodation housing 55a to form the water tank accommodation space 55c along with the upper accommodation housing 55a.

By forming the first wall 51 and the upper accommodation housing 55a integrally with the front frame 180 of the inner cabinet 100, a structurally stable and aesthetic shoes care device 1 can be formed, and the water supply tank 60 and the drain tank 70 can be stably coupled with the upper accommodation housing 55a.

Since the upper part of the upper accommodation housing 55a is inclined downwards in the direction opposite to the first direction (X direction), even if the width of the front frame 180 of the inner cabinet 100 of the second management device 2b is not increased, a space in which the module housing 200 is coupled to the lower side of the inner cabinet 100 of the second management device 2b may be secured. In addition, the inner cabinet 100 may be easily manufactured by injection molding.

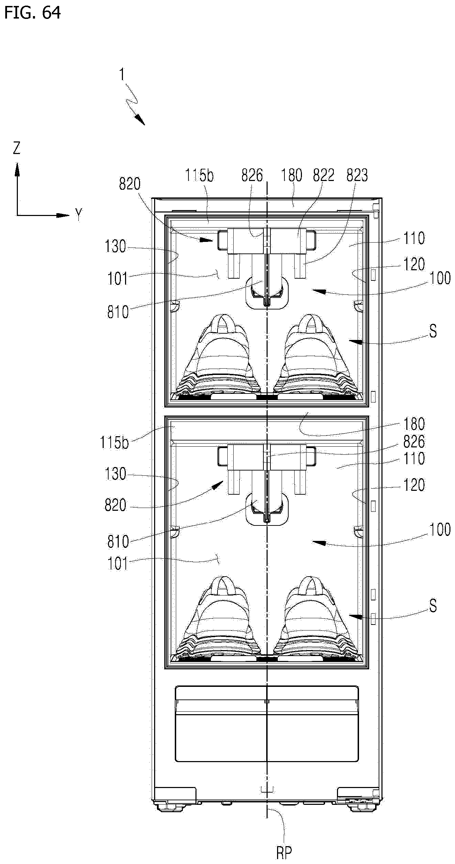

FIG. 64 is a front view illustrating a state in which the door 30 is removed from the shores care device 1 illustrated in FIG. 1b. FIG. 64 illustrates the shoes accommodated in the inner cabinet 100 together.

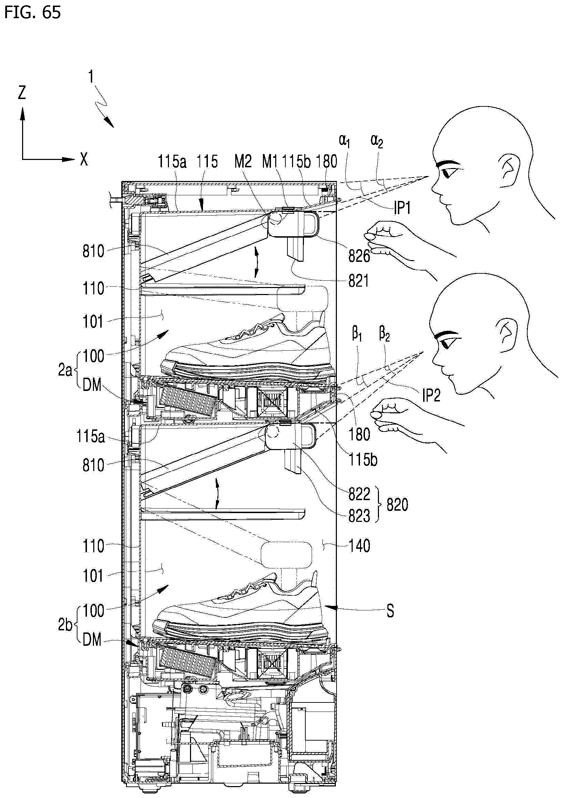

FIG. 65 is a cross-sectional view illustrating a state in which the door 30 is removed from the shores care device 1 illustrated in FIG. 4c. FIG. 65 illustrates the shoes accommodated in the inner cabinet 100 together.

The shoes care device 1 according to one embodiment of the present invention may include the inner cabinet 100, the door 30, the nozzle duct 810, the nozzle 820, the nozzle handle 826, and a nozzle connector 830.

The inner cabinet 100 may have an accommodation space 101 formed inside. The shoes S may be accommodated in the accommodation space 101. In addition, the inner cabinet 100 may have a main opening 140 that opens the accommodation space 101 in the first direction (X direction) as the horizontal direction.

In one embodiment, the first direction (X direction) may be a direction from the rear of the shoes care device 1 to the front thereof. The first direction (X direction) may be parallel to a front-rear direction. In addition, it is described that the second direction (Y direction) is parallel to a left-right direction, and the third direction (Z direction) is parallel to the vertical direction.

The inner cabinet 100 may include the inner rear plate 110, the first inner side plate 120, the second inner side plate 130, and the inner upper plate 115. The inner rear plate 110, the first inner side plate 120, the second inner side plate 130, and the inner upper plate 115 may be formed integrally with each other.

The inner rear plate 110 forms a wall surface (border surface) at the rear of the accommodation space 101. That is, the inner rear plate 110 forms a wall surface (border surface) vertically erected at the rear of the accommodation space 101. The inner rear plate 110 may form a surface orthogonal to the first direction (X direction).

The inner upper plate 115 may form a ceiling of the accommodation space 101. The inner upper plate 115 may include the first region 115a and the second region 115b. The first region 115a and the second region 115b may be formed integrally with each other.

The inner cabinet 100 may include the front frame 180. The front frame 180 may extend ahead of the inner upper plate 115 with respect to the first direction (X direction). The front frame 180 may form an edge of the main opening 140. The front surface of the front frame 180 may be orthogonal to the first direction (X direction).

The shoes care device 1 according to one embodiment of the present invention includes management devices 2a and 2b. The management devices 2a and 2b may include the inner cabinet 100 and the drying module DM. In one embodiment, the shoes care device 1 may include the first management device 2a and the second management device 2b.

That is, the first management device 2a may include the inner cabinet 100 and a drying module DM. Further, the second management device 2b may include the inner cabinet 100 and the drying module DM.

The first management device 2a and the second management device 2b may be provided adjacent to each other. The first management device 2a may be disposed above the second management device 2b.

Accordingly, the inner cabinet 100 of the first management device 2a may be disposed above the inner cabinet 100 of the second management device 2b. Each of the front frames 180 may be connected to the inner cabinet 100 of the first management device 2a and the inner cabinet 100 of the second management device 2b.

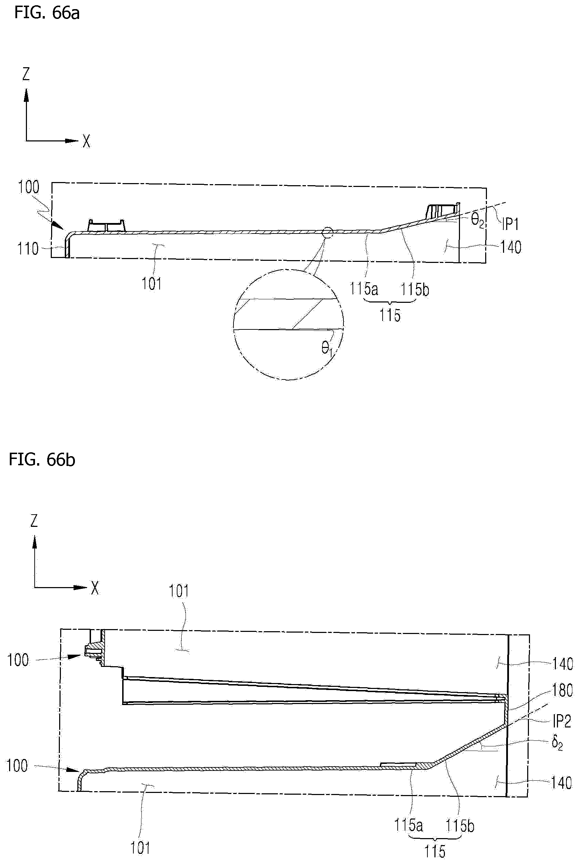

FIG. 66a is a cross-sectional view illustrating the inner cabinet 100 according to one embodiment of the present invention and is a cross-sectional view illustrating the inner upper plate 115 of the inner cabinet 100 of the first management device 2a.

FIG. 66b is a cross-sectional view illustrating the inner cabinet according to one embodiment of the present invention and is a cross-sectional view illustrating the inner upper plate 115 of the inner cabinet 100 of the second management device 2b.

The first region 115a refers to a region extending in an upwardly inclined direction from the upper side of the inner rear plate 110. The height of the first region 115a may get larger toward the first direction (X direction). An increase of the height of the first region 115a per unit distance in the first direction (X direction) may remain constant.

In one embodiment, based on the first direction (X direction), the first region 115a of the first management device 2a may form a spacing angle θ1 with respect to the horizontal plane.

The inner upper plate 115 of the first management device 2a may have a constant thickness in the first region 115a based on the first direction (X direction). Accordingly, based on the first management device 2a, the bottom surface of the first region 115a may form a spacing angle θ1 with respect to the horizontal plane.

In one embodiment, based on the first direction (X direction), the first region 115a of the second management device 2b may form a spacing angle δ1 with respect to the horizontal plane.

The inner upper plate 115 of the second management device 2b may have a constant thickness in the first region 115a based on the first direction (X direction). Accordingly, based on the second management device 2b, the bottom surface of the first region 115a may form a spacing angle δ1 with respect to the horizontal surface.

In one embodiment, θ1 and δ1 may be the same angle. Based on the first direction (X direction), the first region 115a of the first management device 2a and the first region 115a of the second management device 2b may form the same length.

The first region 115a of the first management device 2a may be parallel to the second direction (Y direction). Accordingly, as shown in FIG. 64, when the main opening 140 is viewed from the front of the shoes care device 1 with respect to the first management device 2a, the bottom surface of the first area 115a may not form a height in the second direction (Y direction).

The first region 115a of the second management device 2b may be parallel to the second direction (Y direction). Accordingly, as shown in FIG. 64, when the main opening 140 is viewed from the front of the shoes care device 1 with respect to the second management device 2b, the bottom surface of the first area 115a may not form a height in the second direction (Y direction).

The second region 115b refers to a region extending in front of the first region 115a based on the first direction (X direction). The second region 115b forms an upwardly inclined surface. The height of the second region 115b may get larger toward the first direction (X direction). An increase of the height of the second region 115b per unit distance in the first direction (X direction) may remain constant.

In one embodiment, based on the first direction (X direction), the second region 115b of the first management device 2a may form a spacing angle θ2 with respect to horizontal plane.

The inner upper plate 115 of the first management device 2a may have a constant thickness in the second region 115b based on the first direction (X direction). Accordingly, based on the first management device 2a, the bottom surface of the second region 115b may form a spacing angle θ2 with respect to the horizontal surface.

The first region 115a may be inclined upwards in the first direction (X direction) to become smaller than the inclination angle formed by the second region 115b. That is, based on the first direction (X direction), the inclination of the second region 115b of the first management device 2a may be greater than the inclination of the first region 115a of the first management device 2a. Accordingly, θ2 may be an angle greater than θ1.

As illustrated in FIG. 65, when the user's gaze coincides with a virtual surface (hereinafter, referred to as the 'first virtual surface IP1') extending an inclined surface of the second region 115b of the first management device 2a, the user's viewing angle that views the front frame 180 connected to the inner upper plate 115 of the first management device 2a may be α1.

Meanwhile, when the inner upper plate 115 of the first management device 2a does not form the second area 115b but consists of only the first region 115a, the user's viewing angle that views the front frame 180 connected to the inner upper plate 115 of the first management device 2a may be α2. The dotted line illustrated in FIG. 65 may mean a virtual surface extending the first region 115a.

Based on the upper end of the front frame 180, α2 may be an angle greater than α1. Accordingly, as the inner upper plate 115 of the first management device 2a forms the second region 115b, the user's viewing angle that views the accommodation space 101 of the first management device 2a may be extended by an angle of α2-α1.

In one embodiment, based on the first direction (X direction), the second region 115b of the second management device 2b may form a spacing angle δ2 with respect to the horizontal plane.

The inner upper plate 115 of the second management device 2b may have a constant thickness in the second region 115b based on the first direction (X direction). Accordingly, based on the second management device 2b, the bottom surface of the second region 115b may form the spacing angle δ2 with respect to the horizontal plane.

The first region 115a may be inclined upwards in the first direction (X direction) to become smaller than the inclination angle formed by the second region 115b. That is, based on the first direction (X direction), the inclination of the second region 115b of the second management device 2b may be greater than the inclination of the first region 115a of the second management device 2b. Accordingly, δ2 may be an angle greater than δ1.

As illustrated in FIG. 65, when the user's gaze coincides with a virtual surface (hereinafter, referred to as a 'second virtual surface IP2') extending the inclined surface of the second management device (2b), the user's viewing angle that views the front frame 180 connected to the inner upper plate 115 of the second management device 2b may be β1.

Meanwhile, when the inner upper plate 115 of the second management device 2b does not form the second region 115b but consists of only the first region 115a, the user's viewing angle that views the front frame 180 connected to the inner upper plate 115 of the second management device 2b may be β2. The dotted line illustrated in FIG. 65 may mean a virtual surface extending the first region 115a.

Based on the upper end of the front frame 180, β2 may be an angle greater than β1. Accordingly, as the inner upper plate 115 of the second management device 2b forms the second area 115b, the user's viewing angle that views the accommodation space 101 of the second management device 2b may be extended by an angle of β2-β1.

In one embodiment, based on the first direction (X direction), the second region 115b of the first management device 2a and the second region 115b of the second management device 2b may have the same length.

With respect to the first direction (X direction), the second region 115b of the second management device 2b may have a greater inclination angle than the second region 115b of the first management device 2a. That is, δ2 may be an angle greater than θ2.

The second management device 2b is provided in a position lower than that of the first management device 2a. Accordingly, even if the user bends his knee or waist to look at the accommodation space 101 of the second management device 2b, the height difference between the user's gaze and the accommodation space 101 may be greater than the height difference between the user's gaze and the accommodation space 101 of the first management device 2a when the user's gaze views the accommodation space 101 of the first management device 2a.

Based on the first direction (X direction), as the second region 115b of the second management device 2b has a greater inclination angle with respect to the horizontal plane than the second region 115b of the first management device 2a, even if the height difference between the user's gaze and the accommodation space 101 of the second management device 2b can be sufficiently larger than the height difference between the user's gaze and the accommodation space 101 of the first management device 2a, the user's viewing angle that views the accommodation space 101 can be sufficiently expanded.

A length of the second region 115b of the second management device 2b based on a direction in which the spacing angle δ2 is formed with respect to the first direction (X direction) may be formed longer than a length of the second region 115b of the first management device 2a based on a direction in which the spacing angle θ2 is formed with respect to the first direction (X direction).

The second region 115b of the first management device 2a may be parallel to the second direction (Y direction). Accordingly, as shown in FIG. 64, when the main opening 140 is viewed from the front of the shoes care device 1 with respect to the first management device 2a, the bottom surface of the second area 115b may not form a height in the second direction (Y direction).

The second region 115b of the second management device 2b may be parallel to the second direction (Y direction). Accordingly, as shown in FIG. 64, when the main opening 140 is viewed from the front of shoes care device 1 with respect to the second management device 2b, the bottom surface of the second area 115b may not form a height in the second direction (Y direction).

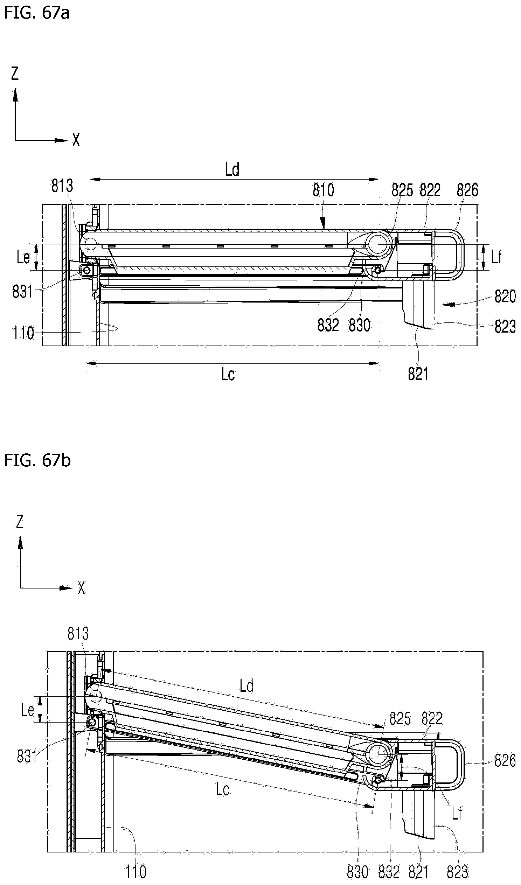

FIG. 67a is a view illustrating a state in which an angle of the nozzle duct 810 illustrated in FIG. 65 is adjusted and is a view illustrating a state in which the length direction of the nozzle duct 810 is parallel to the horizontal direction thereof.

FIG. 67b is a view illustrating a state in which the angle of the nozzle duct 810 illustrated in FIG. 65 is adjusted and is a view illustrating a state in which the angle of the nozzle duct 810 is adjusted to descend the nozzle 820.

The nozzle duct 810 may form a passage for forcibly ventilated air. The nozzle duct 810 may be installed to protrude forward from the inner rear plate 110. That is, the nozzle duct 810 may be installed to protrude from the inner rear plate 110 into the inner cabinet 100.

One end of the nozzle duct 810 may be hinge-coupled to the inner rear plate 110. One end of the nozzle duct 810 may be hinge-coupled to the inner rear plate 110 through the nozzle duct hinge axis 813. One end of the nozzle duct 810 may rotate around the nozzle duct hinge axis 813.

The nozzle 820 may be coupled to the other end of the nozzle duct 810. Accordingly, the nozzle duct 810 may form a passage through which the forcibly ventilated air is moved to the nozzle 820.

The nozzle 820 is configured to inject air into the shoes.

When an angle of the nozzle duct 810 is adjusted, at least a part of the nozzle 820 may be inserted into the inner side of the shoes. The air injected by the nozzle 820 may be injected inside the shoes in the accommodation space 101. The nozzle 820 may be hinge-coupled to the other end of the nozzle duct 810.

The nozzle 820 may be hinge-coupled to the other end of the nozzle duct 810. The nozzle 820 may include the nozzle body 822 and the nozzle protrusion 823.

The nozzle body 822 may be hinge-coupled to the nozzle duct 810. That is, the nozzle body 822 may be coupled to a nozzle hinge axis 825 formed in the other end of the nozzle duct 810. Accordingly, the nozzle 820 may be rotated around the nozzle hinge axis 825.

The nozzle protrusion 823 may protrude downwards from the nozzle body 822. The nozzle protrusion 823 may have the lower discharge port 821 formed in a lower end thereof. The nozzle 820 may inject air downwards through the lower discharge port 821 while a part of the lower part of the nozzle protrusion 823 is inserted into the shoes.

The nozzle handle 826 may be formed on the nozzle body 822. The nozzle handle 826 may protrude in the first direction (X direction) from the front surface of the nozzle body 822 in the first direction (X direction). The user may adjust the angle of the nozzle duct 810 by holding the nozzle handle 826 and applying force.

The nozzle handle 826 may ascend vertically on the reference plane RP. Based on the second direction (Y direction), the nozzle handle 826 may ascend vertically from the center of the accommodation space 101.

One end of the nozzle connector 830 may be hinge-coupled to the inner rear plate 110. A first connection hinge axis 831 may be formed in one end of the nozzle connector 830. The first connection hinge axis 831 may be rotatably coupled to the inner rear plate 110. Accordingly, the nozzle connector 830 may rotate around the first connection hinge axis 831.

The other end of the nozzle connector 830 may be hinge-coupled to the nozzle 820. A second connection hinge axis 832 may be formed in the other end of the nozzle connector 830. The second connection hinge axis 832 may be rotatably coupled to the nozzle body 822. Accordingly, the nozzle connector 830 may rotate around the second connection hinge axis 832.

The nozzle duct hinge axis 813 and the first connection hinge axis 831 may be rotatably coupled to the inner rear plate 110. Accordingly, the nozzle duct hinge axis 813 and the first connection hinge axis 831 may be rotatably fixed to a specific position of the inner rear plate 110.

That is, positions of the nozzle duct hinge axis 813 and the first connection hinge axis 831 may not be changed with respect to the inner cabinet 100.

The nozzle hinge axis 825 and the second connection hinge axis 832 may be rotatably coupled to the nozzle body 822. Accordingly, the nozzle hinge axis 825 and the second connection hinge axis 832 may be rotatably fixed to a specific position of the nozzle body 822.

That is, positions of the nozzle hinge axis 825 and the second connection hinge axis 832 may not be changed based on the nozzle body 822. However, when the nozzle duct 810 rotates based on the inner cabinet 100, the positions of the nozzle hinge axis 825 and the second connection hinge axis 832 may be changed.

In one embodiment, a distance Ld between the nozzle duct hinge axis 813 and the first connection hinge axis 831 and may be the same as a distance Lc between the nozzle hinge axis 825 and the second connection hinge axis 832. In addition, the distance Le between the nozzle duct hinge axis 813 and the nozzle hinge axis 825 may be the same as the distance Lf between the first connection hinge axis 831 and the second connection hinge axis 832.

Accordingly, the nozzle duct hinge axis 813, the nozzle hinge axis 825, the first connection hinge axis 831 and the second connection hinge axis 832 may form each vertex shape of a parallelogram in which two pairs of sides are parallel to each other.

Accordingly, even if the nozzle duct 810 rotates with respect to the inner cabinet 100, the nozzle duct 810 and the nozzle connector 830 may remain parallel to each other. In addition, even if the nozzle duct 810 rotates with respect to the inner cabinet 100, the nozzle body 822 may maintain an angle.

Therefore, even if the nozzle duct 810 rotates with respect to the inner cabinet 100, the nozzle 820 may inject air in a predetermined direction, that is, in a downward direction.

In addition, even if the nozzle duct 810 rotates with respect to the inner cabinet 100, the nozzle handle 826 may maintain a constant angle. Accordingly, even if the nozzle duct 810 rotates, a hand though which the nozzle handle 826 is held may maintain a constant angle. Accordingly, manipulability of the nozzle handle 826 may be improved.

As shown in FIG. 65, the user may hold the nozzle handle 826 and rotate the nozzle duct 810 so that the nozzle 820 ascends. The user may rotate the nozzle duct 810 to bring the upper surface of the nozzle body 822 into contact with the bottom surface of the inner upper plate 115.

Based on a state in which the upper surface of the nozzle body 822 is in contact with the lower surface of the inner upper plate 115 by adjusting the angle of the nozzle duct 810, the first region 115a and the nozzle 820 may form an attractive force by magnetic force.

That is, one of the first region 115a and the nozzle body 822 may include a first magnetic body M1. Further, the other one of the first region 115a and the nozzle body 822 may include a second magnetic body M2. The first magnetic body M1 and the second magnetic body M2 may form the attractive force by magnetic force.

In one embodiment, the first magnetic body M1 and the second magnetic body M2 may be formed of permanent magnets. Alternatively, one of the first and second magnetic bodies M1 and M2 may be made of a permanent magnet, and the other of the first and second magnetic bodies M1 and M2 may be made of a ferromagnetic material.

In one embodiment, the first region 115a may include the first magnetic body M1. Further, the nozzle body 822 may include the second magnetic body M2.

When the shoes are not treated, the upper surface of the nozzle body 822 may be brought into contact with the lower surface of the inner upper plate 115 by magnetic force between the first magnetic body M1 and the second magnetic body M2.

As described above, even if the nozzle duct 810 rotates with respect to the inner cabinet 100, the nozzle handle 826 may maintain a constant angle. Accordingly, whenever the upper surface of the nozzle body 822 comes into contact with the lower surface of the inner upper plate 115, the first magnetic body M1 and the second magnetic body M2 may form a constant manpower all the times.

The upper surface of the nozzle body 822 may be formed to correspond to the lower surface of the first region 115a. Accordingly, while the upper surface of the nozzle body 822 is brought into contact with the lower surface of the inner upper plate 115, the upper surface of the nozzle body 822 may be in close contact with the lower surface of the inner upper plate 115.

Therefore, when the shoes are not processed, it is possible to suppress the movement of the nozzle body 822 due to external impact and gravity, as well as the occurrence of failures and noise accordingly.

When the shoes are treated, the user may hold the nozzle handle 826 and rotate the nozzle duct 810 so that the nozzle 820 descends. The user may rotate the nozzle duct 810 to insert at least a part of the nozzle protrusion 823 into the shoes.

Based on the state in which the upper surface of the nozzle 820 is in contact with the lower surface of the inner upper plate 115, the nozzle 820 may be disposed in a lower part of the first region 115a, and the nozzle handle 826 may be disposed in a lower part of the second region 115b.

In one embodiment, the front surface of the nozzle body (822) may be located directly below the boundary between the first region 115a and the second region 115b with respect to the first direction (X direction).

Accordingly, at least a part of the nozzle handle 826 may be located in front of the first direction (X direction) based on the virtual surfaces extending the inclined surface of the second region 115b, that is, the first virtual surface IP1 and the second virtual surface IP2.

Accordingly, when the user's gaze coincides with or is lower than the first and second virtual surfaces IP1 and IP2, some or all of the nozzle handles 826 may be exposed to the user's viewing angle. Accordingly, the user may immediately check the position of the nozzle handle 826 to grip the nozzle handle 826 and rotate the nozzle duct 810.

As shown in FIG. 65, the first virtual surface IP1 and the second virtual surface IP2 may be positioned above the shoes. In addition, when treating the shoes, the user may hold the nozzle handle 826 and rotate the nozzle duct 810 so that the nozzle 820 descends.

Accordingly, based on a state in which the nozzle handle 826 is moved by adjusting the angle of the nozzle duct 810, at least a part or all of the nozzle handle 826 may move in front of the first direction (X direction) based on the virtual surfaces extending the inclined surface of the second area 115b, that is, the first virtual surface IP1 and the second virtual surface IP2.

Accordingly, based on a state in which the nozzle handle 826 is moved by adjusting the angle of the nozzle duct 810, part or all of the nozzle handle 826 may be exposed to the user" viewing angle. Accordingly, the user may grip the nozzle handle 826 and rotate the nozzle duct 810 while visually checking the position of the nozzle handle 826.

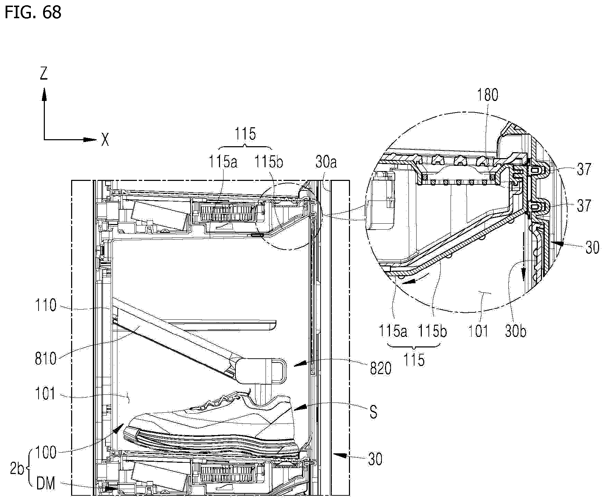

FIG. 68 is a cross-sectional view illustrating a state in which the door 30 is removed from the shoes care device 1 illustrated in FIG. 4c. FIG. 68 illustrates the inside of the inner cabinet 100 of the second management device 2b.

The door 30 is configured to open and close the inside of the shoes care device 1. That is, the door 30 may be configured to open and close the main opening 140. In one embodiment, the shoes care device 1 may include one door 30.

The door 30 may form one surface of the shoes care device 1. In one embodiment, the door 30 may form a front surface of the shoes care device 1 based on the first direction (X direction). In shoes care device 1, the door 30 may be hinge-coupled.

The door 30 may include a front plate 30a and a rear plate 30b. The front plate 30a may form the front surface of the shoes care device 1 in a state where the door 30 closes the accommodation space 101. The rear plate 30b may form a surface for closing the accommodation space 101 in a state where the door 30 closes the accommodation space 101.

A packing 37 may be coupled to the rear plate 30b. With the door 30 closing the accommodation space 101, the packing 37 may be compressed between the rear plate 30b and the front surface of the inner cabinet 100 along the circumference of the main opening 140. The packing 37 may remove a gap between the front surface and rear plate 30b of the inner cabinet 100.

When using the shoes care device 1, frostiness in which air containing water vapor becomes water droplets and is attached to the wall may occur on the inner surface of the inner cabinet 100 and on the rear plate 30b.

As described above, the second region 115b of the first management device 2a may form the spacing angle θ2 with respect to the horizontal plane. The second region 115b of the second management device 2b may form the spacing angle δ2 with respect to the horizontal plane. Accordingly, moisture formed in the second region 115b may move to the boundary of the first region 115a along the inclined surface of the second region 115b.

Accordingly, moisture generated in the inner cabinet 100 may not move to the rear plate 30b. Accordingly, the amount of moisture formed on the rear plate 30b may be suppressed.

As described above, the first region 115a of the first management device 2a may form the spacing angle θ1 with respect to the horizontal plane. The first region 115a of the second management device 2b may form the spacing angle δ1 with respect to the horizontal plane.

Accordingly, the moisture moving to the boundary of the first region 115a may move to the inner rear plate 110 along the inclined surface of the first region 115a. Accordingly, the moisture generated on the inner upper plate 115 may not drop to the shoes. Accordingly, contamination of shoes caused by frostiness may be prevented.

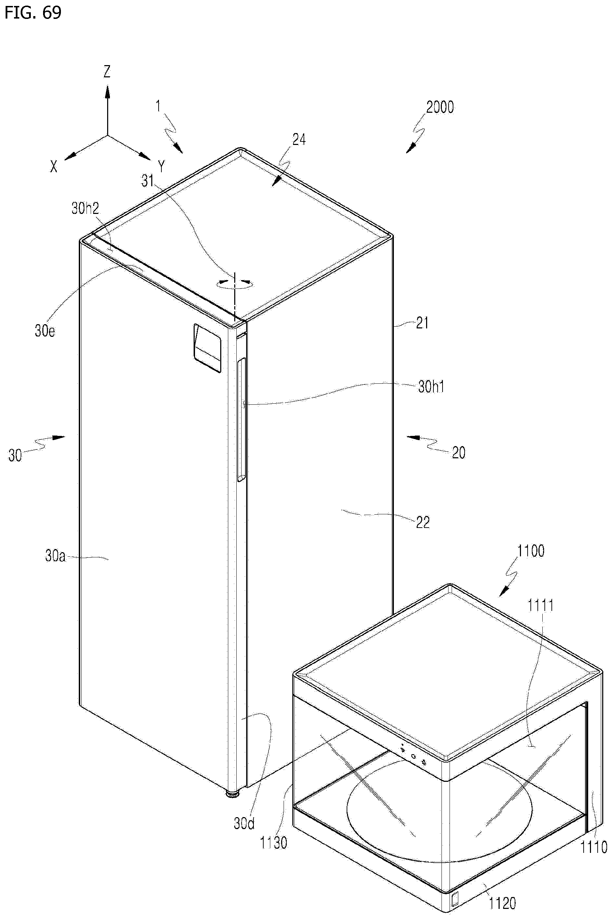

FIG. 69 is a perspective view illustrating the shoes care device 1 and a visible shoes care device 1100 of a shoes management set 1000 according to one embodiment of the present invention.

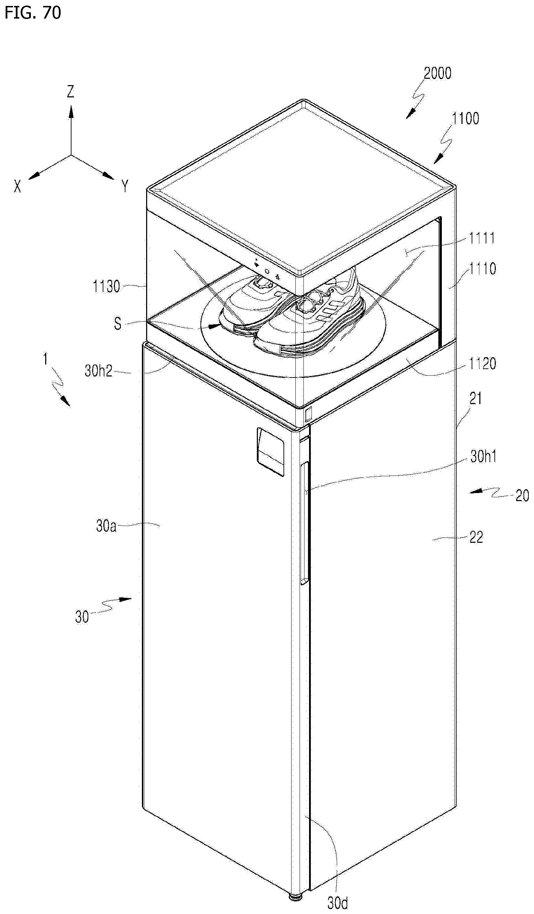

FIG. 70 is a perspective view illustrating a state in which the visible shoes care device 1100 of the shoes management set 1000 of FIG. 69 is stacked on an upper surface of the shoes care device 1.

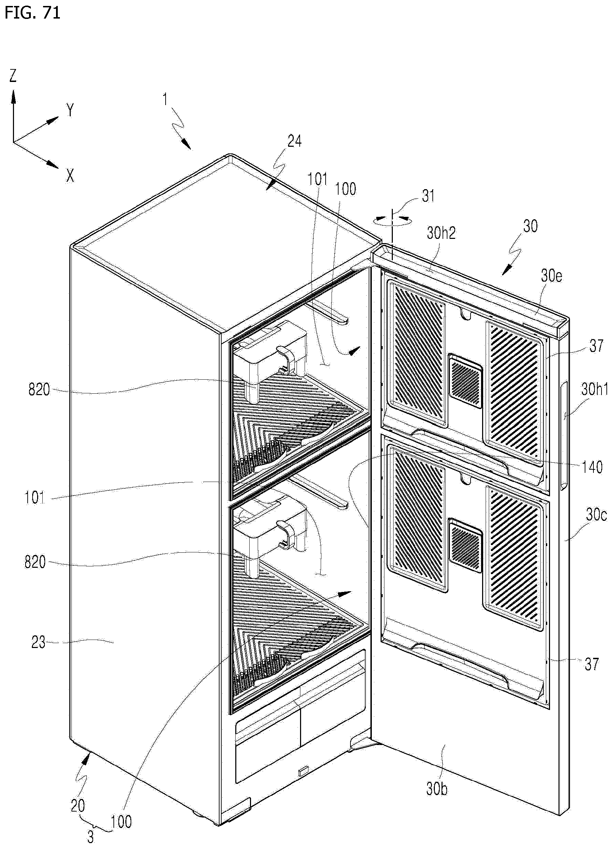

FIG. 71 is a perspective view of the shoes care device 1 of FIG. 69 when viewed from another direction, illustrating a state in which the door 30 is opened.

The shoe management set 1000 according to one embodiment of the present invention may include the shoes care device 1 and the visible shoes care device 1100.

The shoes care device 1 may form the accommodation space 101 in which air is forcibly circulated to treat the shoes. The shoes care device 1 may include a cabinet 3 and the door 30.

The cabinet 3 may include the inner cabinet 100 and the outer cabinet 20.

The inner cabinet 100 may form the accommodation space 101 inside. The inner cabinet 100 may have the main opening 140 that opens the accommodation space 101 in the first direction (X direction) as the horizontal direction. In one embodiment, the inner cabinet 100 may form two accommodation spaces 101. The two accommodation spaces 101 may be vertically spaced apart from each other.

In one embodiment, the first direction (X direction) may be a direction from the rear of the shoes care device 1 to the front. The first direction (X direction) may be parallel to the front-rear direction.

In addition, hereinafter, except for a particularly limited case, it will be described that the second direction (Y direction) is parallel to the left-right direction, and the third direction (Z direction) is parallel to the vertical direction.

The outer cabinet 20 may include the outer rear plate 21, the first outer side plate 22, the second outer side plate 23, and an outer upper plate 24.

The outer rear plate 21, the first outer side plate 22, the second outer side plate 23, and the outer upper plate 24 may be formed integrally with each other. Alternatively, the outer rear plate 21, the first outer side plate 22, the second outer side plate 23, and the outer upper plate 24 may be individually formed.

The outer upper plate 24 may form an upper surface of the cabinet 3. At least a part of the outer upper plate 24 may form a surface orthogonal to the third direction (Z direction). The outer upper plate 24 may form an upper wall surface of the outer cabinet 20 in the third direction (Z direction).

The door 30 may be configured to open and close the main opening 140. In shoes care device 1, the door 30 may be hinge-coupled to the cabinet 3. The hinge rotation axis 31 of the door 30 may be formed in the vertical direction. That is, in shoes care device 1, the door 30 may be configured to be rotatable bidirectionally around the rotation axis 31 in the vertical direction.

The door 30 may include the front plate 30a, the rear plate 30b, a first side plate 30c, a second side plate 30d, and an upper plate 30e. The front plate 30a may form the front surface of the shoes care device 1 in a state where the door 30 closes the accommodation space 101. The rear plate 30b may form a surface for closing the accommodation space 101 in a state where the door 30 closes the accommodation space 101.

The packing 37 may be coupled to the rear plate 30b. With the door 30 closing the accommodation space 101, the packing 37 may be compressed between the rear plate 30b and the front surface of the inner cabinet 100 along the circumference of the main opening 140. Accordingly, with the door 30 closing the accommodation space 101, an upper surface of the door 30 and the upper surface of the cabinet 3 may be spaced apart by a predetermined distance by the packing 37.

A concave groove (hereinafter, referred to as a 'first concave groove 30h1') may be formed in the first side plate 30c and the second side plate 30d. The user may pull and open the door 30 by applying force to the inner surface of the first concave groove 30h1. A concave groove (hereinafter, referred to as a 'second concave groove 30h2') may be formed in the upper plate 30e. The user may pull and open the door 30 by applying force to the inner surface of the second concave groove 30h2.

The outer cabinet 20 and the door 30 may form an overall appearance of the shoes care device 1. The outside of the shoes care device 1 may be formed in a hexahedral shape. That is, in a state where the outer cabinet 20 and the door 30 are coupled to each other and the door 30 is closed, the appearance of the shoes care device 1 may be formed in a hexahedral shape.

The shoes care device 1 may include the nozzle 820. The nozzle 820 may be provided in the accommodation space 101. The nozzle 820 may inject the forcibly ventilated air while being inserted into the shoes.

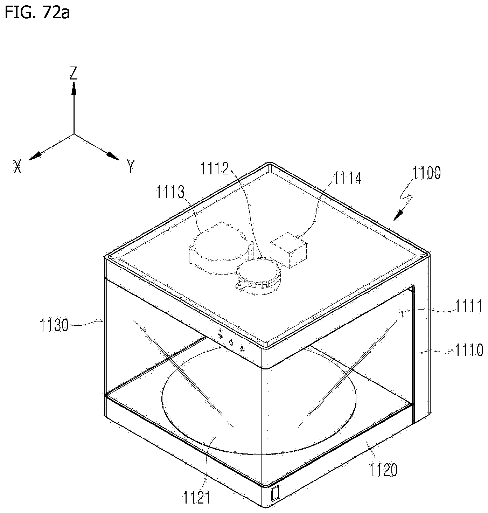

FIG. 72a is a perspective view illustrating the visible shoes care device 1100 of FIG. 69.

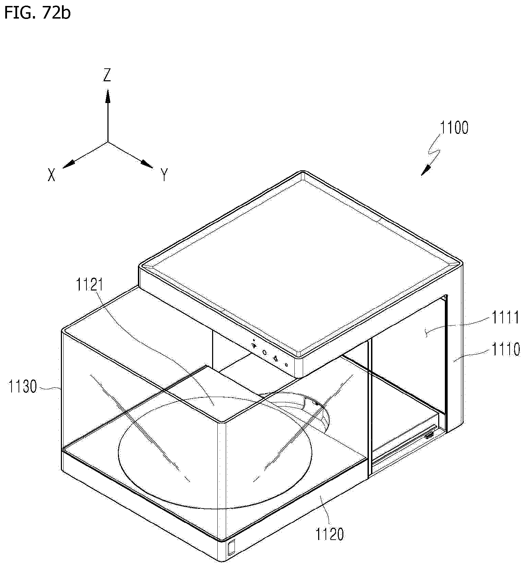

FIG. 72b is a view illustrating a state in which a display space 1111 of the visible shoes care device 1100 of FIG. 72 is opened.

The visible shoes care device 1100 may have the display space 1111 formed inside such that the shoes are displayed. The visible shoes care device 1100 may include a main body 1110, a moving body 1120, and a perspective window 1130.

The main body 1110 may form the display space 1111. In one embodiment, the main body 1110 may form a substantially ' 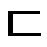 ' shape. The display space 1111 may form a substantially hexahedral shape.

The main body 1110 may form boundaries of three of the six surfaces of the display space 1111. That is, the main body 1110 may form boundaries of a rear surface in the first direction (X direction), and an upper surface and a lower surface in the third direction (Z direction), among the six surfaces of the display space 1111.

An upper part of the main body 1110 may be provided with a lighting 1112, a fan 1113 and a heater 1114.

The lighting 1112 may illuminate the display space 1111. The air forcibly ventilated by the fan 1113 may be introduced into the display space 1111 through a ventilation outlet 1115. Air in the display space 1111 may be introduced into the fan 1113 through a vent 1116. Accordingly, the air forcibly ventilated by the fan 1113 may be circulated in the display space 1111.

The heater 1114 may transfer thermal energy to the air forcibly ventilated by the fan 1113. The temperature of the display space 1111 may be adjusted by the heater 1114.

The moving body 1120 may include a turntable 1121. The shoes may be settled on the turntable 1121. A lower part of the moving body 1120 may form a shape surrounding the lower part of the main body 1110. The moving body 1120 may form a substantially ' 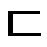 ' shape.

The moving body 1120 may be slidably coupled to the main body 1110 in a direction parallel to the first direction (X direction). For example, a rail parallel to the first direction (X direction) may be formed in the main body 1110. Furthermore, the moving body 1120 may be provided with a slider that slides along the rail.

The moving body 1120 may include the perspective window 1130. The perspective window 1130 may form a surface in contact with the display space 1111. The perspective window 1130 may form a substantially ' 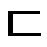 ' shape.

The perspective window 1130 may form boundaries of three of the six surfaces of the display space 1111. That is, the perspective window 1130 may form boundaries the front surface in the first direction (X direction) and opposites surfaces in the second direction (Y direction), among the six surfaces of the display space 1111.

As illustrated in FIG. 72A, the moving body 1120 may move backward in the first direction (X direction) to close the display space 1111. As illustrated in FIG. 72b, the moving body 1120 may slide in the first direction (X direction) to open the display space 1111.

The user may insert the shoes into the display space 1111 through a gap between the main body 1110 and the moving body 1120. The user may withdraw the shoes accommodated in the display space 1111 through the gap between the main body 1110 and the moving body 1120.

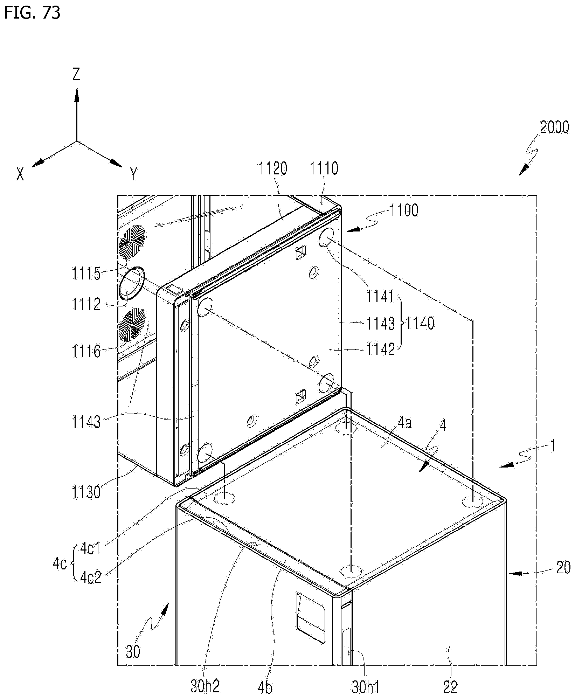

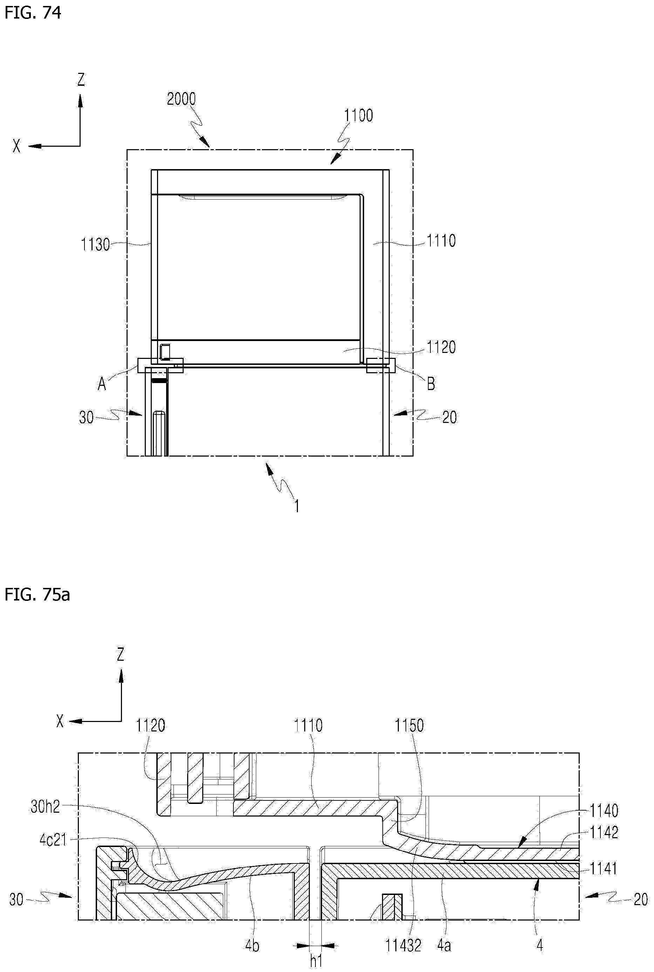

FIG. 73 is a perspective view illustrating a bottom surface of the visible shoes care device 1100 and the upper surface of the shoes care device 1, which constitutes the shoes management set 1000 of FIG. 69. FIG. 74 is a partial side view of the shoes management set 1000 of FIG. 70.

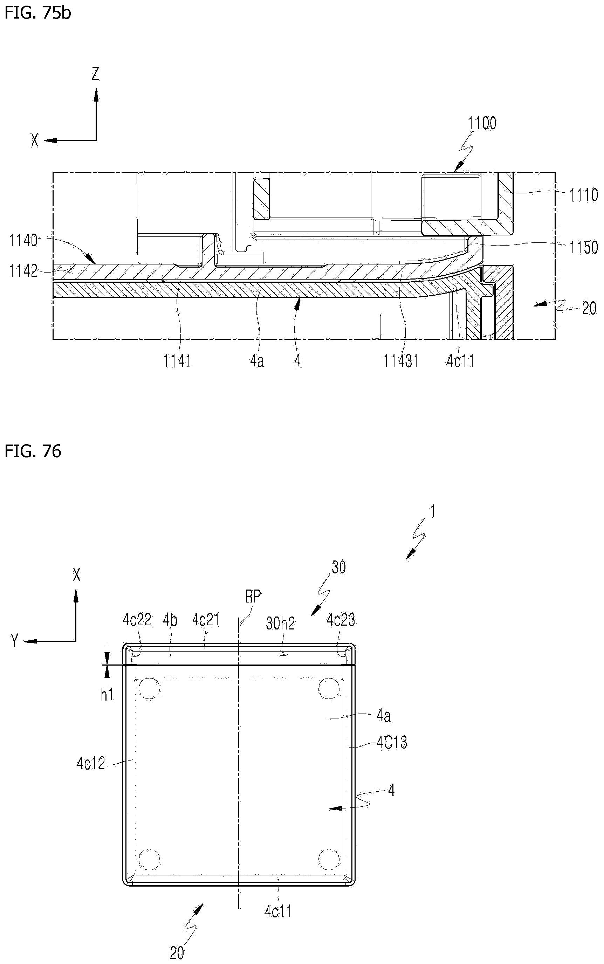

FIG. 75a is a partial cross-sectional view of the shoes management set 1000 of FIG. 74 cut in an XZ plane and is a view illustrating Part A of FIG. 74. FIG. 75b is a partial cross-sectional view of the shoes management set 1000 of FIG. 74 cut into the XZ plane and is a view illustrating Part B of FIG. 74.

FIG. 76 is a top plan view illustrating the upper surface of the shoes care device 1 of FIG. 69.

The accommodation portion 4 recessed downwards may be formed on the upper surface of the shoes care device 1. The accommodation portion 4 may form a symmetrical shape with respect to the reference plane RP parallel to the first direction (X direction) and the vertical direction.

The cabinet 3 and the door 30 may form the accommodation portion 4 together. The outer upper plate 24 may form the upper surface of the cabinet 3. The upper plate 30e may form the upper surface of the door 30. Accordingly, the outer upper plate 24 and the upper plate 30e may form the accommodation portion 4 together.

The accommodation portion 4 may include an accommodation bottom portion 4a, an accommodation extension portion 4b, and an accommodation side portion 4c.

The accommodation bottom portion 4a may be formed on the upper surface of the cabinet 3. The accommodation bottom portion 4a may form a surface on which an article may be settled. In one embodiment, the accommodation bottom portion 4a may form a plane parallel to a horizontal plane. In one embodiment, the accommodation bottom portion 4a may have a substantially rectangular shape.

Here, the article may mean an object having a constant volume. For example, the article may refer to an electronic product, household goods, office goods, exhibition goods, etc. In one embodiment, as the accommodation bottom portion 4a forms a plane parallel to the horizontal plane, various article can be stably maintained with the articles settled in the accommodation bottom part 4a. In one embodiment, the article may refer to the visible shoes care device 1100.

The accommodation extension portion 4b may be formed on the upper surface of the door 30. With the door 30 closing the accommodation space 101, the upper surface of the door 30 and the upper surface of the cabinet 3 may be spaced apart from each other by a predetermined distance h1 by the packing 37. Accordingly, the accommodation extension portion 4b and the accommodation bottom portion 4a may be spaced apart from each other by the distance of h1 in a direction parallel to the first direction (X direction).

The accommodation extension portion 4b may form a surface adjacent to the accommodation bottom portion 4a, which has the same height as the accommodation bottom portion 4a. Accordingly, even if the visible shoes care device 1100 (or the article) settled in the accommodation bottom portion 4a is slightly pushed to the accommodation extension portion 4b due to external force or tilting of the shoes case device 1, the visible shoes care device 1100 (or the article) can be prevented from being reversed due to the height difference between the accommodation bottom portion 4a and the accommodation extension portion 4b.

The accommodation side portion 4c may form an edge of the accommodation portion 4. The accommodation side portion 4c may form a height greater than that of the accommodation bottom portion 4a. The accommodation side portion 4c may include a first accommodation side portion 4c1 and a second accommodation side portion 4c2.

The first accommodation side portion 4c1 may be formed on the upper surface of the cabinet 3 along the edge of the accommodation portion 4. The first accommodation side portion 4c1 may form a surface connected to the accommodation bottom portion 4a. The first accommodation side portion 4c1 may include an eleventh accommodation side portion 4c11, a twelfth accommodation side portion 4c12, and a thirteenth accommodation side portion 4c13.

The eleventh accommodation side portion 4c11 may be formed in the rear part in the first direction (X direction) with respect to the accommodation bottom portion 4a. The eleventh accommodation side portion 4c11 may be formed in the second direction (Y direction). As the eleventh accommodation side portion 4c11 goes to the edge of the accommodation portion 4, that is, moves away from the accommodation bottom portion 4a to the rear part in the first direction (X direction), it may rise upwards in the third direction (Z direction).

In the process in which the user settles the visible shoes care device 1100 (or the article) on the accommodation bottom portion 4a, the eleventh accommodation side portion 4c11 may form an inclined surface guiding the bottom surface of the visible shoes care device 1100 (or the article) to the accommodation bottom portion 4a, that is, the first direction (X direction).

In addition, when the visible shoes care device 1100 (or the article) settled in the accommodation bottom portion 4a is pushed to the eleventh accommodation side portion 4c11 due to the external force or the tilting of the shoes case device 1, the eleventh accommodation side portion 4c11 may form a boundary where the visible shoes care device 1100 (or article) is pushed to the rear part in the first direction (X direction) due to the height difference from the accommodation bottom portion 4a.

Accordingly, various visible shoes care devices 1100 (or articles) settled in the accommodation bottom portion 4a can stably maintain a state of being settled in the accommodation bottom portion 4a.

In addition, as the eleventh accommodation side portion 4c11 moves to the edge of the accommodation portion 4, that is, gets away from the accommodation bottom portion 4a to the rear in the first direction (X direction), the gradient may increase.

Accordingly, even if the visible shoes care device 1100 (or article) settled in the accommodation bottom portion 4a is slightly pushed to the eleventh accommodation side portion 4c11 due to the external force or the tilting of the shoes case device 1, the visible shoes care device 1100 (or the article) can be prevented from being reversed due to the height difference between the accommodation bottom portion 4a and the eleventh accommodation side portion 4c11.

In addition, the eleventh accommodation side portion 4c11 can stably form a boundary where the visible shoes care device 1100 (or the article) is pushed to the rear part in the first direction (X direction).

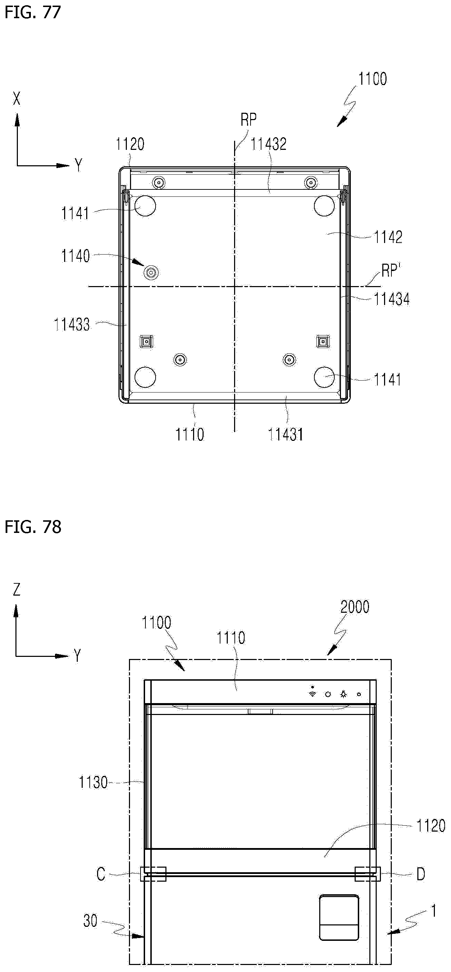

FIG. 77 is a bottom view illustrating a bottom surface of the visible shoes care device 1100 of FIG. 69. FIG. 78 is a partial front view of the shoes management set 1000 of FIG. 70.

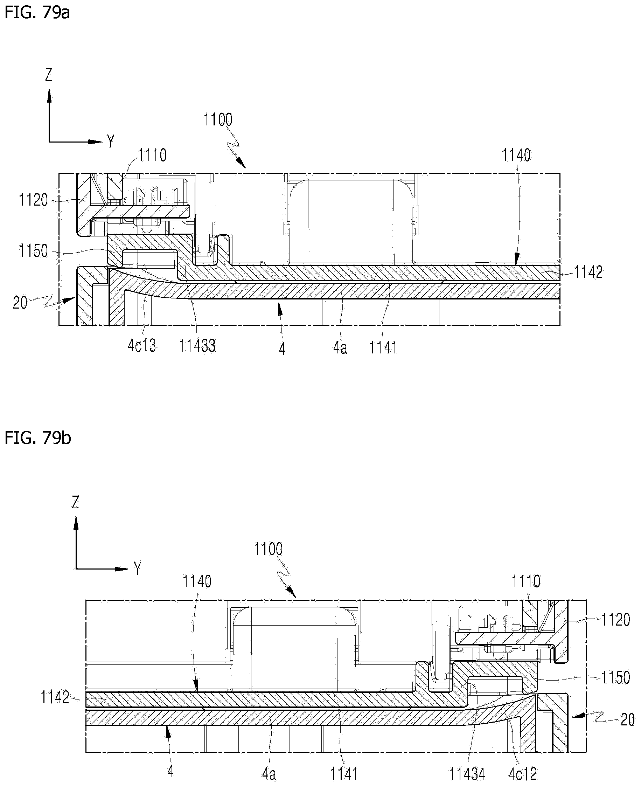

FIG. 79a is a partial cross-sectional view of the shoes management set 1000 of FIG. 78 cut into an YZ plane and is a view illustrating Part C of FIG. 78

FIG. 79b is a partial cross-sectional view of the shoes management set 1000 of FIG. 78 cut into the YZ plane and is a view illustrating Part D of FIG. 78.

The twelfth accommodation side portion 4c12 may be formed on one side in the second direction (Y direction) with respect to the accommodation bottom portion 4a. The twelfth accommodation side portion 4c12 may be formed in the first direction (X direction). As the twelfth accommodation side portion 4c12 goes to the edge of the accommodation portion 4, that is, moves away from the accommodation bottom portion 4a to one side in the second direction (Y direction), it may rise upwards in the third direction (Z direction).

In the process in which the user settles the visible shoes care device 1100 (or the article) on the accommodation bottom portion 4a, the twelfth accommodation side portion 4c12 may form an inclined surface guiding the bottom surface of the visible shoes care device 1100 (or the article) to the accommodation bottom potion 4a, that is, to the other side in the second direction (Y direction).

In addition, when the visible shoes care device 1100 (or the article) settled in the accommodation bottom portion 4a is pushed to the twelfth accommodation side portion 4c12 due to the external force or the tilting of the shoes case device 1, the twelfth accommodation side portion 4c12 may form a boundary where the visible shoes care device 1100 (or the article) is pushed to the rear part in the first direction (X direction) due to the height difference from the accommodation bottom portion 4a.

Accordingly, various visible shoes care devices 1100 (or articles) settled in the accommodation bottom portion 4a can stably maintain the state of being settled in the accommodation bottom portion 4a.

In addition, the twelfth accommodation side portion 4c12 may form a surface continuous with the surface of the accommodation bottom portion 4a. Further, as the twelfth accommodation side portion 4c12 moves to the edge of the accommodation portion 4, that is, gets away from the accommodation bottom portion 4a to one side in the second direction (Y direction), the gradient may increase.

Accordingly, even if the visible shoes care device 1100 (or the article) settled in the accommodation bottom portion 4a is slightly pushed to the twelfth accommodation side portion 4c12 due to the external force or the tilting of the shoes case device 1, the visible shoes care device 1100 (or the article) may be prevented from being reversed due to the height difference between the accommodation bottom portion 4a and the twelfth accommodation side portion 4c12.

In addition, the twelfth accommodation side portion 4c12 can stably form a boundary where the visible shoes care device 1100 (or the article) is pushed to one side in the second direction (Y direction).

The thirteenth accommodation side portion 4c13 may be formed on the other side in the second direction (Y direction) with respect to the accommodation bottom portion 4a. The thirteenth accommodation side portion 4c13 may be formed in the first direction (X direction). As the thirteenth accommodation side portion 4c13 goes to the edge of the accommodation portion 4, that is, moves away from the accommodation bottom portion 4a to the other side in the second direction (Y direction), it may rise upwards in the third direction (Z direction).

In the process in which the user settles the visible shoes care device 1100 (or the article) on the accommodation bottom portion 4a, the thirteenth accommodation side portion 4c13 may form an inclined surface guiding the bottom surface of the visible shoes care device 1100 (or the article) to the accommodation bottom portion 4a, that is, to one side in the second direction (Y direction).

In addition, when the visible shoes care device 1100 (or the article) settled in the accommodation bottom portion 4a is pushed to the thirteenth accommodation side portion 4c13 due to the external force or the tilting of the shoes case device 1, the thirteenth accommodation side portion 4c13 may form a boundary where the visible shoes care device 1100 (or the article) is pushed to the other side in the second direction (Y direction) dur to the height difference from the accommodation bottom portion 4a.

Accordingly, various visible shoes care devices 1100 (or articles) settled in the accommodation bottom portion 4a can stably maintain the state of being settled in the accommodation bottom portion 4a.

In addition, the thirteenth accommodation side portion 4c13 may form a surface continuous with the surface of the accommodation bottom portion 4a. Further, as thirteenth accommodation side portion 4c13 moves to the edge of the accommodation portion, that is, gets away from the accommodation bottom portion 4a to the other side in the second direction (Y direction), the gradient may increase.

Accordingly, even if the visible shoes care device 1100 (or the article) settled in the accommodation bottom portion 4a is slightly pushed to the thirteenth accommodation side portion 4c13 due to the external force or the tilting of the shoes case device 1, the visible shoes care device 1100 (or article) may be prevented from being reversed due to the height difference between the accommodation bottom portion 4a and the thirteenth accommodation side portion 4c13.

In addition, the thirteenth accommodation side portion 4c13 can stably form a boundary where the visible shoes care device 1100 (or the article) is pushed to the other side in the second direction (Y direction).

The second accommodation side portion 4c2 may be formed on the upper surface of the door 30 along the edge of the accommodation portion 4. The second accommodation side portion 4c2 may form a surface connected to the accommodation extension portion 4b. The second accommodation side portion 4c2 may include a twenty-first accommodation side portion 4c21, a twenty-second accommodation side portion 4c22, and a twenty-third accommodation side portion 4c23.

The twenty-first accommodation side portion 4c21 may be formed in the front part in the first direction (X direction) with respect to the accommodation extension portion 4b. The twenty-first accommodation side portion 4c21 may be formed in the second direction (Y direction). As the twenty-first accommodation side portion 4c21 goes to the edge of the accommodation portion 4, that is, moves away from the accommodation extension portion 4b to the front part in the first direction (X direction), it may rise upwards in the third direction (Z direction).

As described above, the accommodation extension portion 4b may have the same height as the accommodation bottom portion 4a on a surface adjacent to the accommodation bottom portion 4a. Accordingly, with the door 30 closed, in the process in which the user settles the visible shoes care device 1100 (or the article) on the accommodation bottom portion 4a, the twenty-first accommodation side portion 4c21 may form an inclined surface guiding the bottom surface of the visible shoes care device 1100 (or the article) to the accommodation bottom portion 4a, that is, the rear part in the first direction (X direction), through the accommodation extension portion 4b.

In addition, with the door 30 closed, the visible shoes care device 1100 (or the article) on the accommodation bottom portion 4a is pushed to the twenty-first accommodation side portion 4c21 due to the external force or the tilting of the shoes care device 1, the twenty-first accommodation side portion 4c21 may form a boundary where the visible shoes care device 1100 (or the article) is pushed to the front part in the first direction (X direction) due to the height difference from the accommodation bottom portion 4a.

Accordingly, various the visible shoes care devices 1100 (or articles) settled in the accommodation bottom portion 4a may not drop outside the accommodation portion 4.

In addition, the twenty-first accommodation side portion 4c21 may form a surface continuous with the surface of the accommodation extension portion 4b. Further, the twenty-first accommodation side portion 4c21 moves to the edge of the accommodation portion 4, that is, gets way from the accommodation extension portion 4b to the front part in the first direction (X direction), the gradient may increase.

Accordingly, with the door 30 closed, even if the visible shoes care device 1100 (or the article) settled in the accommodation bottom portion 4a is slightly pushed to the twenty-first accommodation side portion 4c21 due to the external force or the tilting of the shoes care device 1, the visible shoes care device 1100 (or the article) can be prevented from being reversed due to the height difference between the accommodation extension portion 4b and the twenty-first accommodation side portion 4c21.

In addition, the twenty-first accommodation side portion 4c21 can stably form a boundary where the visible shoes care device 1100 (or the article) is pushed to the front side in the first direction (X direction).

The twenty-second accommodation side portion 4c22 may be formed on one side in the second direction (Y direction) with respect to the accommodation extension portion 4b. The twenty-second accommodation side portion 4c22 may be formed in the first direction (X direction). The twenty-second accommodation side portion 4c22 goes to the edge of the accommodation portion 4, that is, moves away from the accommodation extension portion 4b to one side in the second direction (Y direction), it may rise upwards in the third direction (Z direction). The twenty-second accommodation side portion 4c22 may form a surface continuous with the surface of the accommodation extension portion 4b.

The door 30 may be hinge-coupled to the cabinet 3 around the rotation axis 31 in the vertical direction. As described above, the second concave groove 30h2 may be formed in the upper plate 30e. The user may pull and open the door 30 by applying force to the inner surface of the second concave groove 30h2. The second accommodation side portion 4c2 and the accommodation extension portion 4b may form the second concave groove 30h2.

The twenty-first accommodation side portion 4c21 may be formed in the front part in the first direction (X direction) with respect to the accommodation extension portion 4b. Accordingly, an action force of opening the door 30 may be applied to the door 30 through the second accommodation side portion 4c2.

As illustrated in FIG. 75A, the accommodation extension portion 4b may become lower toward the first direction (X direction). As described above, the twenty-first accommodation side portion 4c21 may form a surface continuous with the surface of the accommodation extension portion 4b. Accordingly, an area of the second accommodation side portion 4c2 to which the action force of opening the door 30 is applied may increase downwards in the third direction (Z direction).

Accordingly, the user's finger may form a wide contact surface with the second accommodation side portion 4c2. Accordingly, the action force of opening the door 30 may be dispersed on the contact surface. Therefore, when the door 30 is opened, as the pressure acting on a unit area of a finger surface decreases, the user can feel that the door 30 is easily opened.

The action force applied to the twenty-first accommodation side portion 4c21 through the user's finger may be divided into an X-axis component and a Z-axis component. A force applied to the eleventh accommodation side portion 4c11 through the bottom surface of the visible shoes care device 1100 (or the article) may also be divided into an X-axis component and a Z-axis component.

As illustrated in FIGS. 75a and 75b, the gradient of the second accommodation side portion 4c2 may be greater than the gradient of the first accommodation side portion 4c1 with respect to a direction parallel to the first direction (X direction). That is, the gradient of the twenty-first accommodation side portion 4c21 may be greater than the gradient of the eleventh accommodation side portion 4c11.

Accordingly, the action force applied to the twenty-first accommodation side portion 4c21 through the user's finger may be greater in the X-axis direction component than in the Z-axis direction component. Accordingly, the user may feel that the door 30 is easily opened.

In addition, the force applied to the eleventh accommodation side portion 4c11 through the bottom surface of the visible shoes case device 1100 (or the article) may be greater in the Z-axis direction component than in the X-axis direction component. Accordingly, while the bottom surface of the visible shoes case device 1100 (or the article) is in contact with the eleventh accommodation side portion 4c11, the possibility of reversing the visible shoes case device 1100 (or the article) can be reduced.

The first accommodation side portion 4c1 and the second accommodation side portion 4c2 may have the same height along the edge of the accommodation portion 4. Therefore, with the door 30 closed, the accommodation part 4 can be observed as an integral concave part having the accommodation side portion 4c of a certain height along the edge thereof, and the aesthetics of the upper surface of the shoes care device 1 can be improved.

An insertion portion 1140 that is convex downwards may be formed on the bottom surface of the main body 1110 such that it can be inserted into the accommodation portion 4. The insertion portion 1140 may be inserted into the accommodation portion 4 below the Z-axis. By inserting the insertion portion 1140 into the accommodation portion 4, the visible shoes care device 1100 may be stacked on the upper surface of the shoes care device 1.

The insertion portion 1140 may include a settling portion 1141, an insertion bottom portion 1142, and an insertion side portion 1143.

The settling portion 1141 is a component that can be settled in the accommodation bottom portion 4a, and is provided in a plural form. Three or more settling portions 1141 may be provided to prevent the reversing of the visible shoes care device 1100.

In one embodiment, four settling portions 1141 may be provided. The settling portions 1141 may protrude from the insertion bottom portion 1142 to a lower part of the Z-axis. The settling portions 1141 may form various shapes. In one embodiment, the settling portions 1141 may form a circular shape in a plan view.

The insertion bottom portion 1142 may be spaced apart upwards from the accommodation bottom portion 4a by the settling portion 1141. The insertion bottom portion 1142 may form a plane parallel to the horizontal plane. Accordingly, a gap between the insertion bottom portion 1142 and the accommodation bottom portion 4a in the Z-axis direction may be the same as the gap along the X-axis direction and the Y-axis direction. In one embodiment, the insertion bottom portion 1142 may have a substantially rectangular shape.

A dotted line of FIG. 76 may mean an edge of the insertion bottom portion 1142. The length of the insertion bottom portion 1142 with respect to the Y-axis direction may be substantially the same as the length of the accommodation bottom portion 4a.

The length of the insertion bottom portion 1142 may be smaller than the length of the accommodation bottom portion 4a with respect to the first direction (X direction). Accordingly, the settling portions 1141 protruding from the insertion bottom portion 1142 to the lower part of the Z-axis may be settled in the accommodation bottom portion 4a.

The insertion side portion 1143 may be formed along the edge of the insertion bottom portion 1142. The insertion side portion 1143 may be connected to the insertion bottom portion 1142. The insertion side portion 1143 may include a first insertion side portion 11431, a second insertion side portion 11432, a third insertion side portion 11433, and a fourth insertion side portion 11434.

The first insertion side portion 11431 may be formed in the rear part in the first direction (X direction) with respect to the insertion bottom portion 1142. The first insertion side portion 11431 may be formed in the second direction (Y direction). As the first insertion side portion 11431 goes to the edge of the insertion portion 1140, that is, moves away from the insertion bottom portion 1142 to the rear part in the first direction (X direction), it may rise upwards in the third direction (Z direction).

At least a part of the first insertion side portion 11431 may be lower than an upper end of the eleventh accommodation side portion 4c11. Accordingly, in the process in which the user settles the visible shoes care device 1100 (or the article) on the accommodation bottom portion 4a, the first insertion side portion 11431 may form an inclined surface corresponding to the eleventh accommodation side portion 4c11.

When the visible shoes care device 1100 (or the article) settled in the accommodation bottom portion 4a is pushed to the eleventh accommodation side portion 4c11 by the external force or the tilting of the shoes care device 1, the first insertion side portion 11431 may form an inclined surface in close contact with to the eleventh accommodation side portion 4c11.

When the visible shoes care device 1100 (or the article) settled in the accommodation bottom portion 4a is pushed to the eleventh accommodation side portion 4c11 by the external force or the tilting of the shoes care device 1, the first insertion side portion 11431 and the eleventh accommodation side portion 4c11 may form friction force. Accordingly, various visible shoes care devices 1100 (or articles) settled in the accommodation bottom portion 4a can stably maintain the state of being settled in the accommodation bottom portion 4a.

The second insertion side portion 11432 may be formed in the front part in the first direction (X direction) with respect to the insertion bottom portion 1142. The second insertion side portion 11432 may be formed in the second direction (Y direction). As the second insertion side portion 11432 goes to the edge of the insertion portion 1140, that is, moves away from the insertion bottom portion 1142 to the front part in the first direction (X direction), it may rise upwards in the third direction (Z direction).

The first insertion side portion 11431 and the second insertion side portion 11432 may form a symmetrical shape with respect to a reference plane RP' parallel to the second direction (Y direction) and the vertical direction.

That is, as shown in FIG. 70, when the visible shoes care device 1100 rotates 180 degrees around the Z-axis, the second insertion side portion 11432 may be located in the rear part in the first direction (X direction) with respect to the insertion bottom portion 1142.

Furthermore, the first insertion side portion 11431 may be positioned in the front part in the first direction (X direction) with respect to the insertion bottom portion 1142. In the process in which the user settles the visible shoes care device 1100 (or the article) in the accommodation bottom portion 4a, the second insertion side portion 11432 may form an inclined surface corresponding to the eleventh accommodation side portion 4c11.

When the visible shoes care device 1100 (or the article) settled in the accommodation bottom portion 4a is pushed to the eleventh accommodation side portion 4c11 by the external force or the tilting of the shoes care device 1, the second insertion side portion 11432 and the eleventh accommodation side portion 4c11 may form frictional force. Accordingly, various visible shoes care devices 1100 (or articles) settled in the accommodation bottom portion 4a can stably maintain the state of being settled in the accommodation bottom portion 4a.

The third insertion side portion 11433 may be formed on one side in the second direction (Y direction) with respect to the insertion bottom portion 1142. The third insertion side portion 11433 may be formed in the first direction (X direction). The third insertion side portion 11433 may form a vertical surface extending upwards in the Z-axis direction from the insertion bottom portion 1142. At least a part of the third insertion side portion 11433 may be higher than an upper end of the thirteenth accommodation side portion 4c13.

When the visible shoes care device 1100 (or the article) settled in the accommodation bottom portion 4a is pushed to the thirteenth accommodation side portion 4c13 by the external force or the tilting of the shoes care device 1, the third insertion side portion 11433 may form a surface in close contact with the thirteenth accommodation side portion 4c13. Accordingly, various visible shoes care devices 1100 (or articles) settled in the accommodation bottom portion 4a can stably maintain the state of being settled in the accommodation bottom portion 4a.

The fourth insertion side portion 11434 may be formed on the other side in the second direction (Y direction) with respect to the insertion bottom portion 1142. The fourth insertion side portion 11434 may be formed in the first direction (X direction). The fourth insertion side portion 11434 may form a vertical surface extending upwards in the Z-axis direction from the insertion bottom portion 1142. At least a part of the fourth insertion side portion 11434 may be higher than an upper end of the twelfth accommodation side portion 4c12.

When the visible shoes care device 1100 (or the article) settled in the accommodation bottom portion 4a is pushed to the twelfth accommodation side portion 4c12 by the external force or the tilting of the shoes care device 1, the fourth insertion side portion 11434 may form a surface in close contact with the twelfth accommodation side portion 4c12. Accordingly, various visible shoes care devices 1100 (or articles) settled in the accommodation bottom portion 4a can stably maintain the state of being settled in the accommodation bottom portion 4a.

As shown in FIGS. 74 and 75A, at least a part of the second accommodation side portion 4c2 may be disposed ahead of the visible shoes care device 1100 with respect to the first direction (X direction) in a state where the display space 1111 is closed.

As described above, the moving body 1120 may form a shape surrounding a lower part of the main body 1110. Accordingly, the moving body 1120 may be provided ahead of the main body 1110 in the first direction (X direction) under the main body 1110.

That is, with the display space 1111 closed, based on the first direction (X direction), at least a part of the second accommodation side portion 4c2 may be disposed ahead of the moving body 1120 in the first direction (X direction). That is, the second accommodation side portion 4c2 and the moving body 1120 may be spaced apart from each other in the first direction (X direction). The user may open the door 30 by inserting his/her finger into the second concave groove 30h2 through a gap between the second accommodation side portion 4c2 and the moving body 1120.

A separation extension portion 1150 may be formed on the bottom surface of the main body 1110. The separation extension portion 1150 may extend downwards in the Z-axis direction from the bottom surface of the main body 1110. The separation extension portion 1150 may separate the insertion portion 1140 from the bottom surface of the main body 1110 downwards in the Z-axis direction.

The insertion portion 1140 may be spaced apart from the bottom surface of the main body 1110 downwards in the Z-axis direction by the separation extension portion 1150. The separation extension portion 1150 and the insertion side portion 1143 may be connected to each other.

The moving body 1120 and the main body 1110 may be spaced apart from the second accommodation side portion 4c2 in the Z-axis direction above the door 30. That is, a gap (or an interval) between the second accommodation side portion 4c2 and the moving body 1120 may be formed in the X-axis direction and the Z-axis direction.

Accordingly, the gap (or the interval) between the second accommodation side portion 4c2 and the moving body 1120 may extend in a direction approximately perpendicular to the direction in which the user's finger enters. The user can open the door 30 by easily inserting his/her finger into the second concave groove 30h2 through the gap between the second accommodation side portion 4c2 and the moving body 1120.

In addition, as the moving body 1120 and the main body 1110 are spaced apart from the second accommodation side portion 4c2 in the Z-axis direction above the door 30, the moving body 1120 can open and close the display space 1111 in a direction parallel to the first direction (X direction) above the insertion portion 1140 and the accommodation portion 4. Accordingly, even if the visible shoes care device 1100 is stacked on the upper surface of the shoes care device 1, the visible shoes care device 1100 may not interfere with the opening and closing of the door 30.

Accordingly, the user may separately open or simultaneously open the door 30 of the shoes care device 1 and the movable body 1120 of the visible shoes care device 1100. Therefore, even if the visible shoes care device 1100 is stacked on the upper surface of the shoes care device 1, the shoes care device 1 and the visible shoes care device 1100 may be conveniently used without interfering with each other.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### Advantageous Effects

By at least one of exemplary embodiments of the present invention, a dehumidifying part is disposed in a module chamber not only to collect moisture and bacteria in a ventilation air but also to regenerate the dehumidifying part by heating the dehumidifying part in the module chamber, thereby maintaining proper shoes treatment performance all the times.

In addition, by at least one of exemplary embodiments of the present invention, a connection path through which air circulates is formed between a outlet and a nozzle respectively disposed inside an inner cabinet, thereby preventing the air used for dehumidifying and deodorizing shoes from being exposed to the user.

In addition, by at least one of exemplary embodiments of the present invention, the shoes are settled on an upper of a main shelf covering a bottom surface of the inner cabinet, thereby easily managing the bottom surface of the inner cabinet by preventing contamination of the inner cabinet.

In addition, by at least one of exemplary embodiments of the present invention, since the main shelf is detached from the inner cabinet, the main shelf can be easily managed.

In addition, by at least one of exemplary embodiments of the present invention, since a main isolation rib is formed on an upper surface of the main shelf, a space for movement of air or water can be formed between the upper surface of the main shelf and bottom surfaces of the shoes.

In addition, by at least one of exemplary embodiments of the present invention, since the main isolation rib is formed in an oblique direction, an area where the upper surface of the main shelf and the bottom surfaces of the shoe come into contact with each other may be further reduced.

In addition, by at least one of exemplary embodiments of the present invention, since the main isolation rib is formed in a symmetrical shape, placing a pair of shoes on both sides of the main shelf, respectively, may be the most effective.

In addition, by at least one of exemplary embodiments of the present invention, the main isolation rib is formed so that water on the main shelf moves to a front side of the inner cabinet, thereby preventing the water from accumulating in a certain part on the main shelf.

In addition, by at least one of exemplary embodiments of the present invention, since a part of the main shelf corresponding to an upper part of a outlet is formed to penetrate, the water moving on the main shelf can be discharged through the outlet.

In addition, by at least one of exemplary embodiments of the present invention, since the main shelf is inclined downwardly toward the front side of the inner cabinet where the outlet is formed, the water moving on the main shelf can move smoothly toward the outlet.

In addition, by at least one of exemplary embodiments of the present invention, since a guide rib is formed on the main shelf to guide the position of the shoes, the position where the shoes are settled for treatment can be properly guided.

In addition, by at least one of exemplary embodiments of the present invention, since a pair of guide ribs are formed symmetrical with each other, the settled position of the shoes can be properly guided even when treating the pair of shoes.

In addition, by at least one of exemplary embodiments of the present invention, since the guide rib is formed in a curved shape, the settled position of the shoes in an optimal structure can be guided by reflecting the curved shape of the shoes.

In addition, by at least one of exemplary embodiments of the present invention, since the guide rib is formed on the front side of the inner cabinet, a user can easily recognize the settled position of the shoes.

In addition, by at least one of exemplary embodiments of the present invention, a main water stop rib is formed along a circumferential surface of the main shelf, thereby preventing the water moving on the main shelf from flowing to an unintended part.

In addition, by at least one of exemplary embodiments of the present invention, since the main water stop rib is in close contact with an inner surface of the inner cabinet, the water flowing down the inner surface of the inner cabinet can be guided and discharged to the upper surface of the main shelf.

In addition, by at least one of exemplary embodiments of the present invention, a central part of the main shelf is convexly formed, thereby preventing water from accumulating in the central part of the main shelf and minimize an area where the upper surface of the main shelf is in contact with the bottom of the shoes.

In addition, by at least one of exemplary embodiments of the present invention, a space is formed between the main shelf and the bottom surface of the inner cabinet, thereby preventing contamination and bacterial growth caused by residual water from occurring between the main shelf and the bottom surface of the inner cabinet.

In addition, by at least one of exemplary embodiments of the present invention, a door drain surface protrudes on an inner surface of the door to induce condensed water flowing down the inner surface toward the inside of the inner cabinet, thereby preventing the condensed water of the inner surface of the door from leaking out of the door.

In addition, by at least one of exemplary embodiments of the present invention, since the door drain surface protrudes to pass through the upper part of the main water stop rib, the condensed water on the inner surface of the door can be guided and discharged to the upper surface of the main shelf.

In addition, by at least one of exemplary embodiments of the present invention, a depression is formed on the door drain surface to correspond to the shape of the guide rib, thereby preventing the condensed water from being induced to an upper part of the part where the shoes are placed.

In addition, by at least one of exemplary embodiments of the present invention, since a drain rib is formed on an upper part of the depression of the door drain surface, the condensed water on the door drain surface can be guided and discharged to the part where the depression is not formed.

## Claims

1. A shoes care device comprising:
an inner cabinet having an accommodation space configured to accommodate shoes;
a outlet formed on a bottom surface of the inner cabinet so as to suck air inside the inner cabinet;
a connection path forming a flow path through which air in the accommodation space is discharged from the inner cabinet through the outlet and then introduced back into the accommodation space;
a blowing part disposed in the connection path and configured to ventilate air; a dehumidifying part disposed in the connection path and configured to dehumidify air; and
a main shelf installed to cover the bottom surface of the inner cabinet and having an upper surface on which the shoes can be settled.

2. The shoes care device of claim 1, wherein the main shelf is detachably installed from the bottom surface of the inner cabinet.

3. The shoes care device of claim 1, wherein the main shelf has a main isolation rib formed to protrude upwards on an upper surface thereof.

4. The shoes care device of claim 3, wherein the main isolation rib is formed in an oblique direction between a front surface and a rear surface of the inner cabinet.

5. The shoes care device of claim 4, wherein the main isolation rib is formed symmetrically from a central part of the inner cabinet to opposite sides.

6. The shoes care device of claim 5, wherein the bottom surface of the inner cabinet is inclined downwardly toward a front side, and
the main isolation rib is formed to face the front surface of the inner cabinet from a central part of the bottom surface of the inner cabinet to opposite sides.

7. The shoes care device of claim 3, wherein the main shelf is formed to penetrate a part covering the outlet.

8. The shoes care device of claim 7, wherein the outlet is formed in a front side of the bottom surface of the inner cabinet, and
the main shelf is disposed to be inclined downwardly toward the front side of the inner cabinet.

9. The shoes care device of claim 3, wherein the main shelf has a guide rib formed on an upper surface thereof to guide a position of the shoes.

10. The shoes care device of claim 9, wherein a pair of guide ribs are formed symmetrical from the central part of the inner cabinet to opposite sides.

11. The shoes care device of claim 9, wherein the guide rib is formed in a shape including a curved surface.

12. The shoes care device of claim 9, wherein the guide rib is formed in the front side of the bottom surface of the inner cabinet.

13. The shoes care device of claim 3, wherein the main shelf is provided with a main water stop rib continuously protruding upwards along a circumferential surface.

14. The shoes care device of claim 13, wherein the main water stop rib is formed to be in close contact with an inner surface of the inner cabinet.

15. The shoes care device of claim 3, wherein the main shelf is formed in a cross-sectional shape that is convex from the central part of the inner cabinet to opposite sides.

16. The shoes care device of claim 3, wherein the main shelf is installed to be partially spaced apart from the bottom surface of the inner cabinet.

17. The shoes care device of claim 13, further comprising: a door opening and closing the front surface of the inner cabinet,
wherein the door includes a door drain surface that inclinedly protrudes to the inside of the inner cabinet while being closer to the bottom surface of the inner cabinet in a state where the front surface of the inner cabinet is closed.

18. The shoes care device of claim 17, wherein the door drain surface is formed to further protrude from an upper part of the main water stop rib to the inside of the inner cabinet.

19. The shoes care device of claim 18, wherein the main shelf has a guide rib formed on an upper surface thereof to guide the position of the shoes, and
the door drain surface has a depression formed to be partially recessed along the shape of the guide rib.

20. The shoes care device of claim 18, wherein a drain rib extending to the rest of the door drain surface is formed in an upper part of the depression
